# EUROPEAN PATENT SPECIFICATION

(11) **EP 1 095 278 B1**
(45) Date of publication and mention of the grant of the patent: **07.12.2005**
(21) Application number: 99934543.2
(22) Date of filing: 29.06.1999
(51) Int. Cl.: G01N 33/68

(54) **DIFFERENTIAL DIAGNOSIS OF NEURODEGENERATION**
DIFFERENTIELLE DIAGNOSE VON NEURODEGENERATION
DIAGNOSTIC DIFFERENTIEL DE LA NEURODEGENERESCENCE

(30) Priority: 03.07.1998 EP 98870148; 03.11.1998 EP 98870236; 09.04.1999 EP 99870069
(43) Date of publication of application: 02.05.2001
(73) Proprietor: Innogenetics N.V., 9052 Ghent (BE)
(72) Inventor: VANMECHELEN, Eugeen, B-9810 Nazareth - Eke (BE); VANDERSTICHELE, Hugo, B-9000 Gent (BE); VAN DE VOORDE, André, B-9160 Lokeren (BE)
(86) International application number: PCT/EP1999/004483
(87) International publication number: WO 2000/002053

(56) References cited:
- SHOJI M ET AL: "Combination assay of CSF Tau, A-beta-1-40 and A-beta-1-42(43) as a biochemical marker of Alzheimer's disease." JOURNAL OF THE NEUROLOGICAL SCIENCES 158 (2). 1998. 134-140. ISSN: 0022-510X, 30 June 1998 (1998-06-30), XP002083581 cited in the application
- TAKEDA A. ET AL: "Abnormal accumulation of NACP/.alpha.- synuclein in neurodegenerative disorders." AMERICAN JOURNAL OF PATHOLOGY, 152/2 (367-372) ,February 1998 (1998-02), XP002120024
- TABIRA, TAKESHI: "Genes for Alzheimer's disease and related disorders" IGAKU NO AYUMI (1998), 185(9), 633-636 , XP002120025 -& CHEMICAL ABSTRACTS, vol. 129, no. 19, 9 November 1998 (1998-11-09) Columbus, Ohio, US; abstract no. 243201, TABIRA, TAKESHI: "Genes for Alzheimer's disease and related disorders" page 474; column 2; XP002120026

## Description

### FIELD OF THE INVENTION

The present invention relates to the field of diagnosis of neurodegeneration. The present invention relates to new methods for the differential diagnosis of neurodegeneration, making use of a combination assay detecting different neurological markers in body fluids. The present description also relates to new methods for the detection of Rab3a, SNAP25 or α-synuclein in cerebrospinal fluid and to the use of these methods in a combination assay for differential diagnosis of neurodegeneration.

### BACKGROUND OF THE INVENTION

Neurodegeneration is a feature of several neurological disorders. Neurodegeneration may involve axonal damage, gradually evolving neuronal death; abnormalities in neurotransmitter release or receptor function: destruction of myelin; alterations in CNS blood flow, blood/brain barrier dysfunction and/or altered oxygen metabolism: difficulties in other CNS metabolic pathways and/or various other, often unknown, aspects that may cause a malfunctioning of the CNS. Today different diseases have been associated with different aspects af neuronal malfunctioning (for an overview see Wilson et al., 1991). Alzheimer's disease, for example, is the most important of all neurodegenerative diseases in which death and disappearance of nerve cells in the cerebral cortex are involved. It is the most common dementia in elderly, causing distress for patients and families and economic loss in the form of the costs necessary for the long-term care of patients totally disabled by the disease. Frontal temporal lobe dementia is the second most common type of primary degenerative dementia and accounts for approximately 3-10% of all patients with dementia (Brun, 1993; Knopman, 1993). The clinical picture is characterized by the presence of a predominating frontal lobe syndrome (Sjögren, 1997), which also can be observed in other disorders like affective disorders and schizophrenia (Abbruzzese et al. 1997). Lewy Body disease is an illness that presents with progressive dementia or psychosis. Parkinsonian signs, which may be absent or mild at the onset, eventually become common and rigidity is usually severe. Lewy bodies are found profusely in the brainstem, basal forebrain, hypothalamic nuclei and neocortex Parkinson disease is a type of Lewy Body disease occurring in the middle or late life, with very gradual progression and a prolonged course. It can be considered as an example of neuronal system disease, involving mainly the nigrostriatal dopaminergic system. Cerebrovascular disease, on the other hand, is caused by one of several pathologic processes involving the blood vessels of the brain. It is the third leading cause of death after heart disease and cancer in developed countries and has an overall prevalence of 794 per 100 000. Five percent of the population over 65 is affected by stroke, an acute neurologic injury occurring as a result of one of these pathologic processes. Neurodegeneration can also be induced by exposure to certain chemical compounds (table 1), irradiation, chemotherapy or hypoxic-ischemic events. The long-term complications of the treatment (or prophylaxis) of childhood leukemia and brain tumor include behavioral changes, poor school performance, memory loss, intellectual decline, growth retardation, hormonal disturbances, and abnormal CT scans (cerebral atrophy, ventricular dilatation, intracerebral calcifications). Delays in intellectual development (deficits in IQ, memory, attention, visuospatial ability) (Fletcher et al., 1988) or declines in cognitive functioning in leukemia survivors (Ochs et al., 1991) were observed after radiation or chemotherapy without cranial irradiation, respectively. In addition, children younger than 4 years may be particularly vulnerable to the neurotoxic effects of cranial radiotherapy and/or chemotherapy (Moore et al., 1986; Jannoun et al., 1983). For most agents, high-dose therapy, combination chemotherapy, concomitant cranial radiotherapy, and intracarotic or intrathecal injections are more likely to produce neurological complications than standard oral or intravenous therapy. Any portion of the nervous system can be damaged. As cancer patients are treated more aggressively, receive more chemotherapy, and live longer, and as new chemotherapeutic agents are developed and existing agents are used more intensively or in novel ways, neurological complications of cancer chemotherapy will become more common, serious, and complex.

Most neurological conditions for which the patient seeks general medical care are due to readily demonstrated disease processes. It is the task of the clinician to develop a neurological method of analysis that will result in accurate diagnosis of the site of the disorder and of its likely cause. Only after accurate diagnosis an effective management and treatment of the disease is possible. Some techniques for diagnosis, of neurodegeneration in patients have been developed such as positron emission tomography (PET), single photon emission computed tomography (SPECT) and nuclear magnetic resonance spectroscopy (NMRS) making it possible to study brain function and structure. Most neurological diseases, however, are still diagnosed clinically on the basis of exclusion of other forms of disorders and in several cases it is even not possible to discriminate between different neurological disorders. The lewy body type of dementia or Lewy Body Dementia (LBD), for example, which is sensitive to neuroleptics, is clinically very difficult to differentiate from Alzheimer's disease (McKeith et al., 1996; Ballard et al., 1998). Most patients (more than 75%) are neuropathological defined as Alzheimer's disease patients while it is estimated that 15 to 25 % of the clinical diagnosed Alzheimer's disease patients have Lewy Body dementia (Hooten et al., 1998). As Lewy Body dementia is more susceptible to acetylcholinesterase treatment, differentiation of Lewy Body dementia from Alzheimer's disease is essential for optimization of treatment (Levy et al., 1994; Perry et al., 1994; Wilcock et al., 1994).
Also frontal temporal lobe dementia is often misdiagnosed as other types of dementia or other psychiatric disorders since the symptoms of frontal temporal lobe dementia can also be observed in other disorders.
There is no clear-cut difference between vascular disease and Alzheimer's disease and also here the risk of misdiagnosing is evident. As pharmacological treatment of vascular disease is possible, an early correct diagnosis is crucial.
Also recognition and treatment of brain damage caused by inducing agents such as certain chemical compounds, irradiation, chemotherapy or hypoxic-ischemic events, remains a frequent and important clinical problem for most neurologists. In the cases where clinical diagnosis is doubtful, definitive diagnosis can only be done irrevocably by neuropathologic examination. As such, an accurate and differential diagnosis of neurodegeneration is only possible post mortem.
Therefore, a method for the early detection of neurological disorders in patients and for the monitoring of neurological changes induced by different agents, would be of great help for determining whether exposure to the inducing agent can be continued, whether appropriate doses and drugs are being used in individual patients and for starting the right treatments.

A number of neurological markers have recently become available which reflect conditions of the central nervous system (CNS), relating to cell death, axon growth/re-induction, inflammation and/or blood-brain barrier dysfunction.
The microtubule-associated protein tau, for example, is a major protein component of paired helical filaments (PHF) and neurofibrillar tangles (11FT) (Brion et al., 1985; Delacourte and Defossez, 1986; Grundke-Iqbal et al., 1986; Kosik et al., 1986; Wood et al., 1986; Kondo et al., 1988). Tau protein exists in different isoforms, of which 4 to 6 are found in adult brain but only 1 isoform is detected in fetal brain. The diversity of the isoforms is generated from a single gene on human chromosome 17 by alternative mRNA splicing (Himmler, 1989; Goedert et al., 1989; Andreadis et al., 1992). The most striking feature of tau protein, as deduced from molecular cloning, is a stretch of 31 or 32 amino acids, occurring in the carboxy-terminal part of the molecule, which can be repeated either 3 or 4 times. Additional diversity is generated through 29 or 58 amino acid-long insertions in the NH₂-terminal part of tau molecules (Goedert et al., 1989). In vivo tau promotes microtubule assembly and stability in the axonal compartment of neurons by interactions involving its microtubule binding domain which is localized in the repeat region of tau (255-381) (Lewis et al., 1988). In normal circumstances adult brain contains 2 - 3 mole phosphate per mole of tau (Selden and Pollard, 1983; Ksiezak-Reding et al., 1992). Phosphorylation of different sites in normal tau as studied in rat and humans is dependent on the developmental state (Lee et al., 1991; Bramblett et al., 1993; Goedert et al., 1993). Tau variants of 60, 64 and 68 kDa arising as a consequence of phosphorylation have been detected in brain areas showing neurofibrillary tangles (Delacourte et al., 1990; Goedert et al., 1992; Flament et al., 1990, Greenberg and Davies, 1990). These brains contain 6-8 mole phosphate per mole tau (Ksiezak-Reding et al., 1992). In tau isolated from PHF (PHF-tau), phosphorylation occurs at several positions (Iqbal et al., 1989; Lee et al., 1991; Hasegawa et al., 1992). Sofar, the detection of phospho-tau in brain extracts, either via antibodies (Mab Alz50: Ghanbari et al., 1990; Mab Ab423: Harrington et al., 1991; Mab AT120: Vandermeeren et al., 1993; Mab AT180; Mab AT270: International application published under WO 95/17429 and Mab AT8: International application published under WO 93/08302), or via the change in molecular weight (Flament et al., 1990), or else by functional assay (Bramblett et al., 1992) has been used to discriminate dementia with altered cytoskeletal properties from normal aged subjects or from patients with other types of dementia. A combination of monoclonal antibodies each recognizing non-phosphorylated epitopes of tau has been used to detect the presence of tau and PHF-tau in cerebrospinal fluid (Van de Voorde et al., 1995).
The gamma-subunit of neuron-specific enolase (NSE) is a major constituent of neuronal cytosol (Kato et al., 1981). NSE represent 3% of total soluble brain protein. In adults, it is considered to be useful in evaluating active neuronal damage of ischemic, infectious, or tumoral origin (Garcia et al., 1994). NSE in serum of children has not been studied. Nara et al. (1988) showed that a high NSE level in cerebrospinal fluid or serum is correlated with poor outcome and death in comatose children. However, increased serum NSE is not necessarily of CNS origin. Several tissues, including peripheral neurons, endocrine glands, lymphocytes, red blood cells, and platelets contain NSE (Kaiser, 1989), which may compromise the use of this marker alone.
β-amyloid, a 40-43 amino acids long peptide, is derived via proteolytic cleavage from a large precursor protein, called amyloid precursor protein or APP. Amyloid is produced during metabolism of normal cells. The amyloid peptide exhibits a high degree of heterogeneity. Two major forms of β-amyloid have been identified, β-amyloid₍₁₋₄₀₎ and β-amyloid₍₁₋₄₂₎. β-amyloid₍₁₋₄₂₎ is a major constituent of the neuritic plaques of Alzheimer's disease, Down's syndrome and normal aged brains. It might be neurotoxic and it is known to increase the vulnerability of neurons to other insults. In addition, low concentrations of soluble amyloid can induce cholinergic hypoactivity that is not dependent on concurrent neurotoxicity. Acetylcholine plays a critical role in cognitive processes (Auld et al., 1998). The development of high affinity monoclonal antibodies specifically recognizing well-defined epitopes of the peptide has lead to a simple test for the β-amyloid₍₁₋₄₂₎ peptide in unconcentrated cerebrospinal fluid (Citron et al., 1997; Johnson-Wood et al., 1997). This test may also prove to be of value in monitoring long-term effects of drugs, irradiation, or chemical substances interfering with APP processing.
Growth associated protein-43 (GAP-43), also called neuromodulin or B-50, is a nervous tissue specific protein, primarily localized to the axons and presynaptic terminals. GAP-43 is considered to play a major role in neuronal growth, neurite formation, and in regeneration and neuronal sprouting (Skene and Willard, 1981; Basi, 1987; Benowitz et al., 1989; Mercken et al., 1992a). Synapse proteins have different roles in synapse function. Proteins such as synapsin are important in determining the amount of vesicles available for fusion, while Rab3a and rabphilin are important in targeting the vesicles to the membrane. The docking process is determined by a molecular complex of synaptobrevin, SNAP25, Sec and syntaxin, while it is believed that CSP and synaptotagmin play an important role in the Ca²⁺-dependent release of the content of the vesicle. Alpha-synuclein is abundant in synapses of the substantia nigra and basal ganglia, and belongs to a family of proteins including α-synuclein and γ-synuclein.
Such intracellular markers that are present and stable in body fluids and that reflect the metabolic state of neurons in the central nervous system, might be useful in early recognition of neurodegeneration, even before clinical signs are present. A biochemical index of neuronal function that could be used, possibly in combination which other diagnostic methods, would be of a great help to improve the clinical diagnostic accuracy and therapeutic monitoring of neurodegeneration.

Alzheimer's disease is characterized by abundant extracellular senile plaques, intracellular tangles, and synapse loss. Tau and β-amyloid₍₁₋₄₂₎ are essential components of respectively these tangles and plaques, the two diagnostic structures in the neuropathological examination of AD. Both tau and β-amyloid₍₁₋₄₂₎ have been detected in cerebrospinal fluid (CSF) and it is now well-established that CSF-tau and CSF-β-amyloid₍₁₋₄₂₎ can be used as neurological markers for Alzheimer's disease, although it is not yet known how changes in CSF levels relate to the pathophysiology of Alzheimer's disease. CSF-tau is increased in Alzheimer patients compared to age-matched controls and relates to the number of tangles in the brain, while β-amyloid₍₁₋₄₂₎ is reduced in Alzheimer's disease. β-amyloid₍₁₋₄₂₎ is probably not related to plaque formation as it is found reduced in dementia without senile, diffuse plaques such as frontal lobe dementia. Although studies on brain tissue suggest a relationship for plaques and certainly tangles to the degree of dementia, the levels of CSF-tau and CSF-β-amyloid₍₁₋₄₂₎ are not consistently related to the degree of dementia, as defined by the Mini-Mental State, and overlap with other types of dementia occurs as well. The use of β-amyloid₍₁₋₄₀₎ as a neurological marker in addition to tau and β-amyloid₍₁₋₄₂₎ (Shoji et al., 1998) is not improving the diagnostic assay for Alzheimer's disease as the level of β-amyloid₍₁₋₄₀₎ in the Alzheimer's disease patients does not change compared to the normal controls (Motter et al., 1995).
Studies on brain GAP-43 have suggested decreased levels in the frontal cortex in Alzheimer's disease but increased levels in other regions (Coleman et al., 1992). No study has been performed on GAP-43 in body fluids of patients with dementia disorders.
Another important structural alteration in brains of AD patients is synapse loss. In fact, recent studies measuring the amount of tangles, plaques and synapse loss suggest that synapse loss is the major correlate of the degree of dementia (Terry et al., 1991). In the latter study reduction of synaptophysin immunoreactivity was observed. A similar reduction has been documented for other synapse proteins: synaptotagmin, Rab3a, synaptobrevin and syntaxin (Blennow et al., 1996; Davidsson et al., 1996; Shimohama et al., 1997; Ferrer et al., 1998). Also for several forms of Parkinson disease there are strong indications that synapse proteins play a pathological role.
Two mutations in α-synuclein were detected in two rare forms of Familial Parkinson disease and α-synuclein was characterized as a major component in lewy bodies. Lewy body formation in vivo may result from synuclein accumulation, which may be the consequence of a reduction in the fast axonal transport or overexpression of synuclein (Jensen et al., 1998). As synapse proteins appear to be one of the major correlates of the degree of dementia (Terry et al., 1991), it would be useful to provide methods for the detection and quantitation of synapse proteins in body fluids. The presence and quantification of synapse proteins in CSF has not yet been fully explored. Chromogranin has already been used as a marker for synapse loss in CSF, but a reduction was only shown in 'pure' or type I Alzheimer's disease (Blennow et al., 1995). Shortly thereafter synaptotagmin I was shown to be present in CSF (Davidsson et al., 1996). In this study it was demonstrated that synaptotagmin is selectively reduced in the left hippocampal formation and Brodmann area 9 of the frontal cortex of Alzheimer's disease patients. Based on pools of CSF it was suggested that this reduction could also be present in CSF, but it has not been quantified. Davidsson et al. (1996) were not able to detect Rab3a and synaptophysin in CSF.

Also for neurodegeneration induced by exposure to certain chemical compounds, irradiation, chemotherapy or hypoxic-ischemic events, no accurate diagnostic tools are available. Perinatal asphyxia may be associated with neurological sequelae. Early and accurate evaluation of the severity of brain damage after a hypoxic-ischemic event, however, remains one of the most difficult problems in neonatal care. To date, clinical, electroencephalographic, and neuroradiologic evaluation, together with cerebral blood flow studies are the most readily available methods. As it has become increasingly evident that modified brain metabolic activity is reflected by changes in components in the CSF, the detection of CSF neurological markers may complement clinical data in the evaluation of hypoxic-ischemic events (Garcia-Alix et al., 1994). The link between CSF neurological markers and behavioral changes at a later age after chemotherapy, irradiation or a hypoxic-ischemic event has never been assessed.

### AIMS OF THE INVENTION

The aim of the present invention is to provide methods for specific detection, quantification and/or differential diagnosis of neurodegeneration in an individual.
It is another aim of the present invention to provide a method for a more specific detection, quantification and/or differential diagnosis of Alzheimer's disease in an individual.
It is another aim of the present invention to provide a method for a more specific detection, quantification and/or differential diagnosis of Lewy Body Disease in an individual.
It is another aim of the present invention to provide a method for a more specific detection, quantification and/or differential diagnosis of Parkinson disease in an individual.
It is another aim of the present invention to provide a method for a more specific detection, quantification and/or differential diagnosis of frontal temporal lobe dementia in an individual.
It is another aim of the present invention to provide a method for the differentiation of Alzheimer's disease versus Parkinson disease.
It is another aim of the present invention to provide a method for the differentiation of Alzheimer's disease versus Lewy Body dementia.
It is another aim of the present invention to provide a method for the specific detection or quantification of vascular problems in Alzheimer's disease and for the differential diagnosis of different forms of Alzheimer's disease.
It is another aim of the present invention to provide a method for the diagnosis of neurodegeneration induced by chemotherapy or by exposure to chemical compounds or irradiation.
It is another aim of the present invention to provide a method for the diagnosis of neurodegeneration induced by chemotherapy in individuals treated for leukemia or brain tumor.
It is another aim of the present description to provide a new method for the detection of the synapse protein Rab3a in cerebrospinal fluid.
It is another aim of the present description to provide a new method for the detection of Rab3a in cerebrospinal fluid that allows a more specific detection, quantification and/or differential diagnosis of neurodegeneration in an individual.
It is another aim of the present description to provide a new method for the detection of Rab3a in cerebrospinal fluid that allows a more specific detection, quantification and/or differential diagnosis of Alzheimer's disease.
It is another aim of the present description to provide a new method for the detection of the synapse protein α-synuclein in cerebrospinal fluid.
It is another aim of the present description to provide a new method for the detection of α-synuclein in cerebrospinal fluid that allows a more specific detection, quantification and/or differential diagnosis of neurodegeneration in an individual.
It is another aim of the present description to provide a new method for the detection of α-synuclein in cerebrospinal fluid that allows a more specific detection or quantification of Alzheimer's disease and/or Lewy Body Disease and/or that allows the differential diagnosis of Alzheimer's disease versus Lewy Body Disease.
It is another aim of the present invention to provide a diagnostic kit for performing a method as described above.
It is another aim of the present invention to provide a method for therapeutic monitoring and/or determination of the effectiveness of a certain treatment.

### DETAILED DESCRIPTION OF THE INVENTION

The present invention relates to methods for specific detection, quantification and/or differential diagnosis of neurodegeneration in an individual. These methods involve the determination of the level of at least three neurological markers in one or more body fluid samples of said individual, whereby the type and degree of neurodegeneration is reflected by a quantitative change in the level of all of said neurological markers compared to a control sample.
As can be seen from the present examples, by use of at least three neurological markers a more specific and sensitive detection of a number of neurodegenerative conditions was obtained. It is also apparent that by use of at least three neurological markers it became possible to discriminate between a number of neurodegenerative conditions that could not be differentiated on the basis of clinical diagnosis.
The term's neurodegeneration and neurodegenerative condition used in the present application stand for the same and are used interchangeable throughout the application. These terms include any condition of the brain that is associated with a neuronal malfunctioning. Various diseases associated with neurodegeneration are cited in Wilson et al. (1991). They include Alzheimer's disease, stroke (Focal brain injury), diffuse brain injury, vascular disease, Parkinson disease, Lewy Body Disease, Creutzfeld Jacob Disease, Frontal temporal lobe dementia, Guilain Barré Syndrome, Multiple Sclerosis, Normal Pressure Hydrocephalus, Amyotrophic Lateral Sclerosis,
Schizophrenia, Depression, Neurolathyrisme, Epilepsy and Asphyxia. However, this list is not complete. Other diseases known to be associated with neuronal malfunctioning are included as well. Neurodegeneration also includes any kind of brain damage or any condition of the brain that is associated with a neuronal malfunctioning and which is caused by a specific inducing agent.
In a preferred embodiment of the present invention, the neurodegenerative condition to be specifically detected, quantified and/or differentially diagnosed is chosen from the group consisting of Alzheimer's disease, Lewy Body Disease, Parkinson disease and frontal temporal lobe dementia. Lewy Body Disease is used for any disease showing lewy bodies in the brainstem, basal forebrain, hypothalamic nuclei and/or neocortex. Lewy Body Disease includes Parkinson disease, multiple system atrophy and Lewy Body dementia.
In another preferred embodiment of the present invention, the neurodegenerative condition to be specifically detected, quantified and/or differentially diagnosed is induced by hypoxic-ischemic events, chemotherapy, radiotherapy, or by exposure to chemical compounds or irradiation. More particularly, neurodegeneration can be induced by chemotherapy or radiotherapy during the treatment of leukemia or brain tumor.
However, this list of neurodegenerative conditions is not complete. Other conditions in which a malfunctioning of the brain occurs, are included as well.
The expression "specific detection of neurodegeneration" as used in the present invention means that a higher sensitivity and specificity is obtained for the association of a certain disease or a certain cause of neurological disorder with a certain neurodegenerative condition than would be obtained when less than three neurological markers were used for diagnosis.
The expression "quantification of neurodegeneration" as used in the present invention means that the degree of neuronal malfunctioning due to a certain neurodegenerative condition is determined. The expression "differential diagnosis of neurodegeneration" as used in the present invention refers to the discrimination between various neurodegenerative conditions in this way that a certain disease or a certain cause of neurological disorder is associated with a certain neurodegenerative condition.
The specific detection, quantification and/or differential diagnosis of neurodegeneration in an individual is accomplished by the detection of at least three different neurological markers in one or more body fluid samples of said individual; making use of an immuno-assay comprising the steps of:
- bringing one or more body fluid samples obtained from said individual into contact with at least three different antibodies (primary antibodies or capturing antibodies) recognizing each a different neurological marker in the body fluid samples, under conditions being suitable for producing an antigen-antibody complex; and
- detecting the immunological binding of said antibodies to said body fluid samples;
- inference on the level of said neurological markers in said body fluids, the type and degree of neurodegeneration being reflected in the quantitative changes in the level of all of said neurological markers compared to control samples.
The process for the detection of the immunological binding can then be carried out by bringing together said antigen-antibody complex formed by the antigen and the antibody recognizing one of the neurological markers with:
a) a secondary antibody (or detector antibody)
   * that can be a monoclonal antibody recognizing a specific epitope of the antigen-antibody complex but not recognizing the primary antibody alone, or
   * which can be a polyclonal antibody recognizing a specific epitope of the antigen-antibody complex but not recognizing the primary antibody alone, with said polyclonal antibody being preferably purified by immunoaffinity chromatography using immobilized neurological marker or neurological marker-primary antibody complex;
b) a marker either for specific tagging or coupling with said secondary antibody, with said marker being any possible marker known to the person skilled in the art;
c) appropriate buffer solutions for carrying out the immunological reaction between the antibodies and the body fluid samples, between the secondary antibody and the neurological marker-primary antibody complex and/or the bound secondary antibody and the marker on the other hand; and
d) possibly also, for standardization purposes, purified proteins or synthetic peptides reactive with the antibodies used for detection of the neurological markers.
Advantageously, the antibodies used in the invention are in an immobilized state on a suitable support. The antibodies may be present on up to three (or more in case more than 3 neurological markers are detected) different supports or on the same support. When the antibodies are present on the same support (for instance in one microtiter plate well), the immunological binding of each of them may be detected by a specific marker. Alternatively, the antibodies may be present on distinct locations of the same support. In the latter case, detection may occur with a general marker that detects the immunological binding of any of these antibodies. Advantageously, the secondary antibody itself carries a marker or a group for direct or indirect coupling with a marker. Alternatively, the present process may be put into practice by using any other immunoassay format known to the person skilled in the art.
The term "epitope" refers to that portion of the antigen-antibody complex that is specifically bound by an antibody-combining site. Epitopes may be determined by any of the techniques known in the art or may be predicted by a variety of computer prediction models known in the art.
The expression "recognizing", ''reacting with", "immunological binding" or "producing an antigen-antibody complex" as used in the present invention is to be interpreted that binding between the antigen and antibody occurs under all conditions that respect the immunological properties of the antibody and the antigen.
The term body fluids refers to all fluids that are present in the human body including but not limited to blood, lymph, urine and cerebrospinal fluid (CSF).
In a specific embodiment, the present invention relates to a method as described above wherein the body fluid sample is chosen from the group consisting of a cerebrospinal fluid sample and a blood sample. The blood sample can include the whole sample as taken from the patient. More preferably the blood sample includes a plasma sample or a serum sample.
In the methods of the present invention it is also possible to detect the same marker in two different body fluids (in combination with the detection of at least one other marker) or to detect the same marker in three different body fluids. For example, two neurological markers are detected in cerebrospinal fluid and one of these neurological markers is also detected in plasma. As shown in the example section, also detection of the same marker in two different body fluids leads to a more specific and sensitive detection and to a better differential diagnosis of neurodegeneration compared to detection of this maker in only one body fluid.
The neurological markers that are detected in the method of the present invention can be any protein associated with certain types of neuronal cells or cell function, of which the level in one or more body fluids under conditions of neurodegeneration is indicative for the disease process or the cause of neurological disorder. Some neurological markers are elevated and others are reduced in one or more body fluids under a certain neurological condition. Any possible combination of 3, 4, 5, 6, 7, 8 or more neurological markers that have an altered level in a certain body fluid under a certain neurological condition can be used for the specific detection, quantification and/or differential diagnosis of said neurological condition in an individual.
Possible neurological markers used for specific detection, quantification and/or differential diagnosis of neurodegeneration include: tau, neuron-specific enolase (NSE), beta-amyloid₍₁₋₄₂₎, beta-amyloid₍₁₋₄₀₎, neuromodulin, synapse proteins (such as Rab3a, SNAP25, α-synuclein, synapsin, synaptotagmin, synaptobrevin, syntaxin, rabphilin, n-sec, cystein string protein and others), glial fibrillary acidic protein (GFAP), S100, IL6, TNF, IL1, IL2, neurofilament (NF), myelin basic protein (MBP) and 14-3-3. However, this list is not complete. Other neurological markers that are indicative for a certain disease process or cause of neurological disorder can be used as well. The behavior of some of these neurological markers in body fluids of patients suffering from different neurological diseases and brain damage is shown in table 2.
Any monoclonal or polyclonal antibody prepared or present in the art that specifically recognizes one of the above-mentioned neurological markers, can be used for the detection of the neurological marker. Antibodies specifically recognizing tau include Alz50 (Ghanbari et al., 1990), Ab423 (Harrington et al., 1991), AT8 (International application published under WO 93/08302), AT120 (Vandermeeren et al., 1993); AT180 and AT270 (International application published under WO 95/17429) and AT100 (International application published under WO 96/04309). But also other antibodies known in the art specifically recognizing tau can be used. Antibodies that specifically recognize NSE include 10Cl and 2E7 and others from Innogenetics (Gent, Belgium), commercially available antibodies such as can be obtained from Dako (Glostrup, Denmark; Cat No BBS/NC/VI-H14), from Biogenex (San Ramon, CA, USA; Cat Nos MA055-5C and AM055-5M), from RDI (Flanders, NJ, USA; Cat No RDI-TRK4N6), from Roche Diagnostic Systems (Basel, Switzerland; Cat No 07 34373), from Immunosource (Brussels, Belgium; Cat Nos CLA 73/5 and CR7041M) and from Cortex Biochem (San Leandro, CA, USA; Cat No CR7047). This list of antibodies recognizing NSE is not complete and other antibodies, commercially available or described in the art, that recognize NSE, can be used as well. Antibodies that specifically recognize β-amyloid include 2H3, 8E5 (Johnson-Wood et al., 1997), 10H3 (Majocha et al., 1992; Friedland et al., 1994), 2G3 (Citron et al., 1996), BA-27 and BC-05 (Suzuki et al., 1994), BNT77 (Asami-Odaka et al., 1995), 369.2B (König et al., 1996), 22C11 (Lannfelt et al., 1995), 6E10 (Kim et al., 1990) and AMY-33 (Stern et al., 1990). But any other antibodies known in the art specifically recognizing β-amyloid can be used as well. Antibodies that specifically recognize neuromodulin include NM2 (Oestreicher et al., 1994), NM4 (Six et al., 1992), NM1, NM3, NM6, NM7 and NM8 (Mercken et al., 1992a). But any other antibody known in the art that specifically recognizes neuromodulin can be used as well. Antibodies specifically recognizing Rab3a include commercially available antibodies such as can be obtained from Transduction Labs (Lexington, KY, USA; Cat No R35520). Also other antibodies commercially available or described in the art that recognize Rab3a can be used. Antibodies specifically recognizing SNAP25 include commercially available antibodies such as can be obtained from Serotec (Oxford, UK; Cat No SP12), from Sternberger Monoclonals Inc. (Distributed by Affinity Research Products Lim., Mamhead, Exeter, UK; Cat No SMI-81), from Chemicon (Temecula, CA, USA; Cat No MAB33 1) and from Transduction Labs (Lexington, KY, USA; Cat No S35020). This list of antibodies recognizing SNAP25 is not complete and other antibodies commercially available or described in the art that recognize SNAP25 can be used as well. Antibodies specifically recognizing α-synuclein include commercially available antibodies such as can be obtained from Transduction Labs (Lexington, KY, USA; Cat No S63320). Also other antibodies commercially available or described in the art that recognize α-synuclein can be used. Also for the specific detection of other synapse proteins that can possibly be used as neurological markers, various antibodies are commercially available and/or known in the art. Antibodies specifically recognizing S 100 include commercially available antibodies such as can be obtained from Biogenex (San Ramon, CA, USA; Cat Nos MA058-C and AM058-5M) and from Innogenetics (Gent, Belgium; Cat No M-011). This list of antibodies recognizing S100 is not complete and other antibodies commercially available or described in the art that recognize S100 can be used as well. Antibodies specifically recognizing 14-3-3 include commercially available antibodies such as can be obtained from Santa Cruz Biotechnology (Santa Cruz, CA, USA, Cat No sc-1657) and from Transduction Labs (Lexington, KY, USA; Cat No F46820).
This list of antibodies recognizing 14-3-3 is not complete and other antibodies commercially available or described in the art that recognize 14-3-3 can be used as well. Antibodies specifically recognizing neurofilament include commercially available antibodies such as can be obtained from Innogenetics (Gent, Belgium; Cat Nos M-011 and M-005) and from Alexis (Läufelfingen, Switzerland; Cat Nos BC-4000-A-L001 and BC-4010-A-L001). This list of antibodies recognizing neurofilament is not complete and other antibodies commercially available or described in the art that recognize neurofilament can be used as well.
Also fragments derived from these monoclonal antibodies such as Fab, F(ab)'₂, ssFv ("single chain variable fragment") and other antibody like constructs that retain the variable region of the antibody, providing they have retained the original binding properties, can be used in a method of the present invention. Such fragments are commonly generated by, for instance, enzymatic digestion of the antibodies with papain, pepsin, or other proteases. It is well known to the person skilled in the art that monoclonal antibodies, or fragments thereof, can be modified for various uses. Also miniantibodies and multivalent antibodies such as diabodies, triabodies, tetravalent antibodies and peptabodies can be used in a method of the invention. The preparation and use of these fragments and multivalent antibodies has been described extensively in International Patent Application WO 98/29442.
The monoclonal antibodies used in a method of the invention may be humanized versions of the mouse monoclonal antibodies made by means of recombinant DNA technology, departing from the mouse and/or human genomic DNA sequences coding for H and L chains or from cDNA clones coding for H and L chains. Alternatively the monoclonal antibodies used in a method of the invention may be human monoclonal antibodies. The term "humanized antibody" means that at least a portion of the framework regions of an immunoglobulin is derived from human immunoglobulin sequences.

The antibodies used in a method of the present invention may be labeled by an appropriate label of the enzymatic, fluorescent, or radioactive type.
In a specific embodiment of the invention, at least one of the neurological markers to be detected in a method as described above, is chosen from the group consisting of tau, phospho-tau, β-amyloid₍₁₋₄₂₎, β-amyloid₍₁₋₄₀₎, neuromodulin, neuron-specific enolase and/or synapse proteins. Any possible combination of 3, 4, 5, 6, 7, 8 or more markers of which one is chosen from the above group can be used for the specific detection, quantification and/or differential diagnosis of neurodegeneration in an individual. It is clear that also more than one (i.e. 2, 3, 4, 5, 6, 7 or more) or even all neurological markers to be used in a method of the present invention can be chosen from the above group.
In a more specific embodiment of the invention, one, more preferably two, most preferably three of the neurological markers to be detected in a method as described above, are chosen from the following groups:
- tau, β-amyloid_{(1,42)}, and neuromodulin; or
- tau, neuron-specific enolase and neuromodulin; or
- tau, phospho-tau and β-amyloid₍₁₋₄₂₎; or
In another more specific embodiment, tau is detected in one body fluid, preferably CSF and β-amyloid₍₁₋₄₂₎ is detected in 2 different body fluids, preferably CSF and plasma.
In another more specific embodiment of the invention, at least one of the neurological markers to be detected in a method as described above, is a synapse protein chosen from the group consisting of Rab3a, SNAP25 and α-synuclein. Any possible combination of 3, 4, 5, 6, 7, 8 or more markers of which one is chosen from the above group of synapse proteins can be used for the specific detection, quantification and/or differential diagnosis of neurodegeneration in an individual.
Accordingly, the present description also relates to a new method for the detection of Rab3 a in cerebrospinal fluid, comprising at least the following steps:
- bringing a cerebrospinal fluid sample obtained from an individual into contact with a monoclonal antibody (primary antibody or capturing antibody) recognizing Rab3a under conditions being suitable for producing an antigen-antibody complex; and
- detecting the immunological binding of said antibody to said cerebrospinal fluid sample.
Although antibodies specifically recognizing Rab3a are available for the detection of Rab3a in brain tissue, detection of Rab3a in cerebrospinal fluid was not that evident. By their method used, Davidsson et al. (1996) were not able to detect Rab3a in cerebrospinal fluid.
Any antibody that allows specific detection of Rab3a in cerebrospinal fluid can be used in this new method. A preferred monoclonal antibody for use in the method of the invention can be obtained from Transduction Labs (Lexington, KY, USA; Cat No R35520).
Advantageously, the monoclonal antibody used in the invention is in an immobilized state on a suitable support. Alternatively, the present process may be put into practice by using any other immunoassay format known to the person skilled in the art.
The process for the detection of the immunological binding can then be carried out by bringing together said antigen-antibody complex formed by the antigen and the antibody recognizing Rab3 a with:
a) a secondary antibody (or detector antibody)
   * which can be a monoclonal antibody recognizing an epitope of the antigen-antibody complex but not recognizing the primary antibody alone; or
   * which can be a polyclonal antibody recognizing an epitope of the antigen-antibody complex but not recognizing the primary antibody alone, with said polyclonal antibody being preferably purified by immunoaffinity chromatography using immobilized Rab3a or Rab3a-primary antibody complex.
b) a marker either for specific tagging or coupling with said secondary antibody, with said marker being any possible marker known to the person skilled in the art;
c) appropriate buffer solutions for carrying out the immunological reaction between the antibodies and the cerebrospinal fluid sample, between the secondary antibody and the neurological marker-primary antibody complex and/or between the bound second antibody and the marker; and
d) possibly also, for standardization purposes, purified proteins or synthetic peptides reactive with the antibodies that recognize Rab3a.
As illustrated in the present examples, a polyclonal Rab3a serum may be used as a detector antibody.
Advantageously, the secondary antibody itself carries a marker or a group for direct or indirect coupling with a marker.
The present description also relates to a new method for the detection of SNAP25 in cerebrospinal fluid comprising at least the following steps:
- bringing a cerebrospinal fluid sample obtained from an individual into contact with a monoclonal antibody (primary antibody or capturing antibody) recognizing SNAP25 under conditions being suitable for producing an antigen-antibody complex; and
- detecting the immunological binding of said antibody to said cerebrospinal fluid sample.
Although antibodies specifically recognizing SNAP25 are available for the detection of SNAP25 in brain tissue, detection of SNAP25 in cerebrospinal fluid was not that evident and has not been shown before.
Any antibody that allows specific detection of SNAP25 in cerebrospinal fluid can be used in this new method. A preferred monoclonal antibody for use in the method of the invention can be obtained from Serotec (Oxford, UK; Cat No SP12), from Sternberger Monoclonals Inc. (Distributed by Affinity Research Products Lim., Mamhead, Exeter, UK; Cat No SMI-81), from Chemicon (Temecula, CA, USA; Cat No MAB331) or from Transduction Labs (Lexington, KY, USA; Cat No S3S020).
Advantageously, the monoclonal antibody used in the invention is in an immobilized state on a suitable support. Alternatively, the present process may be put into practice by using any other immunoassay format known to the person skilled in the art.
The process for the detection of the immunological binding can then be carried out by bringing together said antigen-antibody complex formed by the antigen and the antibody recognizing SNAP25 with:
a) a secondary antibody (or detector antibody)
   * which can be a monoclonal antibody recognizing an epitope of the antigen-antibody complex but not recognizing the primary antibody alone; or
   * which can be a polyclonal antibody recognizing an epitope of the antigen-antibody complex but not recognizing the primary antibody alone, with said polyclonal antibody being preferably purified by immuno-affinity chromatography using immobilized SNAP25 or SNAP25-primary antibody complex.
b) a marker either for specific tagging or coupling with said secondary antibody, with said marker being any possible marker known to the person skilled in the art;
c) appropriate buffer solutions for carrying out the immunological reaction between the antibodies and the cerebrospinal fluid sample, between the secondary antibody and the neurological marker-primary antibody complex, and/or the bound secondary antibody and the marker; and
d) possibly also, for standardization purposes, purified proteins or synthetic peptides reactive with the antibodies that recognize SNAP25.
As illustrated in the present examples, a polyclonal SNAP25 serum may be used as a detector antibody.
Advantageously, the secondary antibody itself carries a marker or a group for direct or indirect coupling with a marker
The present description also relates to a new method for the detection of α-synuclein in cerebrospinal fluid comprising at least the following steps:
- bringing a cerebrospinal fluid sample obtained from an individual into contact with a monoclonal antibody (primary antibody or capturing antibody) recognizing α-synuclein under conditions being suitable for producing an antigen-antibody complex; and
- detecting the immunological binding of said antibody to said cerebrospinal fluid sample.
Although antibodies specifically recognizing α-synuclein are available for the detection of α-synuclein in brain tissue, detection of α-synuclein in cerebrospinal fluid was not that evident.
As the presence of α-synuclein in cerebrospinal fluid had never been reported before, it was even doubtful if any α-synuclein would be present in CSF. Firstly, the present inventors were able to show that α-synuclein is present in cerebrospinal fluid. In addition, an accurate method was developed for the quantitative detection of α-synuclein in cerebrospinal fluid. The present inventors also showed that α-synuclein is altered under certain neurodegenerative conditions, including but not limited to Lewy Body Disease.
Any antibody that allows specific detection of α-synuclein in cerebrospinal fluid can be used in this new method. A preferred monoclonal antibody for use in the method of the invention can be obtained from Transduction Labs (Lexington, KY, USA; Cat No R35520).
Advantageously, the monoclonal antibody used in the invention is in an immobilized state on a suitable support. Possibly this immobilized state can be a microtiter plate, coated or not coated with anti-IgG. Alternatively, the present process may be put into practice by using any other immunoassay format known to the person skilled in the art.
The process for the detection of the immunological binding can then be carried out by bringing together said antigen-antibody complex formed by the antigen and the antibody recognizing α-synuclein with:
a) a secondary antibody (or detector antibody)
   * which can be a monoclonal antibody recognizing an epitope of the antigen-antibody complex but not recognizing the primary antibody alone; or
   * which can be a polyclonal antibody recognizing an epitope of the antigen-antibody complex but not recognizing the primary antibody alone, with said polyclonal antibody being preferably purified by immuno-affinity chromatography using immobilized α-synuclein or α-synuclein -primary antibody complex.
b) a marker either for specific tagging or coupling with said secondary antibody, with said marker being any possible marker known to the person skilled in the art;
c) appropriate buffer solutions for carrying out the immunological reaction between the antibodies and the cerebrospinal fluid sample, between the secondary antibody and the neurological marker-primary antibody complex, and/or the bound secondary antibody and the marker; and
d) possibly also, for standardization purposes, purified proteins or synthetic peptides reactive with the antibodies that recognize α-synuclein.
Advantageously, the secondary antibody itself carries a marker or a group for direct or indirect coupling with a marker.
In a preferred embodiment these methods for the detection of Rab3a, SNAP25 and/or α-synuclein can be used in combination with a method for detection of one or more other neurological markers in order to specifically detect, quantify and/or differential diagnose neurodegeneration in an individual.
In an even more preferred embodiment, these methods for the detection of Rab3a, SNAP25 and/or α-synuclein can be used in combination with a method for detection of one or more neurological markers chosen from the group consisting of tau, phospho-tau, β-amyloid₍₁₋₄₂₎, β-amyloid₍₁₋₄₀₎, neuromodulin, neuron-specific enolase (NSE).
More particularly, the neurological markers used for the specific detection, quantification and/or differential diagnosis of neurodegeneration can be chosen from one of the following groups:
- tau, phospho-tau, NSE, β-amyloid(₁₋₄₂₎, β-amyloid₍₁₋₄₀₎, neuromodulin or Rab3a; or
- tau, phospho-tau, NSE, β-amyloid(₁₋₄₂₎, β-amyloid₍₁₋₄₀₎, neuromodulin or SNAP25; or
- tau, phospho-tau, NSE, β-amyloid(₁₋₄₂), β-amyloid(₁₋₄₀), neuromodulin or α-synuclein.
Any possible combination of 3, 4, 5, 6 or 7 markers from the above groups can be used for the specific detection, quantification and/or differential diagnosis of neurodegeneration in an individual.
In another embodiment, the methods for the detection of Rab3a, SNAP25 and/or α-synuclein can be used together for the specific detection, quantification and/or differential diagnosis of neurodegeneration. Accordingly the present invention relates to a method wherein two or three of the neurological markers are chosen from the group consisting of Rab3a, SNAP25 and α-synuclein.
A very specific embodiment relates to a method as described above for the specific detection or quantification of Alzheimer's disease and/or Lewy Body Disease and/or for the differential diagnosis of Alzheimer's disease versus Lewy Body Disease, wherein:
- at least the level of α-synuclein is determined in a cerebrospinal fluid sample; and/or
- the level of tau, β-amyloid₍₁₋₄₂₎ and α-synuclein is determined in a cerebrospinal fluid sample.
Another very specific embodiment relates to a method for the specific detection or quantification of Alzheimer's disease and/or for the differential diagnosis of Alzheimer's disease versus other dementia wherein:
- the level of tau, β-amyloid₍₁₋₄₂₎ and Rab3a is determined in a cerebrospinal fluid sample; or
- the level of tau, β-amyloid₍₁₋₄₂₎ and SNAP25 is determined in a cerebrospinal fluid sample.
Another very specific embodiment relates to a method for the specific detection or quantification of Alzheimer's disease and/or Parkinson's disease and/or for the differential diagnosis of Alzheimer's disease versus Parkinson's disease wherein the level of tau, β-amyloid₍₁₋₄₂₎ and neuromodulin is determined in a cerebrospinal fluid sample.
Another very specific embodiment relates to a method for the specific detection or quantification of neurodegeneration induced by chemotherapy, exposure to chemical compounds and/or irradiation wherein the level of tau, neuron-specific enolase and neuromodulin is determined in a cerebrospinal fluid sample.
Another very specific embodiment relates to a method for the specific detection or quantification of neurodegeneration induced by chemotherapy, exposure to chemical compounds and/or irradiation in an individual treated for leukemia or brain tumor wherein the level of tau, neuron-specific enolase and neuromodulin is determined in a cerebrospinal fluid sample.
Another very specific embodiment relates to a method for the specific detection or quantification of neurodegeneration induced by prenatal asphyxia wherein at least three neurological markers are detected.
Another very specific embodiment relates to a method for the specific detection or quantification of Frontal Temporal Lobe dementia and/or for the differential diagnosis of Frontal Temporal Lobe dementia versus other dementia wherein the level of tau, phospho-tau and β-amyloid₍₁₋₄₂) is determined in a cerebrospinal fluid sample.
Another very specific embodiment relates to a method for the specific detection or quantification of vascular problems in Alzheimer's disease, for the differential diagnosis of different forms of Alzheimer's disease and/or for the differential diagnosis of Alzheimer's disease versus other dementia, wherein at least the level of:
- tau and β-amyloid₍₁₋₄₂₎ is determined quantitatively in a cerebrospinal fluid sample and the level of β-amyloid₍₁₋₄₂₎ is determined quantitatively in a plasma sample; or
- phospho-tau and β-amyloid₍₁₋₄₂₎ is determined quantitatively in a cerebrospinal fluid sample and the level of β-amyloid₍₁₋₄₂₎ is determined quantitatively in a plasma sample; or
- tau and phospho-tau is determined quantitatively in a cerebrospinal fluid sample and the level of β-amyloid₍₁₋₄₂₎ is determined quantitatively in a plasma sample; or
- tau, phospho-tau and β-amyloid₍₁₋₄₂₎ is determined quantitatively in a cerebrospinal fluid sample.
The above methods for the specific detection, quantification and/or differential diagnosis of neurodegeneration in an individual by determination of level of at least three neurological markers in body fluids of said individual, can be used alone, in combination with easily monitored neurological endpoints (e.g. leucocyte count) or in combination with the measurement of drug concentrations in plasma.

The present invention also relates to a diagnostic kit for the specific detection, quantification and/or differential diagnosis of neurodegeneration in an individual, comprising at least three antibodies each recognizing a different neurological marker in one or more body fluid samples of said individual.

More particularly, the present invention relates to a diagnostic kit for the specific detection, quantification and/or differential diagnosis of neurodegeneration in an individual, comprising at least a support such as a microtiterplate with, together or in separate wells, at least three antibodies each recognizing a different neurological marker in one or more body fluid samples of said individual.
The present invention also relates to a kit for the specific detection, quantification and/or differential diagnosis of neurodegeneration in an individual, comprising:
- a support such as a microtiterplate comprising, together or in separate wells, at least three antibodies (primary antibodies or capturing antibodies) each recognizing a different neurological marker;
- secondary antibodies (detector antibodies), each recognizing one of the neurological marker-primary antibody complexes:
   * which can be a monoclonal antibody being capable of forming an immunological complex with an epitope of the neurological marker-primary antibody complex but not with the primary antibody alone; or
   * which can be a polyclonal antibody being capable of forming an immunological complex with epitopes of the neurological marker-primary antibody complex but not with the primary antibody alone, with said polyclonal antibody being preferably purified by immunoaffinity chromatography using immobilized neurological marker or neurological marker-primary antibody complex;
- possibly, a marker either for specific tagging or coupling with said secondary antibodies;
- possibly, appropriate buffer solutions for carrying out the immunological reaction between the primary antibodies and the body fluid sample, between the secondary antibodies and the neurological marker-primary antibody complexes and/or between the bound secondary antibodies and the marker;
- possibly, for standardization purposes, purified proteins or synthetic peptides that are specifically recognized by the antibodies of the kit, used for the detection of the neurological marker.
In specific embodiments, the present invention relates to diagnostic kits as described above each designed for performing one or more of the methods as described above.
More particularly the present invention relates to a diagnostic kit as described above comprising at least antibodies that specifically recognize:
- α-synuclein; or
- tau, β-amyloid₍₁₋₄₂₎ and α-synuclein; or
- tau, β-amyloid₍₁₋₄₂₎ and Rab3a; or
- tau, β-amyloid₍₁₋₄₂₎ and SNAP25; or
- tau, β-amyloid₍₁₋₄₂₎ and neuromodulin; or
- tau, neuron-specific enolase and neuromodulin; or
- tau, phospho-tau and β-amyloid₍₁₋₄₂₎; or
- tau and β-amyloid₍₁₋₄₂₎;
The present description also relates to a kit for the detection of Rab3a in cerebrospinal fluid, comprising at least a monoclonal antibody recognizing Rab3a.
The present description also relates to a kit for the detection of Rab3a in cerebrospinal fluid, comprising at least a support such as a microtiterplate comprising a monoclonal antibody recognizing Rab3a.
More particularly, the present description relates to a kit for the detection of Rab3a in
cerebrospinal fluid, comprising:
- at least a support such as a microtiterplate comprising a monoclonal antibody recognizing Rab3a (primary antibody or capturing antibody);
- a secondary antibody (or detector antibody)
   * which can be a monoclonal antibody being capable of forming an immunological complex with an epitope of the Rab3a-primary antibody complex but not with the primary antibody alone; or
   * which can be a polyclonal antibody being capable of forming an immunological complex with an epitope of the Rab3a-primary antibody complex but not with the primary antibody alone, with said polyclonal antibody being preferably purified by immunoaffinity chromatography using immobilized Rab3a or Rab3a-primary antibody complex;
- possibly, a marker either for specific tagging or coupling with said secondary antibody;
- possibly, appropriate buffer solutions for carrying out the immunological reaction between the antibodies and the cerebrospinal fluid sample, between the secondary antibody and the Rab3a-primary antibody complex and/or between the bound secondary antibody and the marker;
- possibly, for standardization purposes, purified proteins or synthetic peptide that are
specifically recognized by the antibodies of the kit, used for the detection of Rab3a.
The present invention also relates to a kit for the detection of SNAP25 in cerebrospinal fluid, comprising at least a monoclonal antibody recognizing SNAP25.
The present description also relates to a kit for the detection of SNAP25 in cerebrospinal fluid, comprising at least a support such as a microtiterplate comprising a monoclonal antibody recognizing SNAP25.
More particularly, the present description relates to a kit for the detection of SNAP25 in cerebrospinal fluid, comprising:
- at least a support such as a microtiterplate comprising a monoclonal antibody recognizing SNAP25 (primary antibody or capturing antibody);
- a secondary antibody (or detector antibody)
   * which can be a monoclonal antibody being capable of forming an immunological complex with an epitope of the SNAP25-primary antibody complex but not with the primary antibody alone, or
   * which can be a polyclonal antibody being capable of forming an immunological complex with an epitope of the SNAP25-primary antibody complex but not with the primary antibody alone, with said polyclonal antibody being preferably purified by immunoaffinity chromatography using immobilized SNAP25 or SNAP25-primary antibody complex;
- possibly, a marker either for specific tagging or coupling with said secondary antibody;
- possibly, appropriate buffer solutions for carrying out the immunological reaction between the antibodies and the cerebrospinal fluid sample, between the secondary antibody and the SNAP25-primary antibody complex and/or between the bound secondary antibody and the marker;
- possibly, for standardization purposes, purified proteins or synthetic peptides that are specifically recognized by the antibodies of the kit, used for the detection of SNAP25.
The present description also relates to a kit for the detection of α-synuclein in cerebrospinal fluid, comprising at least a monoclonal antibody recognizing α-synuclein.
The present description also relates to a kit for the detection of α-synuclein in cerebrospinal fluid, comprising at least a support such as a microtiterplate comprising a monoclonal antibody recognizing α-synuclein.
More particularly, the present description relates to a kit for the detection of α-synuclein in cerebrospinal fluid, comprising:
- at least a support such as a microtiterplate comprising a monoclonal antibody recognizing α-synuclein (primary antibody or capturing antibody) directly linked to the microtiterplate, possibly by an anti-IgG antibody;
- a secondary antibody (or detector antibody)
   * which can be a monoclonal antibody being capable of forming an immunological complex with an epitope of the α-synuclein-primary antibody complex but not with the primary antibody alone, or
   * which can be a polyclonal antibody being capable of forming an immunological complex with an epitope of the α-synuclein-primary antibody complex but not with the primary antibody alone, with said polyclonal antibody being preferably purified by immunoaffinity chromatography using immobilized α-synuclein or α-synuclein-primary antibody complex;
- possibly, a marker either for specific tagging or coupling with said secondary antibody;
- possibly, appropriate buffer solutions for carrying out the immunological reaction between the antibodies and the cerebrospinal fluid sample, between the secondary antibody and the α-synuclein-primary antibody complex and/or between the bound secondary antibody and the marker;
- possibly, for standardization purposes, purified proteins or synthetic peptides that are specifically recognized by the antibodies of the kit, use for the detection of α-synuclein.
The present invention also relates to the use of any method or any kit as described above for therapeutic monitoring and/or determination of the effectiveness of a certain treatment.
The following examples merely serve to illustrate the present invention.

### TABLES

**Table 1.**

| **Neurological complications of chemotherapeutics (partial list).** | | | | | |
|---|---|---|---|---|---|
| **Encephalopatics** | **Cerebral syndromes** | **Myelopathy** | **Peripheral neuropathy** | **Myopathy** | **Stroke-like syndromes** |
| BCNU | cytarabine | IT Methotrexate | cisplatin | corticosteroids | L-asparaginase |
| cisplatin | 5-F-uracil | cytarabine | vincristine | | mtx |
| cytarabine | procarbazine | thiotepa | (cytarabine) | | (ic) BCNU |
| 5-F-uracil | | acc.IT vincristine | (procarbazine) | | (ic) cisplatin |
| ifosfamide | | acc.IT doxorubicin | | | |
| L-asparaginase | | | | | |
| methotrexate | | | | | |
| procarbazine | | | | | |
| corticosteroids | | | | | |
| biological | | | | | |
| response | | | | | |
| modifiers: IL-2, | | | | | |
| Interferon | | | | | |

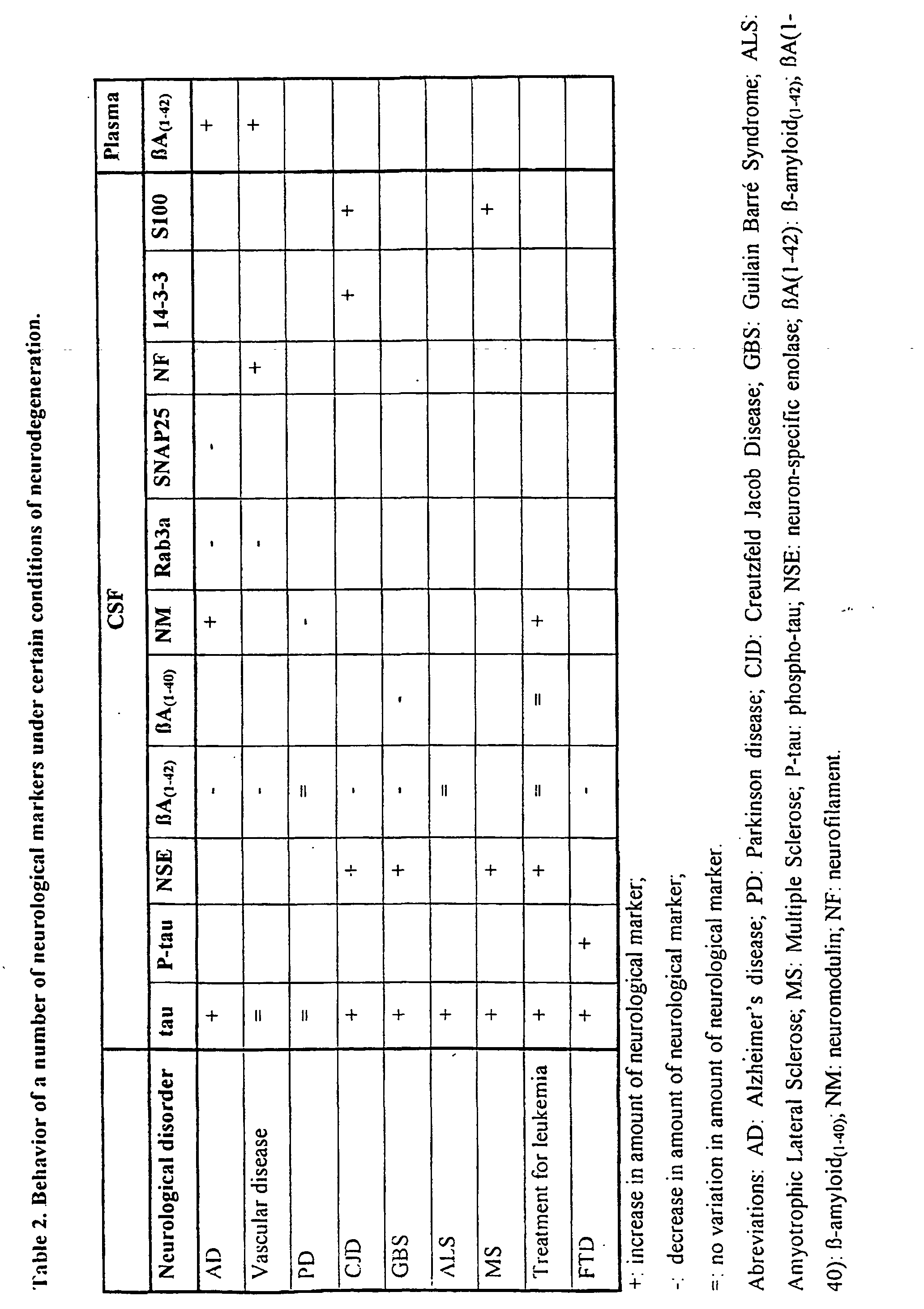

**Table 3.**

| **Titer obtained after immunization of mice with α-synuclein.** | | |
|---|---|---|
| **Animal Antigen** | | **Estimated titer in direct coating** |
| 312 (m3) | 5 µg α-synuclein protein | >512000 |
| 312 (m2) | 5 µg α-synuclein protein | 100000 |
| 309 (m2) | 1°: 50 µg α-synuclein 2°: 5µg peptide (IGP1463) | 512000 (titer of 2000 on peptide) |
| 309 (m4) | 1°: 50 µg α-synuclein 2°: 5µg peptide (IGP1463) | 128000 |
| 313 (m2) | 1°: 50 µg protein 2°: 5 µg protein | 2500000 |
| 308 (m4) | 50 µg protein | 250000 |

**Table 4.**

| **Levels of intracellular proteins in cerebrospinal fluid (mean ± SD)**. | | | |
|---|---|---|---|
| **Markers** | **AD** | **VAD** | **Controls** |
| | n = 32 | n=20 | n=11 |
| Age | 75 ± 7* | 81 ±7* | 68 ± 5 |
| Rab3a (pg/ml) | 16 ± 5* | 16 ± 4* | 23 ± 4 |
| SNAP25 (AU) | 164 ± 12* | 169 ± 12* | 183 ± 10 |
| tau (pg/ml) | 829 ± 440* | 512 ± 209* | 225 ± 128 |
| β-amyloid₍₁₋₄₂₎ (pg/ml) | 310 ± 115 | 343 ± 107 | ** |

| | | | |
|---|---|---|---|
| AU = Arbritary units. *Significantly different from control (p≤0.05; Whitney test). | | | |
| **Data for ß-amyloid₍₁₋₄₂₎ levels in cerebrospinal fluid from control patients were not available. | | | |

**Table 5.**

| **Likelihood ratio's for each marker separately and for combinations of markers.** | | | | |
|---|---|---|---|---|
| | **cut-off** | **Sensitivity** | **Specificity** | **Likelihood Ratio** |
| Rab3a | 230 mOD | 90.4% | 81.8% | 5.0 |
| SNAP25 | 170 mOD | 69.2% | 91.7% | 8.3 |
| tau | 262 pg/nd | 92.9% | 71.4% | 3.3 |
| β-amyloid₍₁₋₄₂₎* | 629 pg/ml | 98.5% | 28.6% | 1.4 |
| tau-β-amyloid_{(1.42)}* | | 91.5% | 79.6% | 4.5 |
| Tau-Rab3a | | 83.9% | 94.8% | 16.2 |
| Tau-SNAP25 | | 64.3% | 97.6% | 27.0 |
| β-amyloid₍₁₋₄₂₎-Rab3a* | | 89.1% | 87.0% | 6.9 |
| β-amyloid₍₁₋₄₂₎-SNAP25* | | 68.2% | 94.0% | 11.5 |
| Tau-β-amyloid₍₁₋₄₂₎-Rab3a* | | 82.7% | 96.3% | 22.3 |
| Tau-β-amyloid₍₁₋₄₂₎-SNAP25 * | | 63.3% | 98.3% | 37.2 |

| | | | | |
|---|---|---|---|---|
| *Data for the β-amyloid₍₁₋₄₂₎ levels in cerebrospinal fluid from control patients were obtained from another study. | | | | |

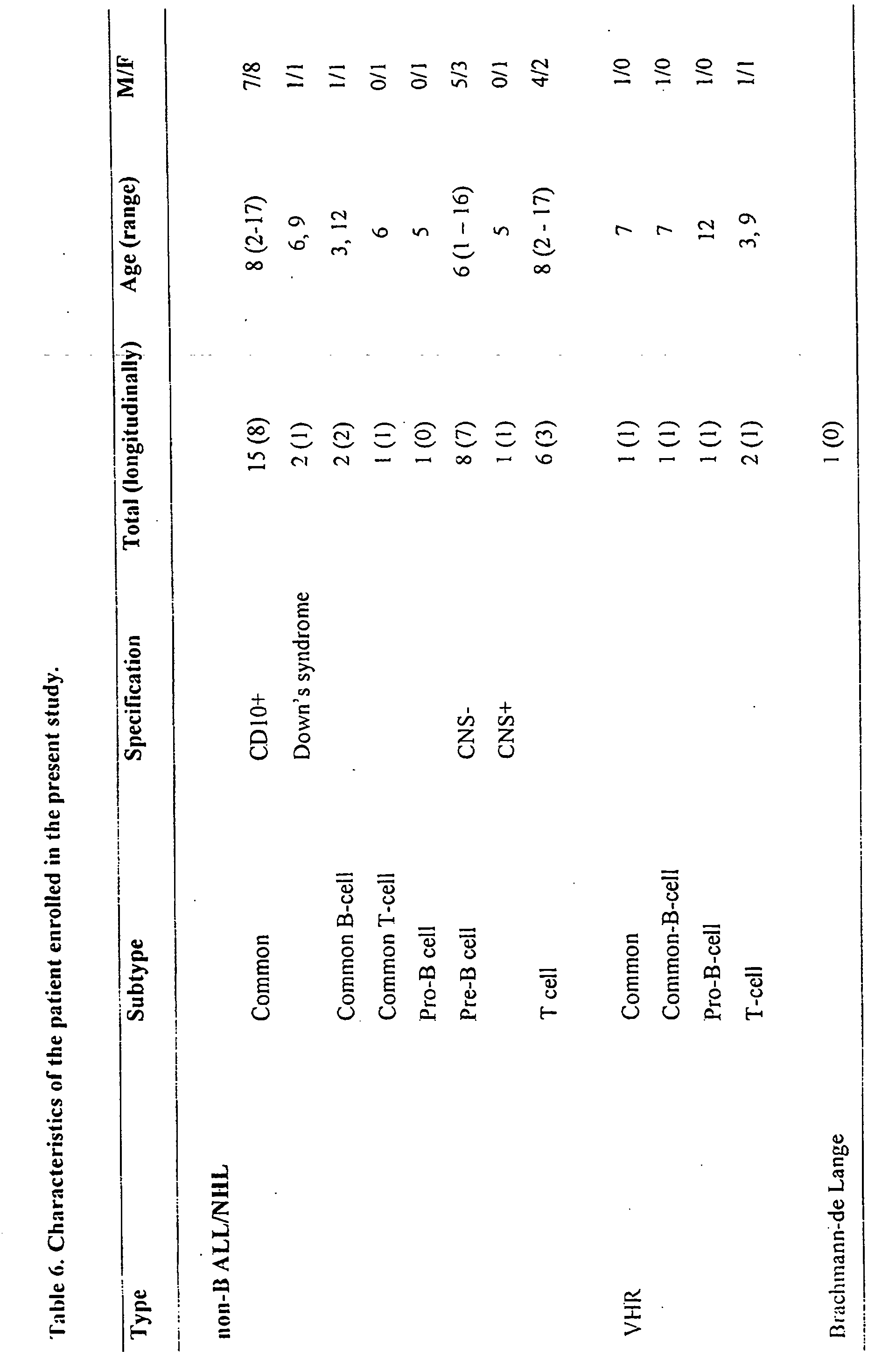

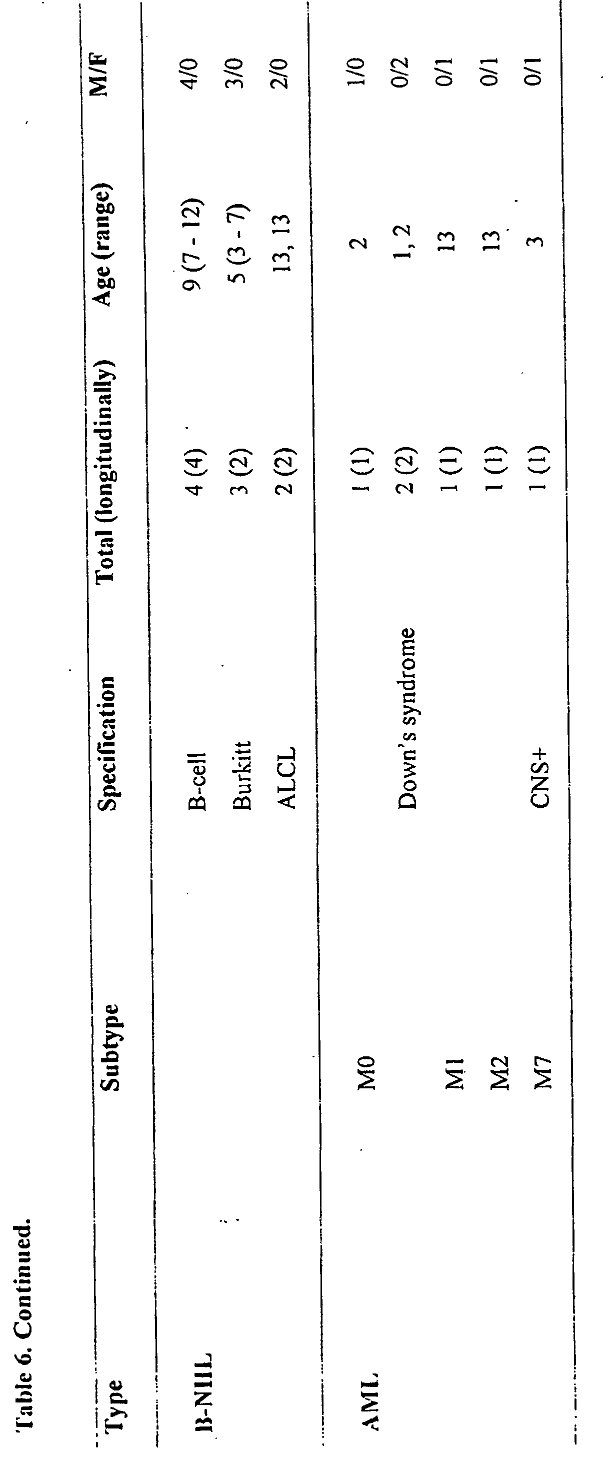

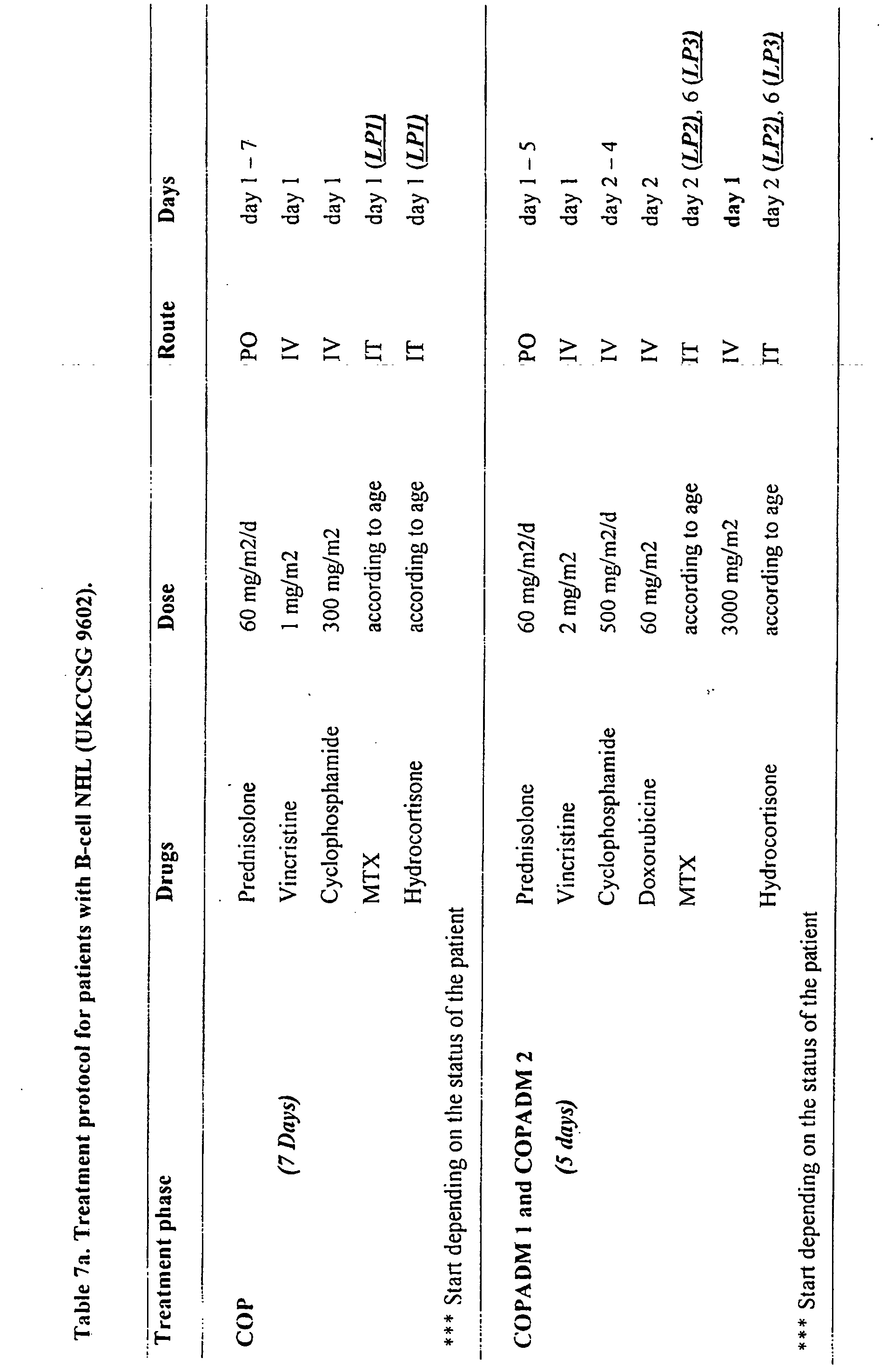

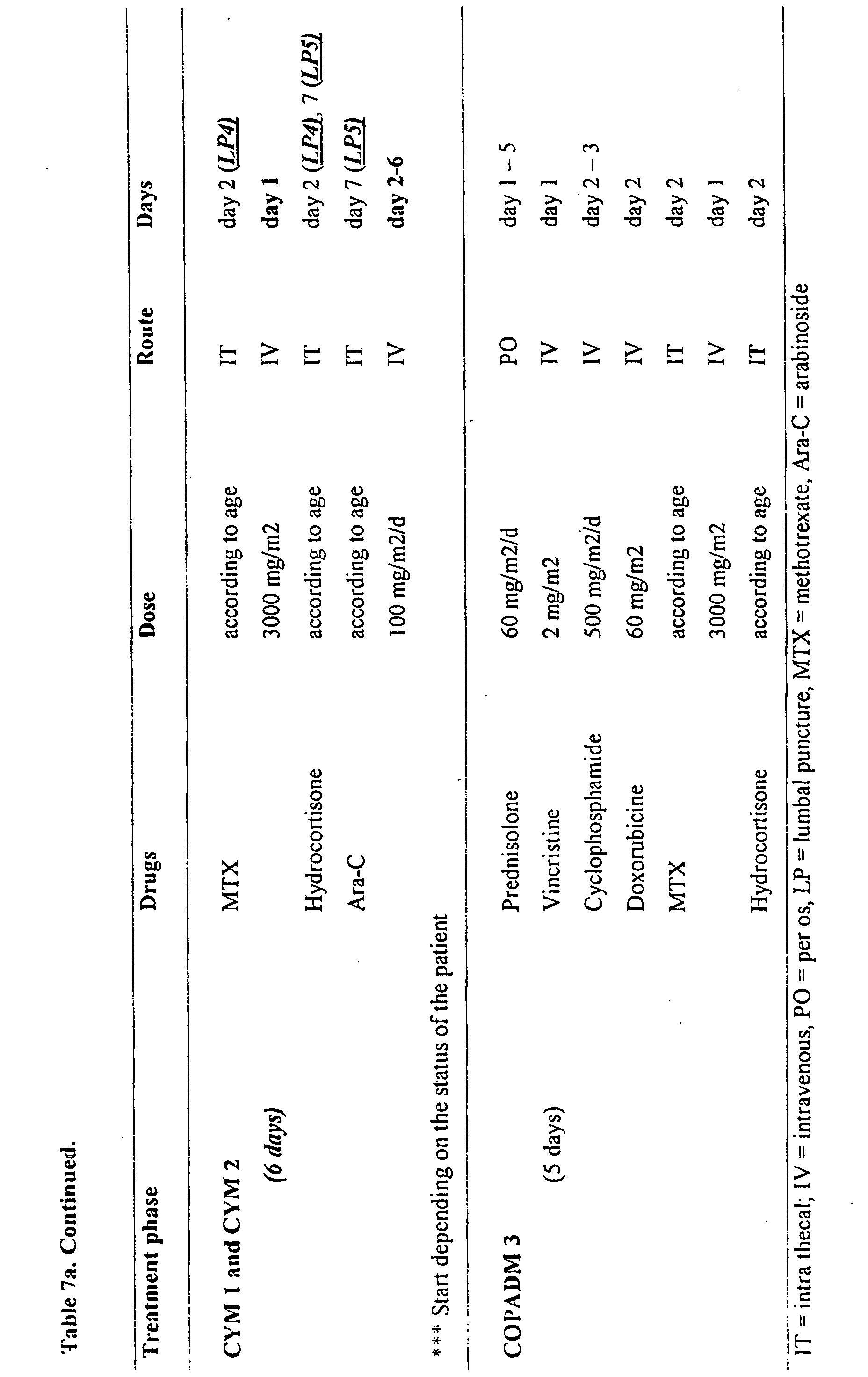

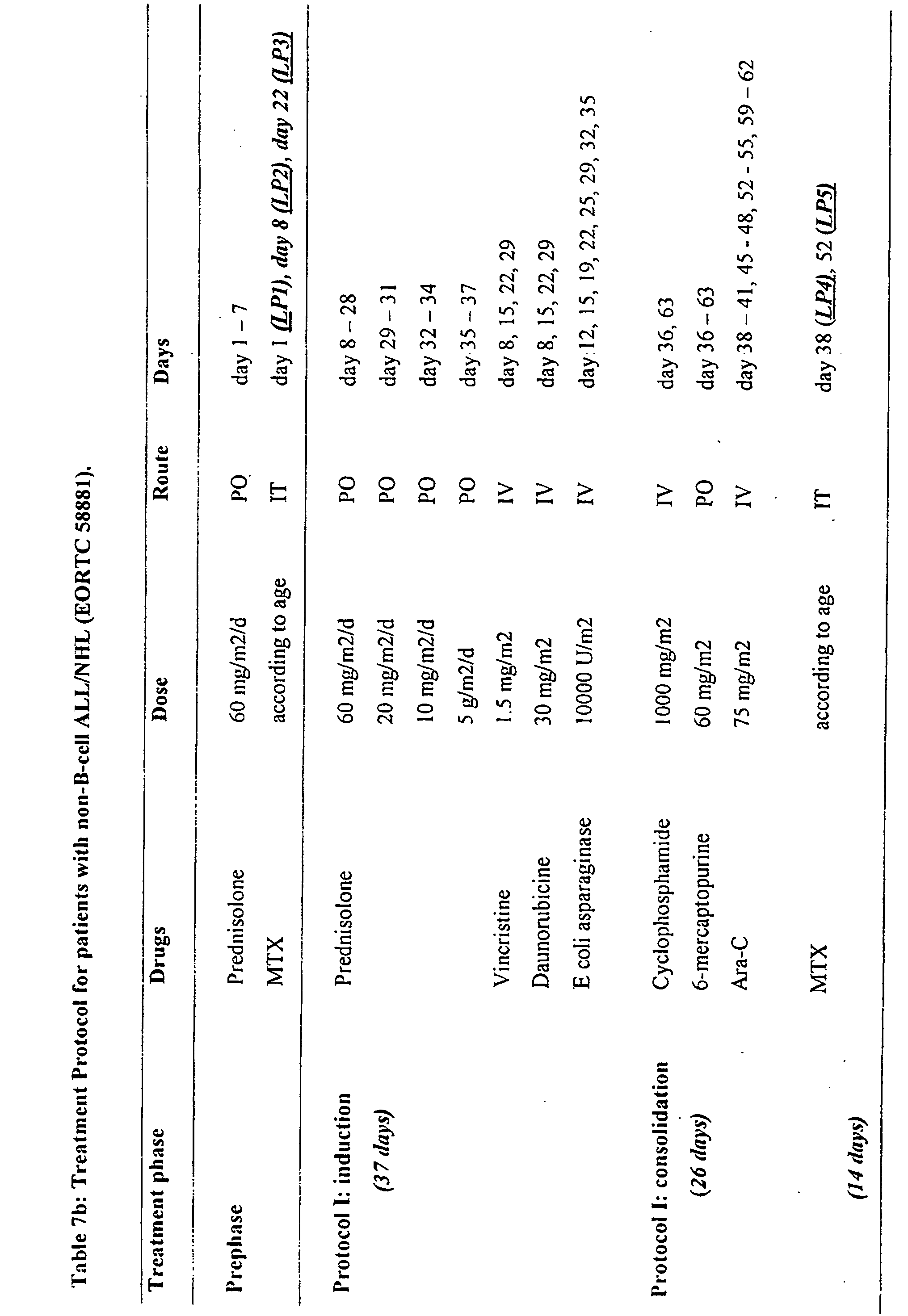

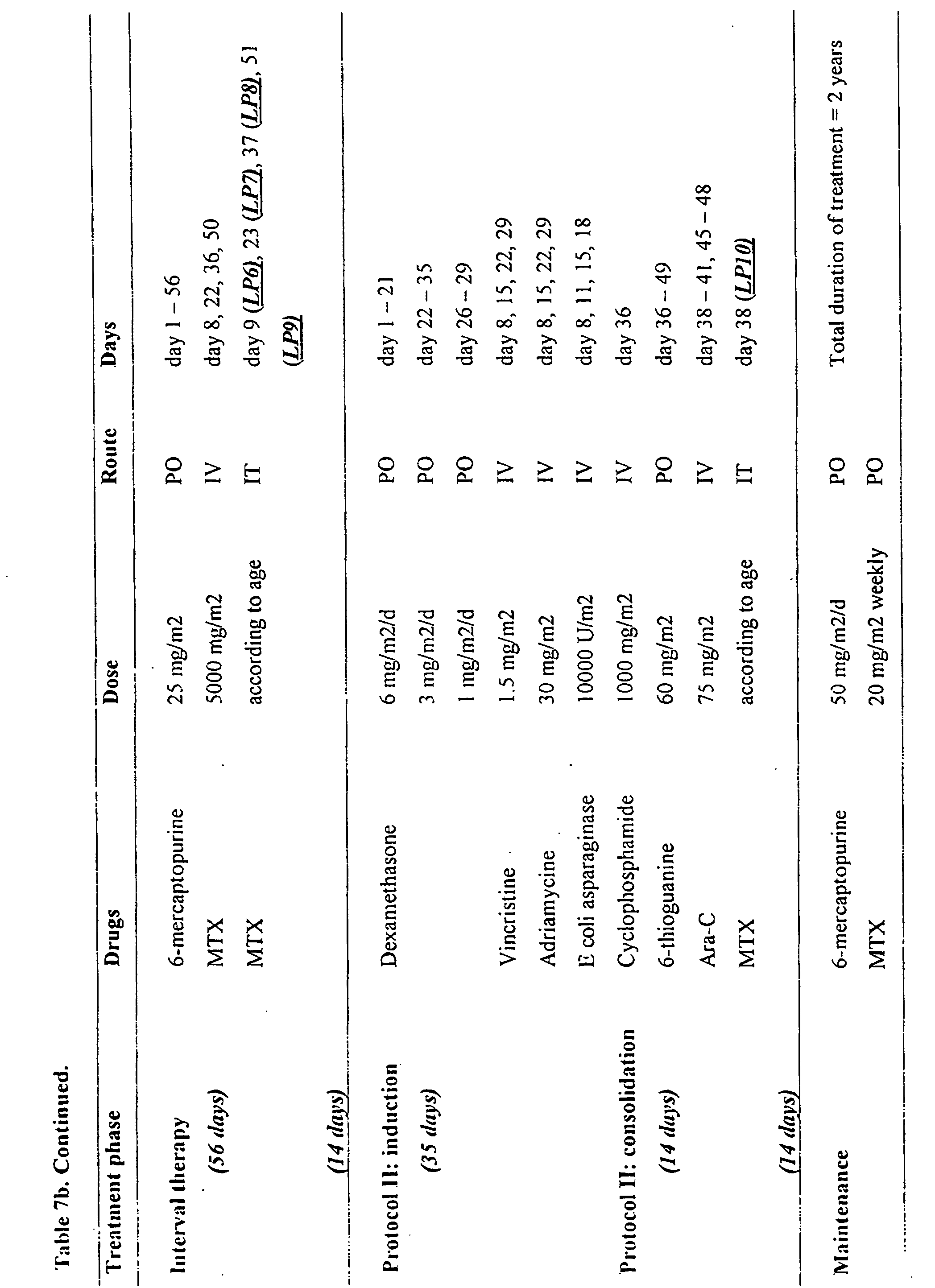

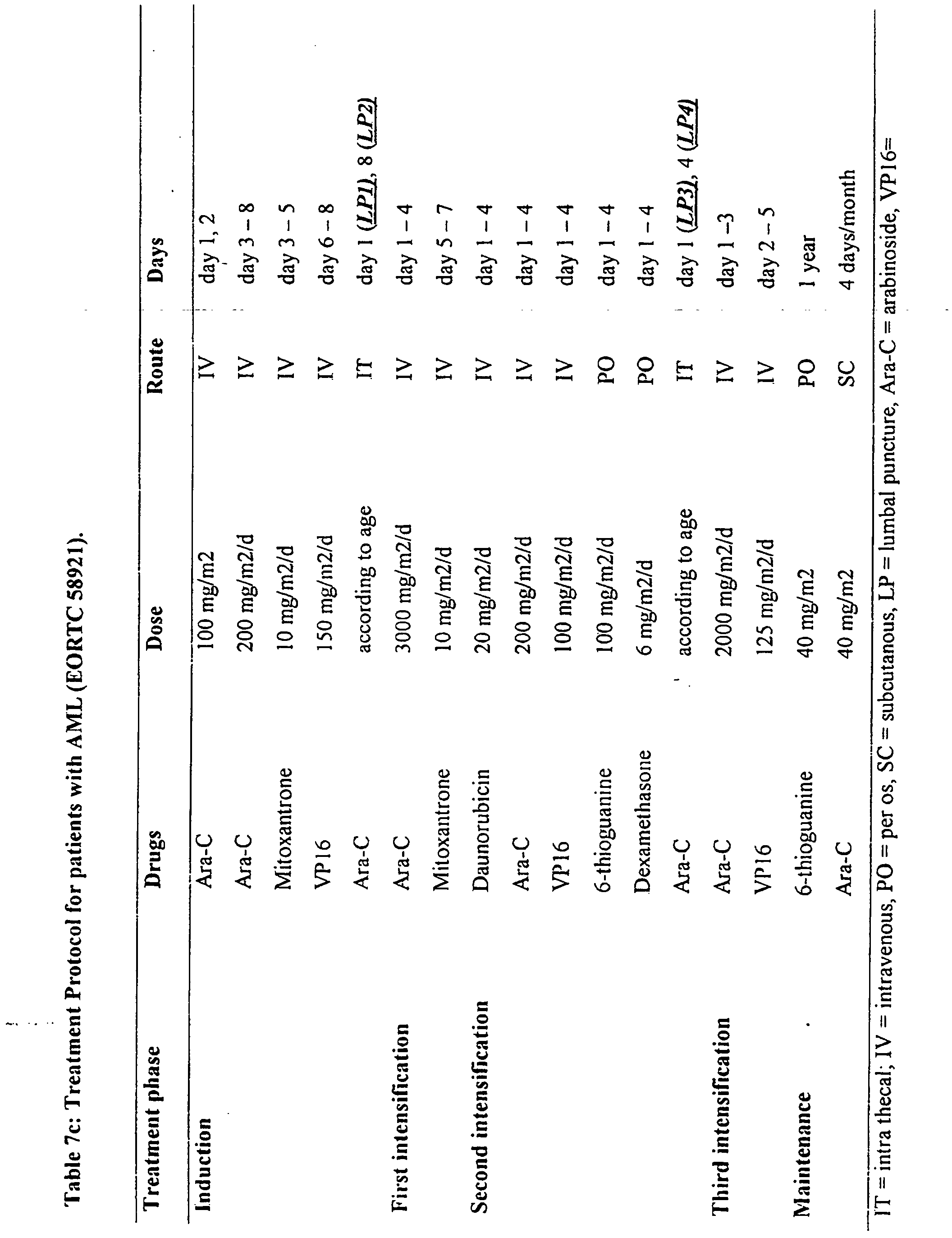

**Table 8.**

| **Average cerebrospinal fluid levels of tau, β-amyloid**_{**(1-42)**} **and neuromodulin for 4 groups of patients as described in example 7.** | | | | | | |
|---|---|---|---|---|---|---|
| **Group** | **n** | **M/F** | **Age** | **tau** | β**-amyloid**_{**(1-42)**} | **NM** |
| Control | 70 | 36/34 | 45 ± 16 | 120 ± 78 | 466 ± 168 | 504 ± 283 |
| Memory Impairment | 7 | 05/02 | 61 ± 10 | 253 ± 139* | 184, 184, 550 | 518 ± 168 |
| AD | 27 | 16/11 | 71 ± 12 | 316 ± 186* | 244 ± 74* | 927 391* |
| VAD | 5 | 02/03 | 68 ± 5 | 235 ± 82 | 272,413 | 737 ± 514 |

| | | | | | | |
|---|---|---|---|---|---|---|
| *Significantly different from controls (p<0.01; two-tailed Wilcoxon test). Levels are expressed in pg/ml, average ± standard deviation. | | | | | | |

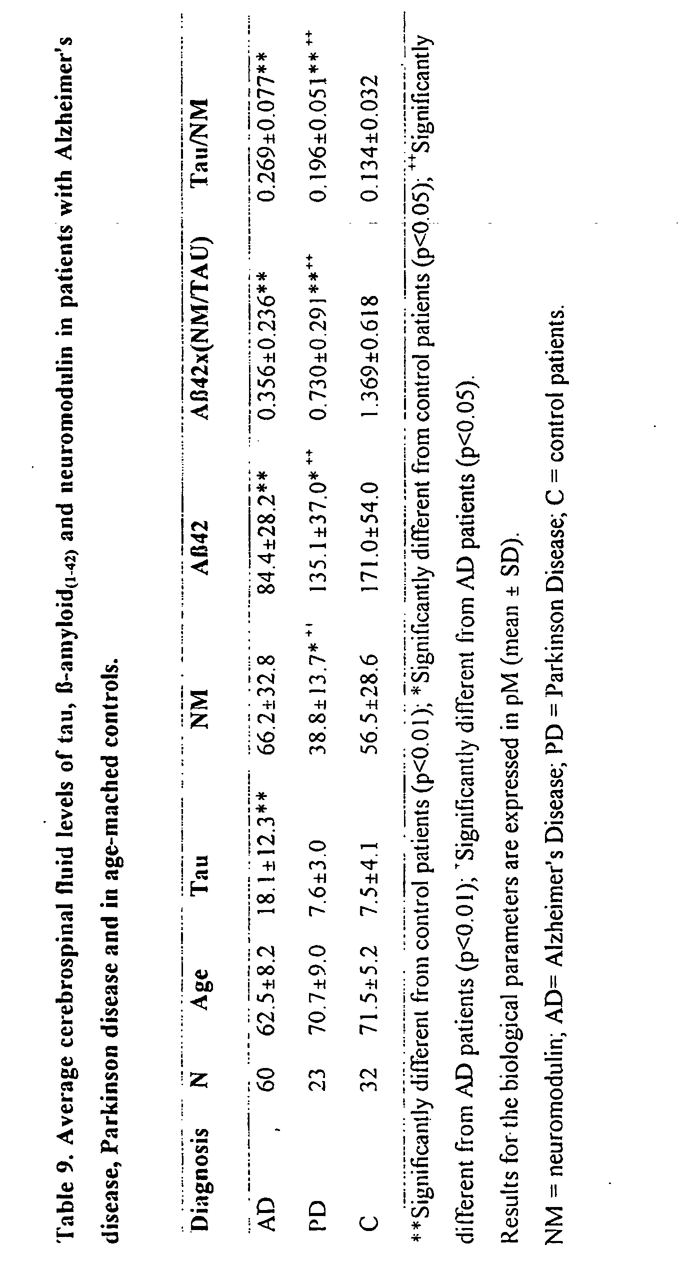

### FIGURE LEGENDS

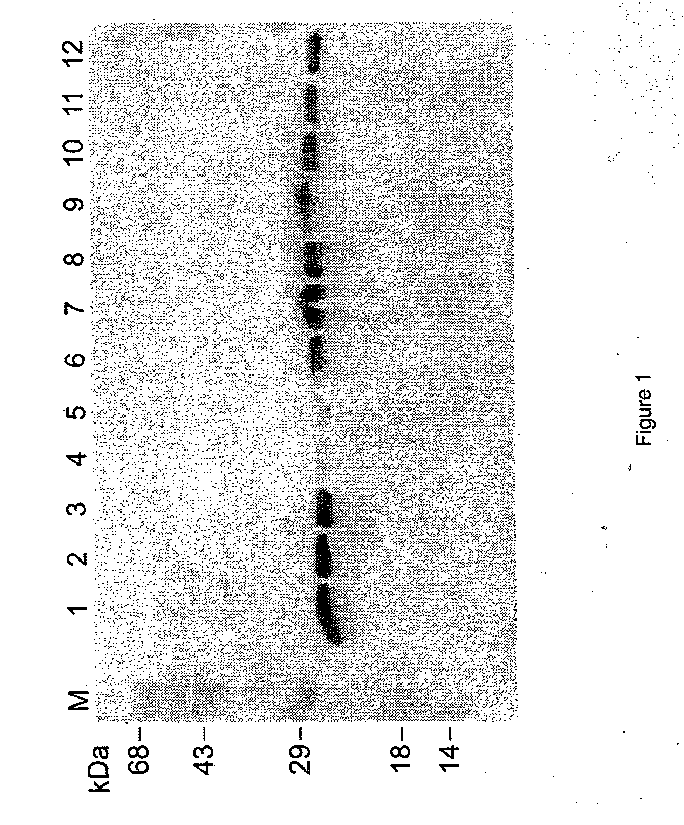

**Figure 1.** Western blot as described in example 1.3, showing Rab3a immunoreactivity in temporal cortex of Alzheimer's disease and control brains. 1. 12.8 µl Control patient n°3; 2. 6.4 µl Control patient n°3; 3. 3.2 µl Control patient n°3; 4. 1.6 µl Control patient n°3; 5. 1.0 µl Control patient n°3; 6. 10 µl Control patient n°3; 7. 10 µl AD patient n°1; 8. 10 µl AD patient n°2; 9. 10 µl Control patient n°1; 10. 10 µl Control patient n°2; 11. 10 µl AD patient n°3; 12. 10 µl Control patient n°4.

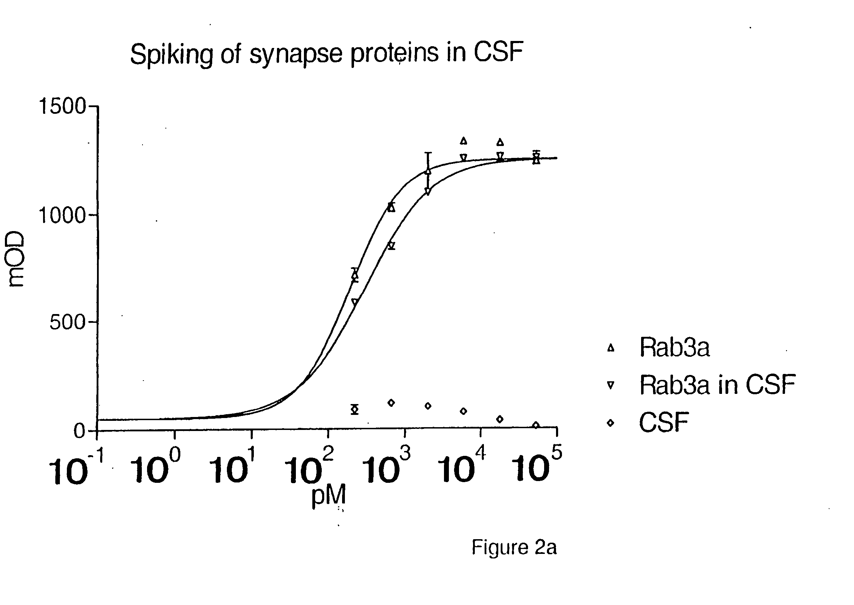

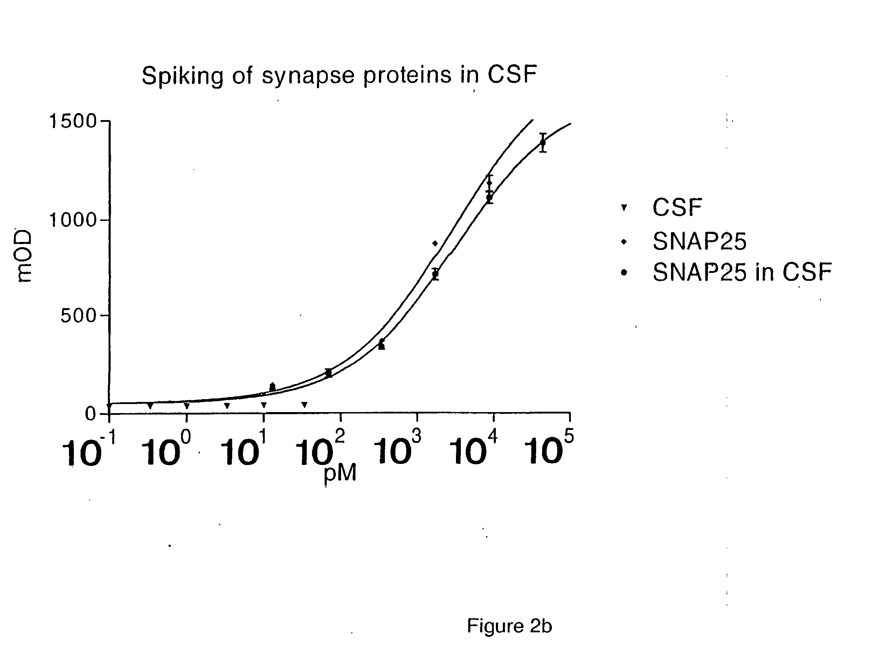

**Figure 2.** Stability of synapse proteins Rab3a (a) and SNAP25 (b) in cerebrospinal fluid. The degradation of synapse proteins was quantified via a sandwich ELISA specific for the synapse protein as described in example 1.5. Purified synapse proteins were spiked in a pool of CSF and incubated overnight at 37°C (legend: Rab3a in CSF; SNAP25 in CSF). As a control the synapse protein was spiked in the same pool of CSF and directly quantified (legend: Rab3a; SNAP25). The stability was also assayed in 1% BSA (results not shown).

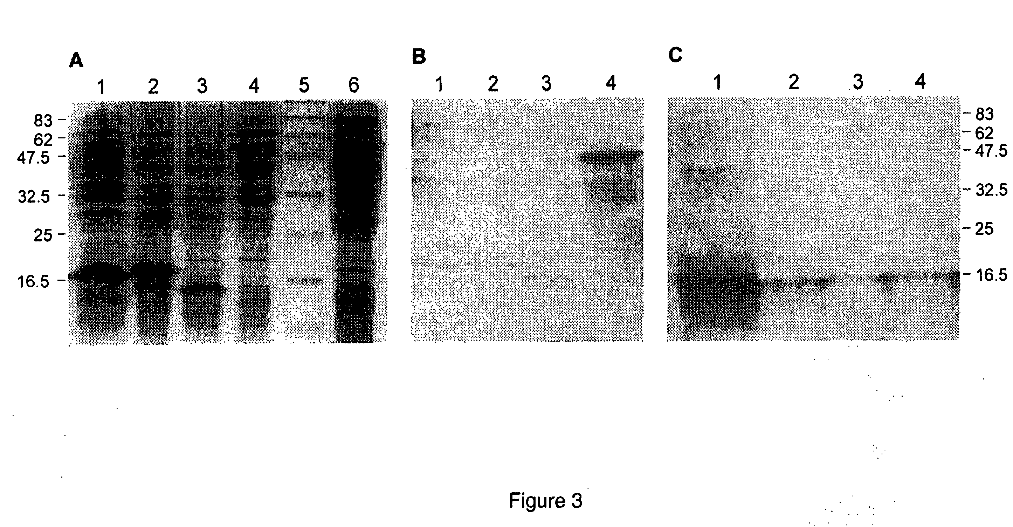

**Figure 3.** Demonstration of the specificity of the antibody from Transduction Labs (Lexington, KY, USA; Cat. No. S63320) for α-synuclein. A: Coumassie stained gel; B: Western blot developed with an anti-His monoclonal antibody to reveal expression of all products; C: Western blot developed with the monoclonal antibody from Transduction Labs (Lexington, KY, USA). Lane 1: *E. coli* expressing α-synuclein; lane 2: *E*. *coli* expressing β-synuclein; lane 3: *E. coli* expressing γ-synuclein; lane 4: control lane with an *E*. *coli* expressing neuron-specific enolase; lane 5: molecular weight standards; lane 6: *E. coli* strain without any plasmid.

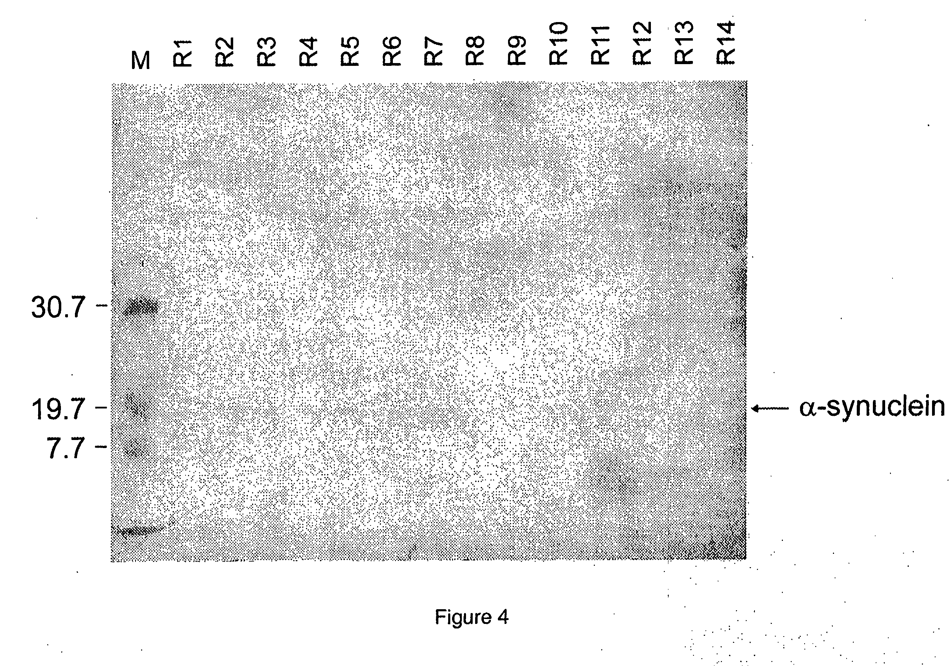

**Figure 4.** Detection of α-synuclein in 200 ml CSF. The pooled CSF was separated according the molecular weight and to isoelectric point via Rotophor as described in example 2.2. M: Molecular weight markers; R1: pI 3; R2: pI 4; R3: pI 4.5; R4: pI 4.5; R5: pI 5; R6: pI 5; R7: pI 5.5; R8: pI 6; R9: pI 6, R10: pI 6.5; R11: pI 6.5; R12: pI 7; R13: pI 7; R14: pI 7.5.

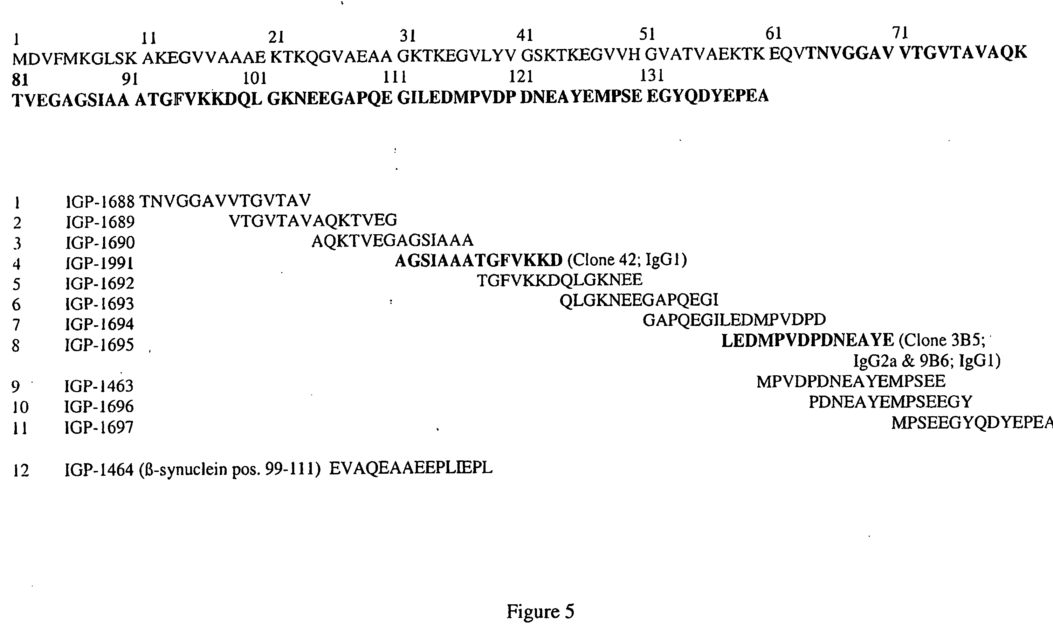

**Figure 5**. Amino acid sequence of α-synuclein and overlapping peptides used for mapping of monoclonal antibodies 3B5 and 9B6 to the carboxyterminus of α-synuclein. The carboxyterminal part used for the synthesis of the peptides is shown in bold. The peptides that were recognized by the monoclonal antibodies and by a commercial antibody (Clone 42, IgG1, Transduction Labs, Lexington, KY, USA) are indicated.

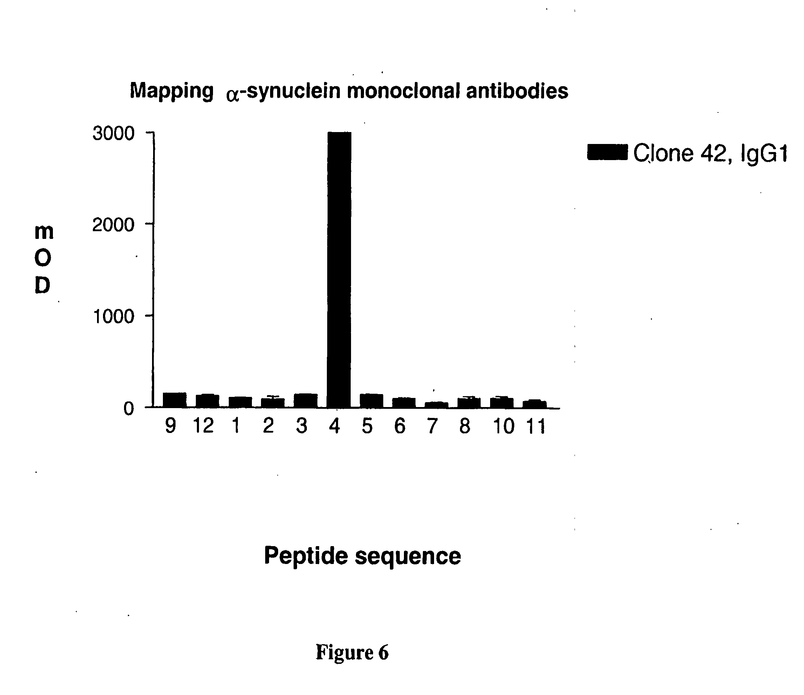

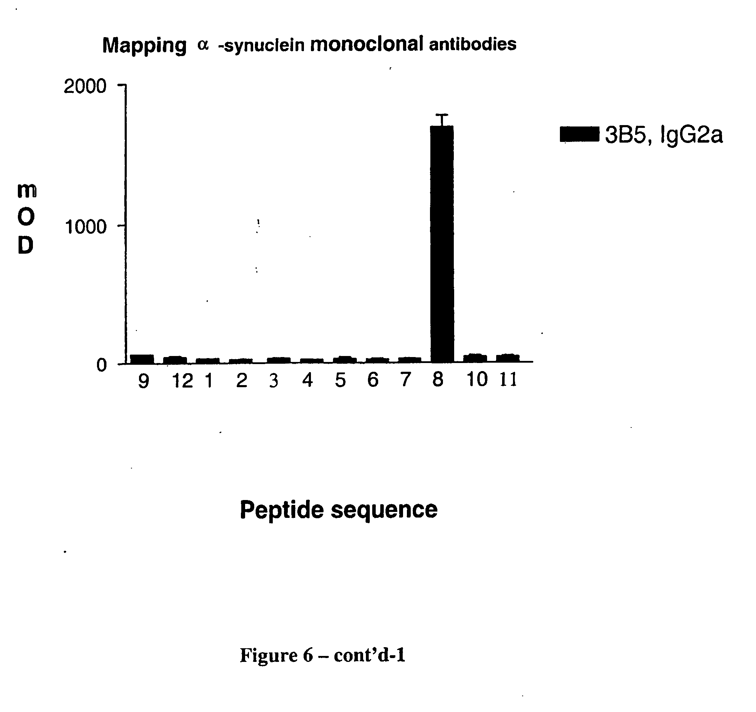

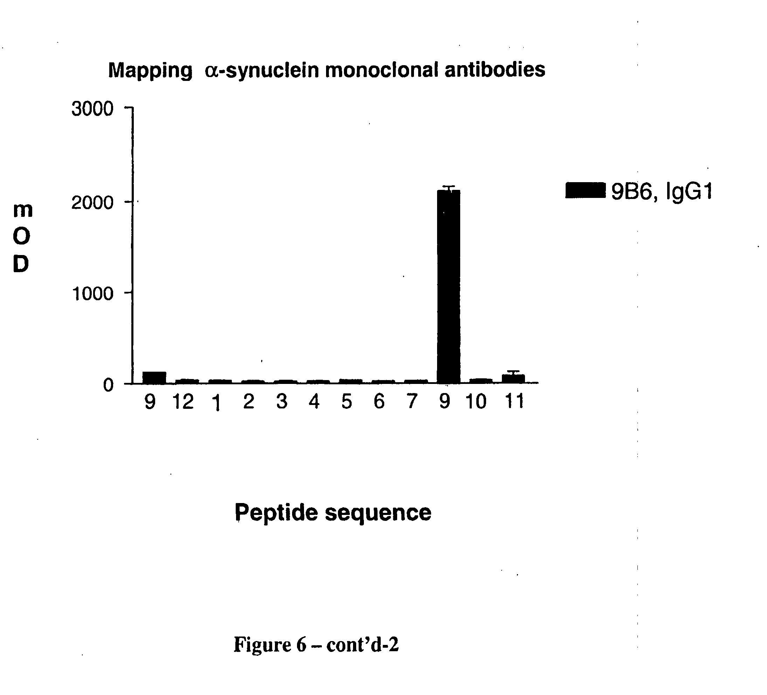

**Figure 6.** Optical density (OD) obtained after reaction of different α-synuclein carboxyterminal peptides with antibodies 3B5, 9B6 and Clone 42. The optical density was measured in an immunoassay as described in example 2.4. Numbers on the X-axis (1-12) correspond with the numbers of the peptides as shown in figure 5.

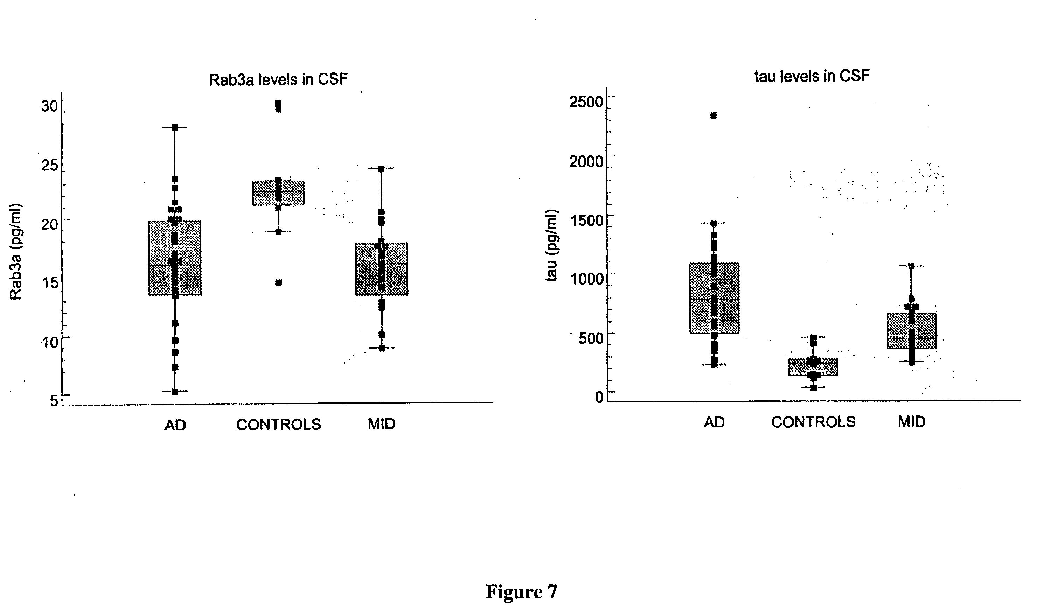

**Figure 7**. Rab3a and tau levels in CSF of 32 AD patients, 20 patients with vascular dementia (MID) and 11 controls measured with a sandwich ELISA as described in examples 1.6 and 3.1.

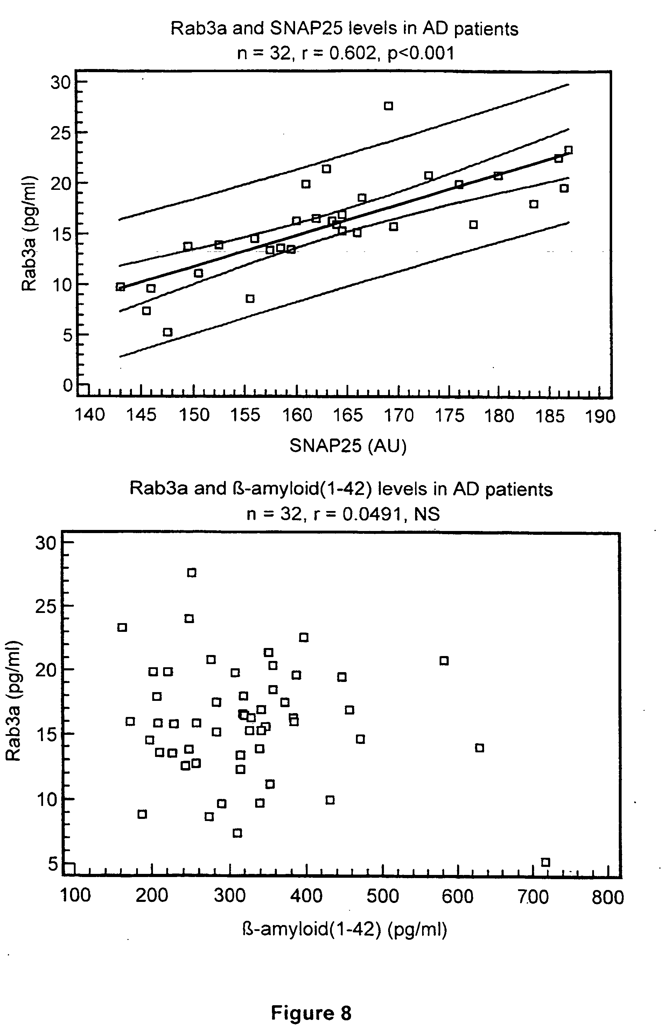

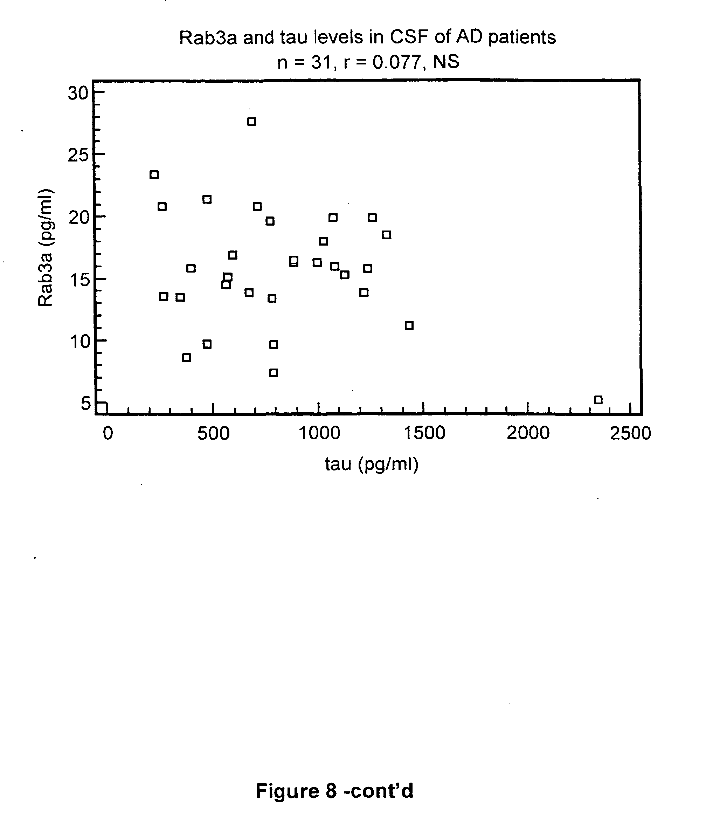

**Figure 8**. Correlations between CSF-levels of Rab3a and SNAP25, Rab3a and β-amyloid₍₁₋₄₂₎ and Rab3a and tau in AD patients described in example 4.1. Levels of Rab3a, SNAP25, tau and β-amyloid₍₁₋₄₂₎ were measured with a sandwich ELISA as described in examples 1.6 and 3.

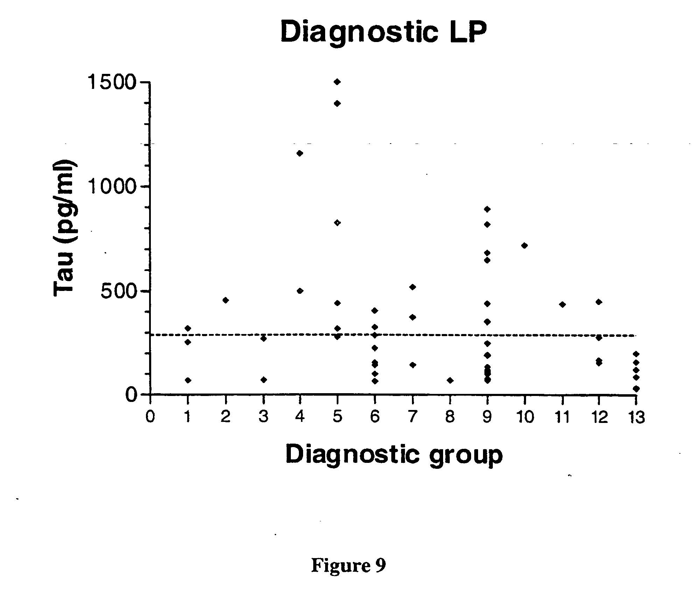

**Figure 9**. Tau values at diagnosis, before any treatment was given: 1. AML (3); 2. AML-CNS+ (1); 3. Down AML (2); 4. Myelodysplasia (2); 5. Others [(Medulloblastoma(2), rhabdomyosarcoma (2), intracranial germinoma (1)]; 6. B-NHL (8); 7. Hodgkin's Disease (3); 8. Down NB ALL (1); 9. NB ALL (21); 10. NB ALL- Brachman syndrome (1); 11. NB ALL-CNS⁺(1); 12. NB ALL-VHR (4); 13. Controls (6) (number of patients).

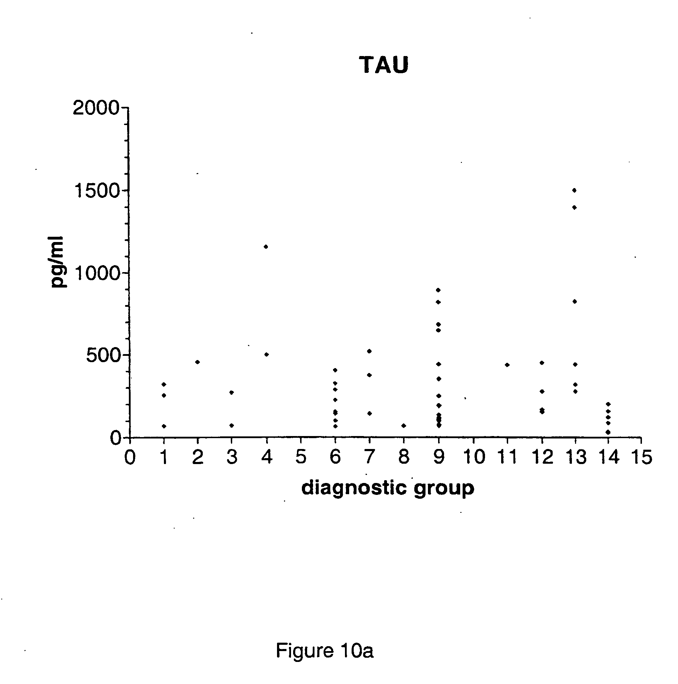

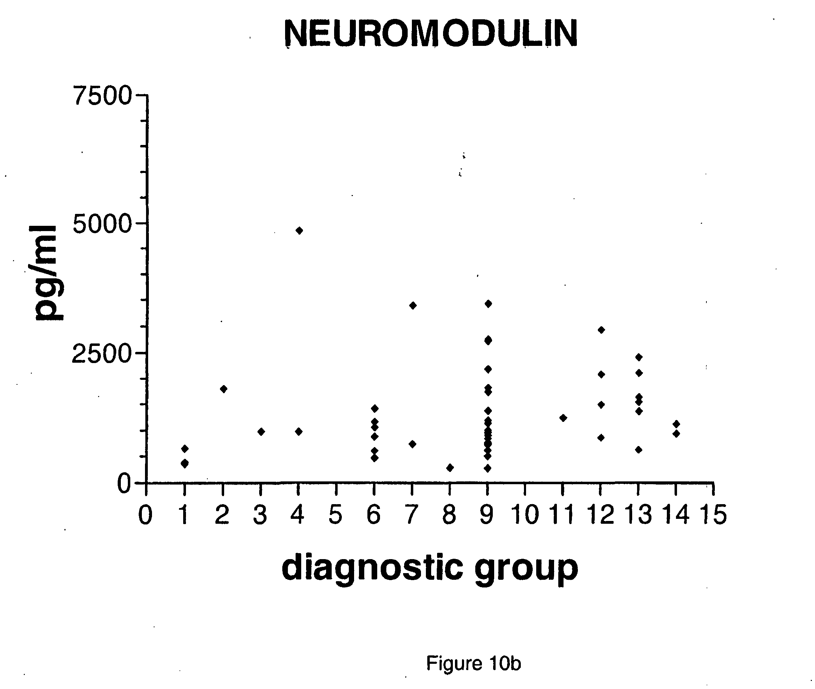

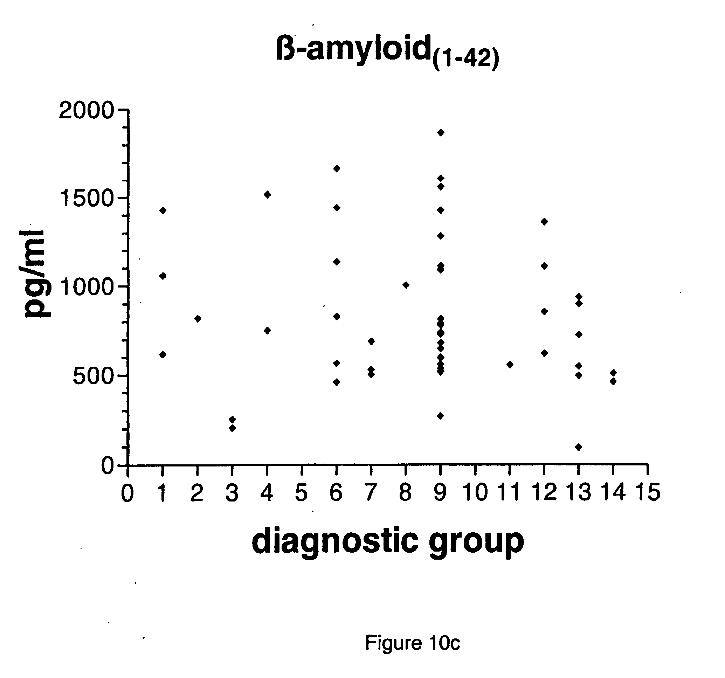

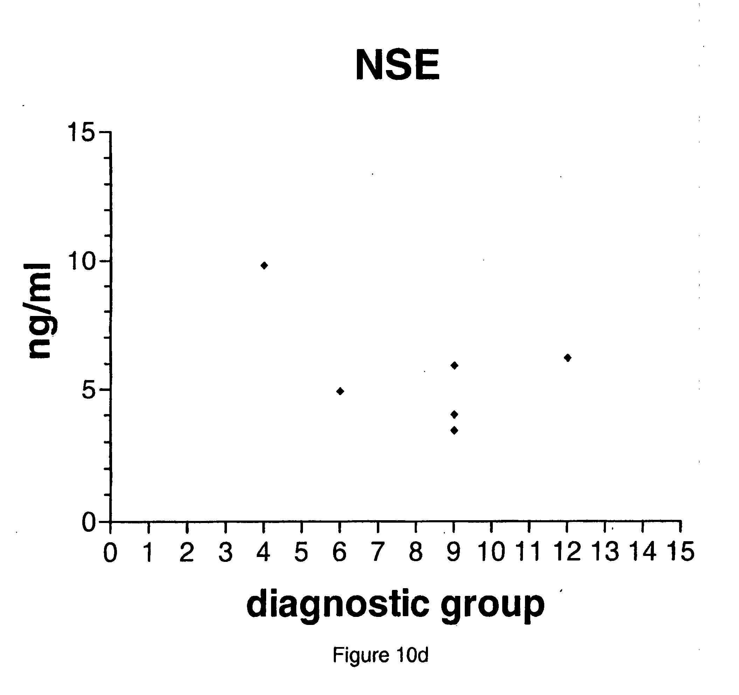

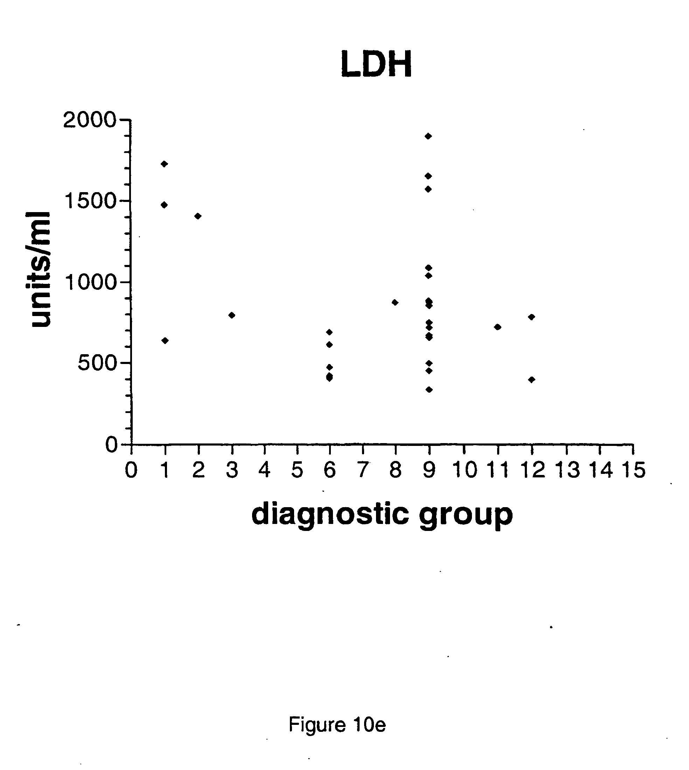

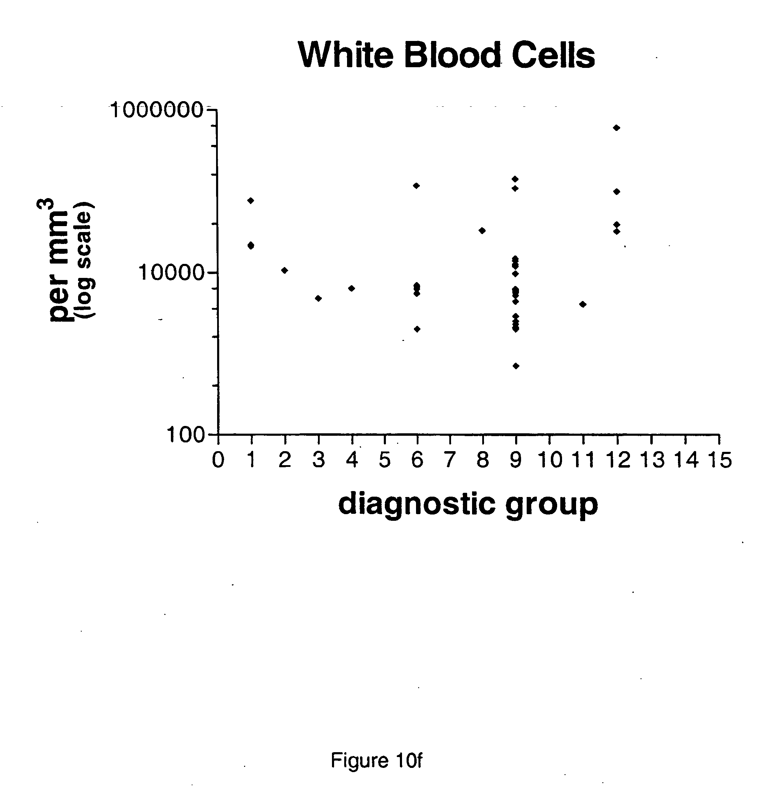

**Figure 10.** Concentrations of the CSF neurological markers tau (a), neuromodulin (b), β-amyloid₍₁₋₄₂₎ (c) and NSE (d), serum LDH (e) and CSF WBC (f) at day 1. 1. AML; 2. AML - CNS+; 3. Down AML/MDS; 4. Myelodysplasia; 5. Chronic myelomic leukemia; 6. B-NHL; 7. Hodgkin's Disease; 8. Down NB ALL; 9. NB ALL; 10. NB ALL - Brachman syndrome; 11. NB ALL - CNS+; 12. NB ALL - VHR; 13. LCH, rhabdomyosarcoma, germinoma, medulloblastoma, choriocarcinoma; 14. Controls, retinoblastoma (healthy), hemofagocytose (gezond HLH). Detection methods for the markers are described in example 3.

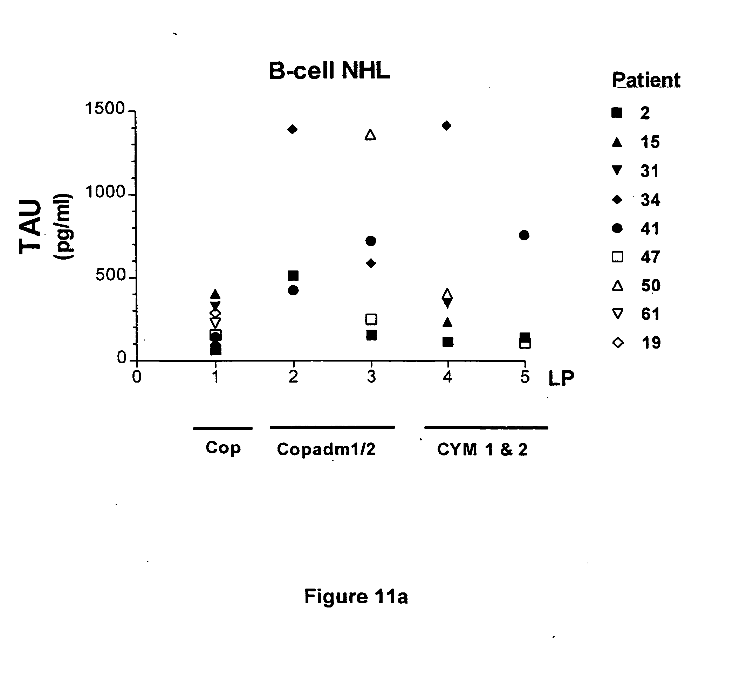

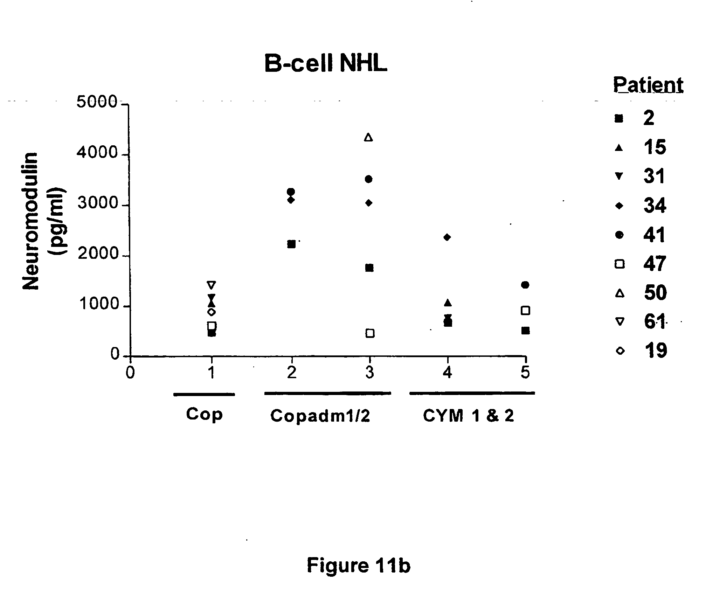

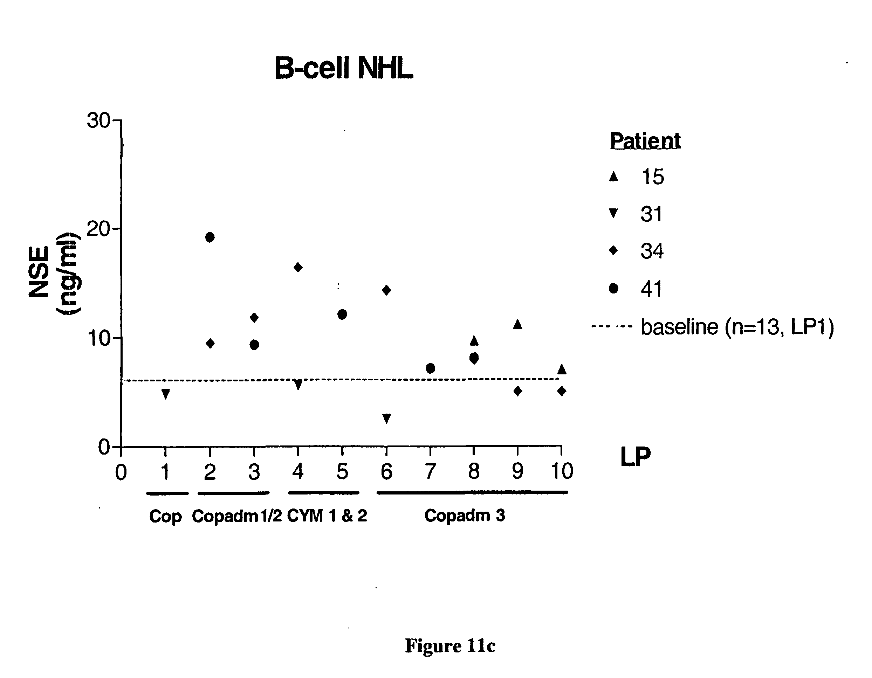

**Figure 11.** Level of the CSF neurological markers tau (a), neuromodulin (b) and neuron-specific enolase (c) in function of lumbar puncture (LP) number during chemotherapy of B-cell NHL patients. The number on the X-axis corresponds to the LP number as given in table 7a. Detection methods for the markers are described in example 3.

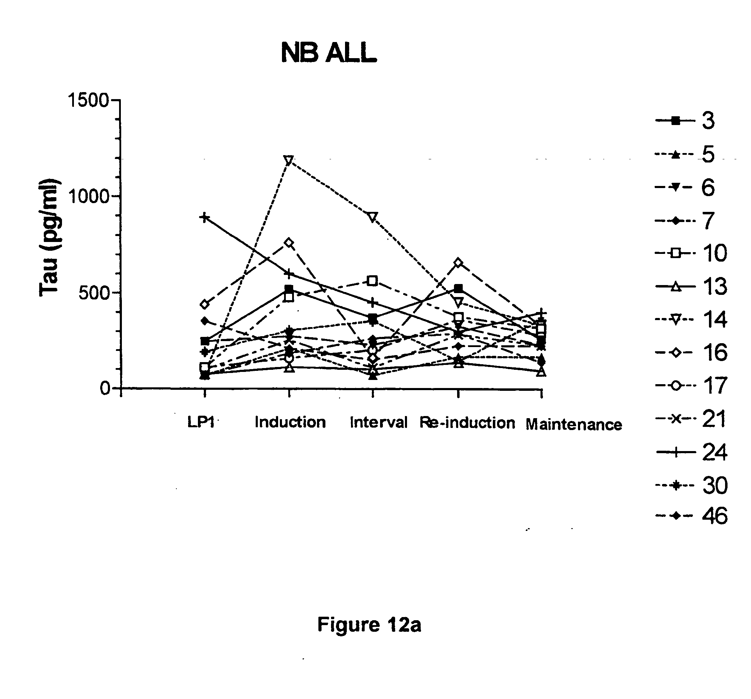

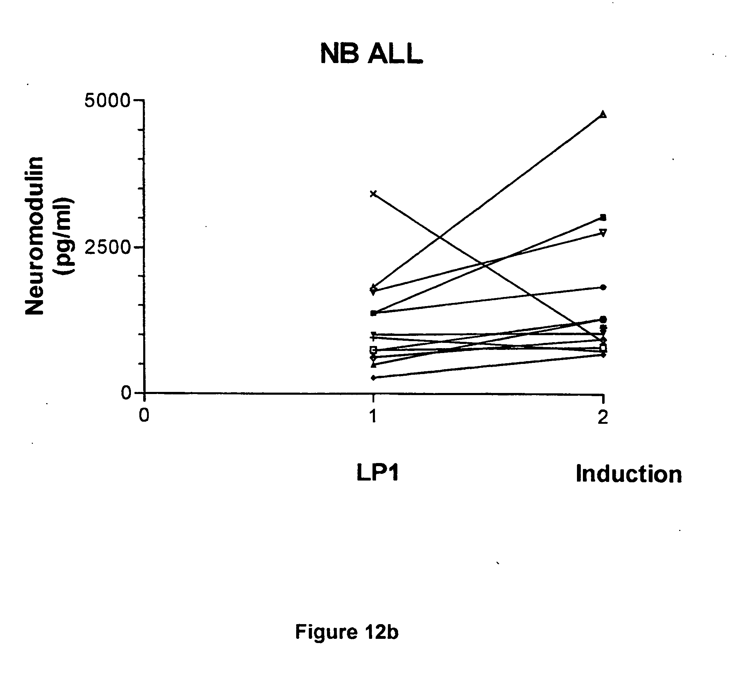

**Figure 12.** Level of the CSF neurological markers tau (a) and neuromodulin (b) for 13 non-B ALL patients at different phases of the chemotherapy treatment. Detection methods for the markers are described in example 3.

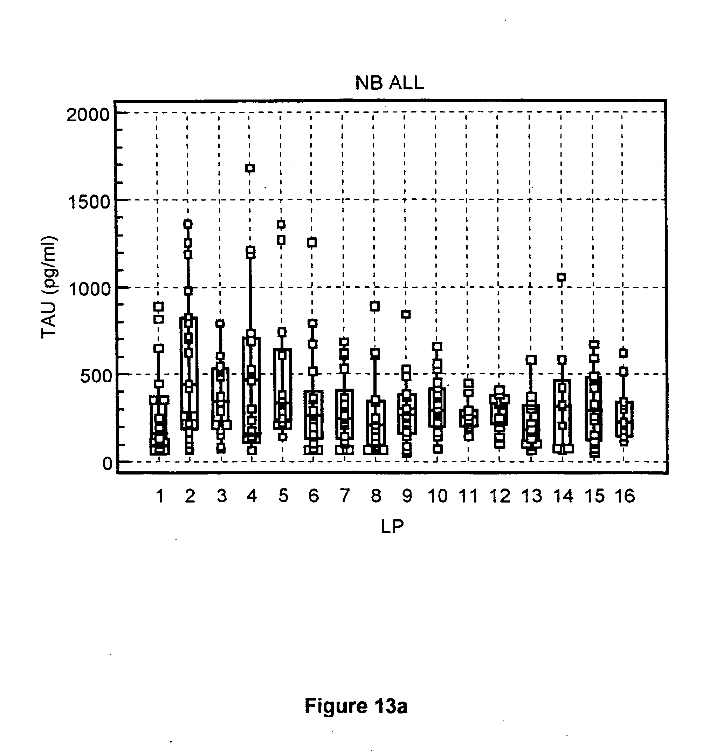

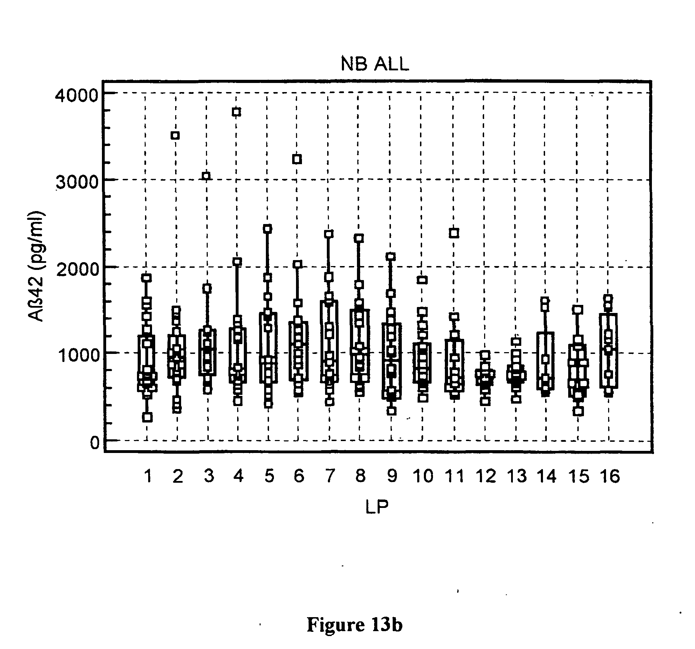

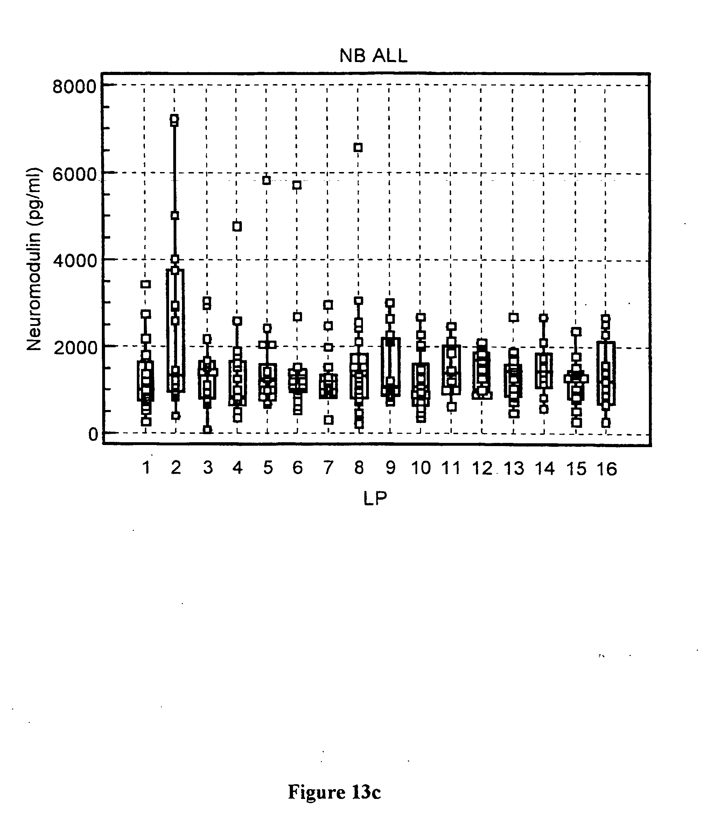

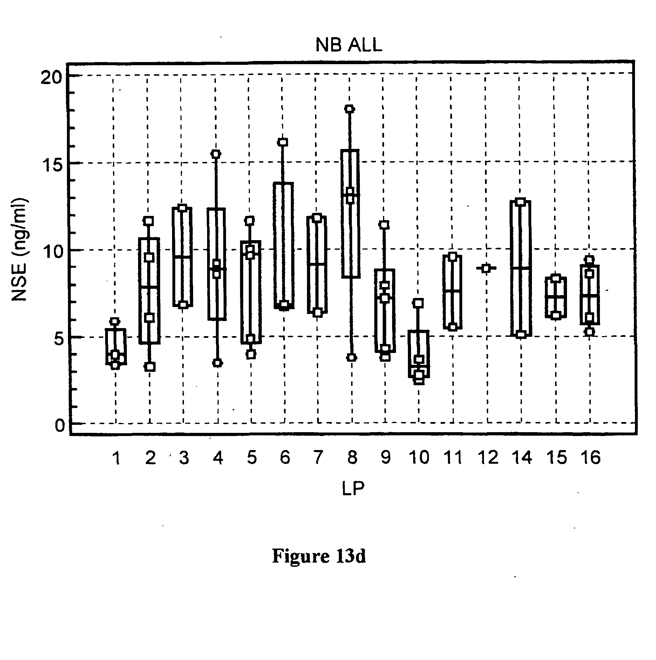

**Figure 13.** Level of the CSF neurological markers tau (a), β-amyloid₍₁₋₄₂₎ (b), neuromodulin (c) and neuron-specific enolase (d) in function of lumbar puncture (LP) number during chemotherapy of non-B-cell ALL patients. The number on the X-axis corresponds to the LP number as given in table 7b. Detection methods for the markers are described in example 3.

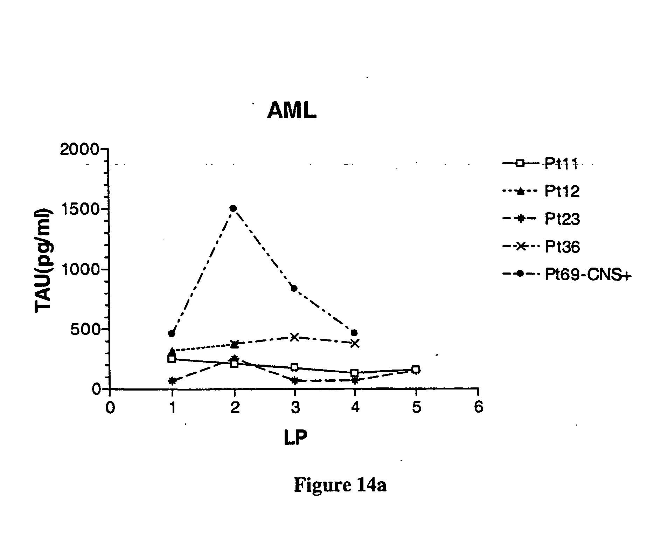

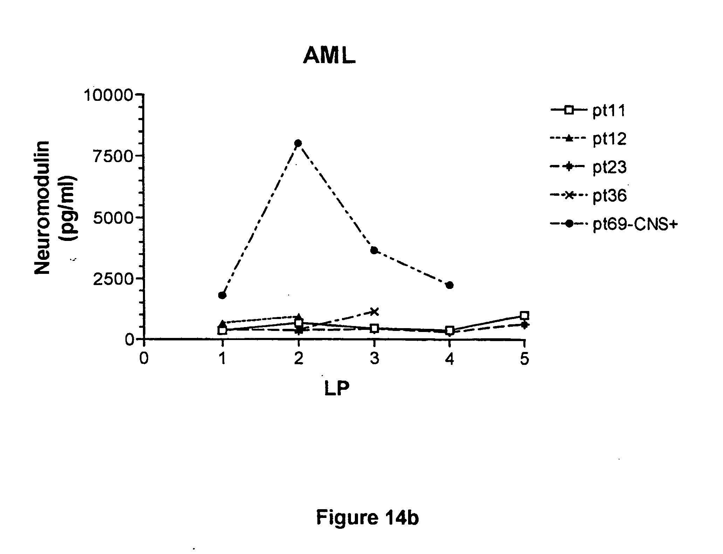

**Figure 14.** Level of tau (a) and neuromodulin (b) in function of lumbar puncture (LP) number during chemotherapy of AML patients. The number on the X-axis corresponds to the LP number as given in table 7c. Detection methods for the markers are described in example 3.

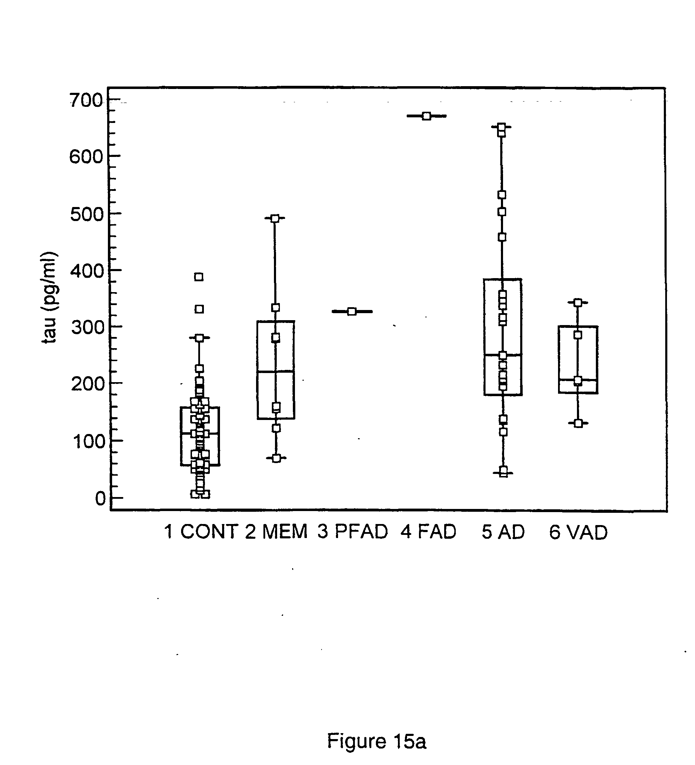

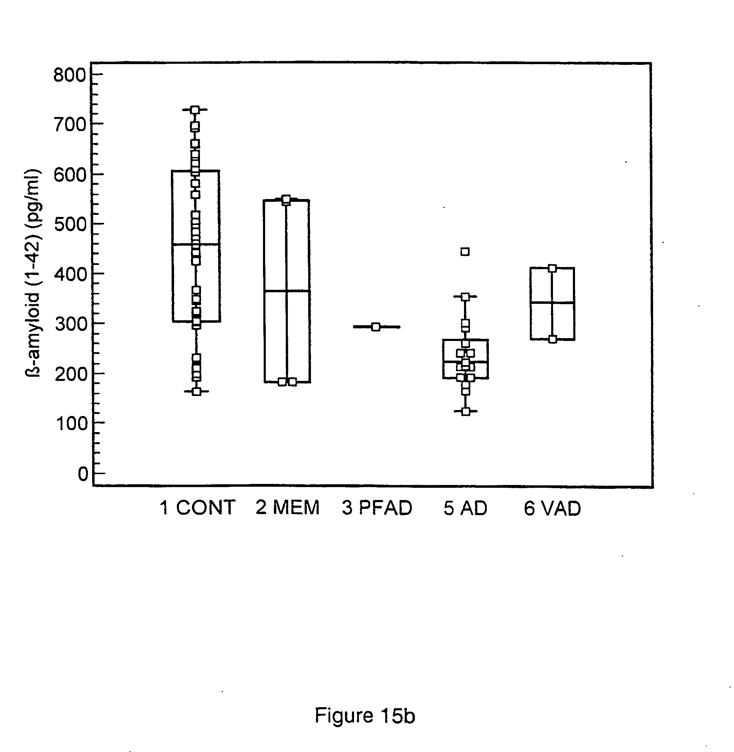

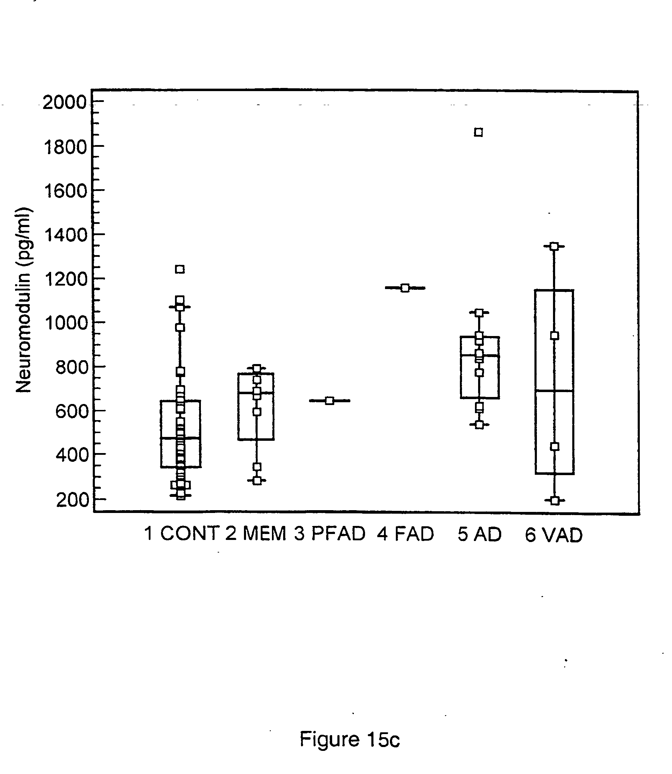

**Figure 15**. Individual levels of tau (a), β-amyloid₍₁₋₄₂₎ (b) and neuromodulin (or Growth-Associated Protein 43) (c) in individuals classified as neurological controls (Guilain-Barré Syndrome, Multiple Sclerosis, etc) (1 CONT), people with memory impairment (2 MEM), a presymptomatic Familial Alzheimer patient (PS1 mutation) (3 PFAD), a Familial Alzheimer patient (PS1 mutation) (4 FAD), Alzheimer patients (5 AD) and patients with vascular dementia (6VAD) as described in example 7. The level of β-amyloid₍₁₋₄₂) was measured as described in example 3. Data are expressed in pg/ml.

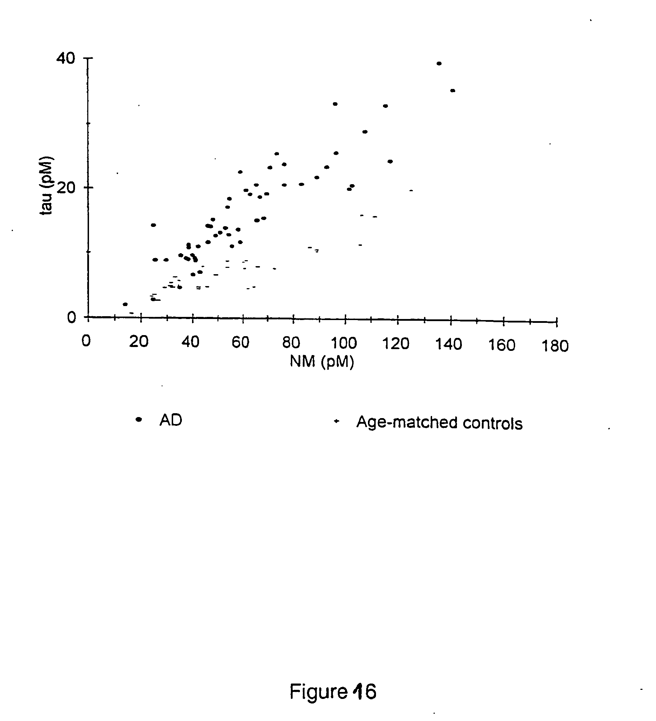

**Figure 16.** Correlation between individual levels of tau and neuromodulin in 60 patients with Alzheimer's disease (AD) and 32 age-mached controls. The level of tau and neuromodulin was measured as described in example 3.

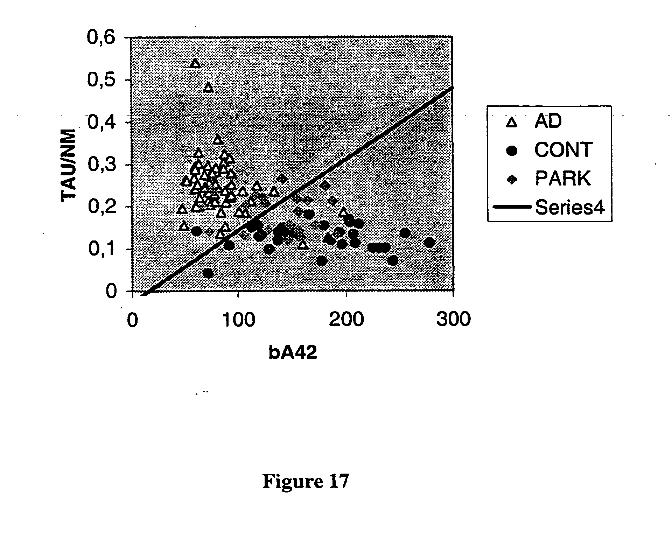

**Figure 17.** Correlation between individual levels of tau/nm and β-amyloid₍₁₋₄₂₎ in patients with Alzheimer's disease (AD), Parkinson disease (PARK) and control patients (CONT). The level of tau, neuromodulin and β-amyloid₍₁₋₄₂₎ was measured as described in example 3.

### EXAMPLES

### Example 1: Detection of Rab3a and SNAP25 in CSF

### 1.1 Cloning of Rab3a and SNAP25

Specific primers were used to amplify the Rab3 and SNAP 25 coding sequence from the Quick-Screen™ human cDNA library (Clontech, Palo Alto, CA, USA; Cat No K1003-1) with an amplification protocol provided by the manufacturer. In short: 35 cycles of 94°C for 45 sec, 60°C annealing for 45 sec and extension at 72°C for 2 min with Taq polymerase (Stratagene, Amsterdam, The Netherlands; Cat No 600131). The program was finalized with an extension of the polymerase reaction at 72°C for 7 min. Reactions were performed on a Perkin-Elmer (Überlingen, Germany) DNA thermal cycler (Model 480). The sequence of the primers for amplification of Rab3a was based on the human Rab3a sequence (Zahraoui et al., 1989): ATG GCA TCG GCC ACA GAC TCG CGC TAT GGG (Tₘ=76°C) for the ATG primer and CGCG TCTAG AGG CTC TCA GCA GGC GCA GTC CTG GTG CGG (Tₘ 77°C) for the reverse primer. The sequence of the primers for the amplification of SNAP25 was based on the human SNAP25 sequence (Zhao et al., 1994): ATG GCC GAA GAC GCA GAC ATG CGC AAT GAG (Tₘ= 75°C) for the ATG primer and CGCG CTAG ACA CTT AAC CAC TTC CCA GCA TCT TTG TTG (Tₘ= 59°C) for the reverse primer.
The PCR products were re-amplified in order to generate sufficient amount of PCR product, polished with T4 DNA polymerase, cut with XbaI and ligated in NcoI-blunted, xbaI cut pIGRHISA (Innogenetics, Gent, Belgium; Cat No 2075). This resulted in pIGRHISARab3a (Innogenetics, Gent, Belgium; Cat No 3008) for Rab3a and in pIGRH6SNAP25a (Innogenetics, Gent, Belgium; Cat No 2941) for SNAP25. The ligated product was transformed into DH1(λ) (Bachmann, 1987) and Tetracycline resistant colonies were analyzed for the presence of an insert. Inserts were sequenced and the plasmids containing the correct sequence (Zahraoui et al., 1989; Zhao et al., 1994) were further used. For Rab3 a several PCR artifacts were present. The correct sequence was assembled from two clones.

### 1.2 Expression and purification of Rab3a and SNAP25

Rab3a and SNAP25 were expressed in *E*. *coli* using a PL based expression system, pIGRHISARab3a and pIGRH6SNAP25a, respectively. The correct plasmid was transformed into MC1061 pACI (Wertman et al., 1986) with a thermosensitive cI repressor. A coumassie stainable band around 25 kDa was visible on a 12.5% acrylamide gel, indicating a reasonable expression level. The recombinant proteins were made as a fusion protein containing 6 additional histidine residues to allow rapid purification over NiIMAC columns. More than 10 mg of recombinant Rab3 a and SNAP25 were purified to at least 95% homogeneity using 3 liter of heat-induced *E*. *coli* (Hochuli, 1988; Van Gelder et al., 1993).

### 1.3 Generation and characterization of Rab3a and SNAP25 specific antibodies

Antibodies to recombinant Rab3a and SNAP25 were raised in rabbits. 50 µg of purified protein was injected intraperitoneal in two rabbits (100 µg/rabbit). Injections were done every 4 weeks and titers were determined in ELISA. The antibodies were characterized on brain extracts. Results for the Rab3a immunoreactivity are shown in figure 1. Tissue samples from Alzheimer's disease and control patients were prepared by rapidly homogenizing in 5 volumes of 1% SDS, 1% sodium vanadate, 10 mM Tris pH 7.4 and boiling in a water bath for 5 minutes. Homogenates were centrifuged (12000g, room temperature) for 5 minutes to remove insoluble material. A small aliquot of the supernatants was used to measure protein concentration using the BCA method (Pierce, Rockford, Illinois, USA). Supernatants were diluted with water to a protein concentration of 2 mg/ml and an equivalent volume of 2x sample buffer (250mM Tris pH 6.8, 3% SDS, 10% glycerol, 0.006% bromophenol blue and 2% β-mercaptoethanol) was added. Gelelectrophoresis was performed according to the Laemmli system on 10-12.5% gels. Proteins were transferred to nitrocellulose (Schleicher and Schuell, Dassel, Germany; Cat No 401196) with a semi-dry blotting procedure. The nitrocellulose filter was blocked with 1% BSA in 10 mM Tris pH 7.5, 150 mM NaCl. Primary and secondary antibodies were added at the appropriate concentrations (± 1µg/ml) in 1% BSA.

### 1.4 Immuno-affinity purification of the Rab3a and SNAP25 specific antibodies

Purified recombinant antigen was dialyzed overnight in 0.3 M NaHCO₃, pH 8.6. The OD₂₈₀ values were determined before and after dialysis to estimate the amount of protein. A Mini-Leak-Medium (KEM-EN-TEC Biozyme, Vancouver, BC, Canada; Cat No 10127, Lot No 60232-5) was used to immunopurify the antibodies according to instructions provided by the manufacturer. Part of the antibodies were biotinylated (Amersham, Place Little Chalfont Buckinghamshire, UK; Cat No RPN 2202). Purification and labeling was monitored by silver staining and Western blot (results not shown).

### 1.5 Evaluation of stability and presence of Rab3a and SNAP25 in CSF

Using specific monoclonal antibodies (Transduction Labs, Lexington, KY, USA; R35520 and S35020) as capturing antibody and the immunopurified polyclonal rabbit antiserum as detector - antibody, the stability of spiked synapse proteins Rab3a and SNAP25 in CSF was assessed after overnight incubation at 37°C. At concentrations of 500 pg/ml recombinant immunoreactivity for SNAP25 and Rab3a did not change overnight in the CSFs tested (Fig. 2).
Direct evidence that both proteins are present in CSF was obtained by extraction of albumin and IgG from 50 ml CSF and fractionation on column. The fractions were dried and dissolved in sample buffer, run on a 12% acrylamide gel and blotted. The PVDF membranes were probed with a Rab3a and SNAP25 monoclonal antibody. Immunoreactive bands of the expected molecular weight and pI value were detected.

### 1.6 Development of a sandwich ELISA for Rab3a and SNAP25 detection and quantitative determination of CSF levels

A sandwich ELISA based on a monoclonal Rab3a or SNAP25 antibody as capturing antibody and a biotinylated immuno-affinity-purified polyclonal antibody as detector antibody was developed. Maxisorp microtiterplates were coated with Affini Pure Goat anti-Mouse IgG (Jackson Immuno Research Laboratories, Inc., West Grove, Pennsylvania, USA; Cat No 115-035-144) 1% BSA (Clinical Grade 98% fatty acid free, ICN, Biomedical Research Products, Costa Mesa, CA, USA; Cat No 105033, Lot No 6p384) in PBS was used as blocking buffer. Mouse anti-Rab3a (Transduction Labs, Lexington, KY, USA; Cat No R35520, IgG2a, Clone 9, Lot No 606-259-1550 lot 2) or anti-SNAP25 (Transduction Labs, Lexington, KY, USA; Cat No S35020, IgG1, Clone 20) diluted 1/1000 in blocking buffer was added. After incubation, recombinant antigen was added at different concentrations (concentration range 40000-2.56 pg/ml). Simultaneously the affinity-purified rabbit anti-Rab3a or anti-SNAP25 antiserum was added at a concentration chosen for optimal background-signal ratio. The biotinylated rabbit antibodies were then detected via horse-radish labeled streptavidine (Immuno Research Laboratories, Inc., West Grove, Pennsylvania, USA; Cat No 016-030-084) at a dilution of 1/2000. Coupled peroxidase was detected via TMB, H₂O₂ substrate solution. Reaction was stopped after 30 min with 2N H₂SO₄ and absorbance was measured at 450 nm. Based on this ELISA as low as 10 pg/ml Rab3a could be detected. The assay for SNAP25 was based on the same principle.

### Example 2: Presence and detection of α-synuclein in cerebrospinal fluid

### 2.1 Evaluation of a commercial antibody for its specificity for α-synuclein

The specificity of a commercial available monoclonal antibody (Transduction Labs, Lexington, KY, USA; Cat. No. S63320, IgGl) on the different synuclein isoforms was evaluated. α-synuclein, β-synuclein and γ-synuclein open reading frames (from ATG to stop codon) were amplified from a human brain cDNA library (HL5018; Clontech, Palo Alto, CA, USA) using primers based on published sequence data (α-synuclein: accession number L08850; β-synuclein: accession number S69965; γ-synuclein: accession number AF010126). As reported, there were some important amino acid changes in the γ-synuclein's original sequence: K12E and K68E and the polymorphism of amino acid 109 in this clone is E109V. The insert was subcloned in a PL-based expression system (ICCG 3307; Innogenetics, Gent, Belgium) adding 6 additional histidines at the amino terminal. The His-tagged synuclein fusion proteins were expressed in *E. coli.* The *E. coli* proteins were subsequently run on a SDS-PAGE and immunoblotted with the commercial monoclonal antibody from Transduction Labs (Lexington, KY, USA). This monoclonal antibody showed to be specific for α-synuclein, mapping the carboxyterminal half of the synuclein protein (Fig. 3).

### 2.2 Evaluation of the presence of α-synuclein in cerebrospinal fluid

CSF was pooled from several patients. 200 ml pooled CSF was separated according to isoelectric point via a Rotophor. Subsequently, the resulting fractions were run on a SDS-PAGE and immunoblotted with the monoclonal antibody from Transduction Labs (Lexington, KY, USA). Two immuno-reactive bands were detected in the 19 kDa range and with a pI ranging from 4 to 6 (Fig. 4). The lower band could be a C-terminal truncated form, since deletion of some negative charged amino acids (6E in the last 20 amino acids) causes a shift in pI towards the more basic end. Based on this immunoreactivity the concentration range of α-synuclein was estimated in the pg/ml range.

### 2.3 Generation and characterization of α-synuclein specific antibodies

Alpha-synuclein was purified from 3 liters of induced E. *coli* cultures resulting in purification of more than 10 mg α-synuclein (more than 90% pure estimated from coumassie and silverstained gels). The protein was injected into mice following several immunization schemes (Table 3). After 4 injections the titer to α-synuclein was evaluated in a coating ELISA. Six mice had a titer above 100000 (titer defined as serum dilution resulting in OD value twice the background).

Three days after the final injection, spleen cells were retrieved from animal 312 (m3) and used for cell fusion mainly according the procedure as described by Kölher and Milstein (1975). In a first screening round hybridoma's were tested for the presence of specific antibodies in a direct coating assay. Subsequently, they were retested on dot-blot of E. *coli* lysates containing α-, β-, γ-synuclein and deletion mutants from α-synuclein in order to select for hybridomas that produce antibodies that recognize a different epitope on the synuclein protein.

### 2.4 Characterization of α-synuclein specific antibodies

Two hybridoma's were isolated, 3B5 (IgG2a) and 9B6 (IgGl) which, on dot-blot, were specific for α-synuclein. In order to determine its precise epitopes, the carboxyterminal part (position 64 to 140) was synthesized as 10 overlapping peptides (Fig. 5). Those peptides were 14 amino acids long and the overlap was 7 amino acids. The biotinylated peptides were captured on streptavidine coated plates at a concentration of 1µg/ml. α-synuclein specific antibodies were incubated on these peptides and subsequently detected with anti-mouse enzyme coupled antibodies. The enzyme, horse-radish peroxidase, was quantified using trimethylbenzidine as substrate. Both 3B5 and 9B6 recognized the peptide containing the sequence LEDMPVDPDNEAYE (position 113-126), suggesting a linear epitope for this monoclonal antibodies (Fig. 6). In the same set of experiments a commercial antibody (Clone 42, IgGl, Transduction Labs, Lexington, KY, USA; Cat No S63320) which has previously been shown to recognize α-synuclein, could also be mapped to a linear epitope (sequence AGSIAAATGFVKKD, position 85-98)(Fig. 6).

### 2.5 Purification and biotinylation of the α-synuclein specific antibodies

After a second and a third round of sub-cloning, production of antibodies is scaled up to 1-2 liters for purification of 10-20 mg antibody. A Mini-Leak-Medium (KEM-EN-TEC Biozyme, Vancouver, BC, Canada; Cat No 10127, Lot No 60232-5) is used to immunopurify the antibodies according to instructions provided by the manufacturer.

Biotinylation is performed according to well-established procedures (Bonhard et al., 1984) using D-Biotinoyl-eta-aminocaproic acid N-Hydroxysuccinimide Ester (Boehringer-Mannheim, Brussels, Belgium; Cat No 1008960).

### 2.6 Development of a sandwich ELISA for α-synuclein detection and quantitative determination of cerebrospinal fluid levels

A sandwich ELISA based on an α-synuclein antibody as capturing antibody and one of the biotinylated monoclonal antibodies as detector antibody is developed. Maxisorp microtiterplates are coated with Affini Pure Goat anti-Mouse IgG (Jackson Immuno Research Laboratories, Inc., West Grove, Pennsylvania, USA; Cat No 115-035-144). 1% BSA (Clinical Grade 98% fatty acid free; ICN, Biomedical Research Products, Costa Mesa, CA, USA; Cat No 105033, Lot No 6p384) + 1% mice serum in PBS is used as blocking buffer. Anti-α-synuclein (Transduction Labs, Lexington, KY, USA) diluted 1/1000 in blocking buffer is added. After incubation, recombinant antigen is added at different concentrations (concentration range 1000-2 pg/ml). Simultaneously the biotinylated anti-α-synuclein monoclonal antibody is added in the presence of 1% mice antibodies at a concentration chosen for optimal background-signal ratio. The biotinylated rabbit antibodies are then detected via horse-radish labeled streptavidine (Jackson Immuno Research Laboratories, Inc., West Grove, Pennsylvania, USA; Cat No 016-030-084) at a dilution of 1/2000. Coupled peroxidase is detected via TMB, H₂O₂ substrate solution. Reaction is stopped after 30 min with 2N H₂SO₄ and absorbance is measured at 450 nm.

### Example 3: Detection of other neurological markers in cerebrospinal fluid

### 3.1 Tau and phospho-tau

Total tau was measured with the tau antigen test, using AT120 as capturing antibody and biotinylated HT7-BT2 as detector antibody (INNOTEST hTau antigen, Innogenetics, Gent, Belgium). Monoclonal antibody AT120 reacts equally well with both normal and hyperphosphorylated human tau protein (Vandermeeren et al., 1993), monoclonal antibody HT7 also reacts equally well with both normal and hyperphosphorylated human tau protein, while monoclonal antibody BT2 preferentially recognizes normal tau (Goedert et al., 1994). Affinity purified tau protein, prepared as described previously (Mercken et al., 1992b), was used as standard.
Phospho-tau was measured with a sandwich ELISA, using as HT7 as capturing antibody and biotinylated AT270 as detector antibody (INNOTEST phospho-tau(181), Innogenetics, Gent, Belgium). AT270 specifically recognizes phospho-tau (International application published under WO 95/17429).

### 3.2 β-amyloid₍₁₋₄₂₎

β-amyloid(₁₋₄₂₎ concentrations were measured with the Innotest β-amyloid₍₁₋₄₂₎ (Innogenetics, Gent, Belgium). The assay is a sandwich-type ELISA, in which a first monoclonal antibody, 21F12 (specific for the carboxy-terminus of amyloid), is used as capturing antibody and biotinylated 3D6 (specific for the amino-terminus), is used as detector antibody. The combination of 21F12/3D6 allows the specific detection of amyloid₍₁₋₄₂₎ peptide. Some cross-reactivity is observed for amyloid₍₁₋₄₃₎ but not for shorter peptides (Citron et al., 1997; Johnson-Wood et al., 1997). In brief, 21F12 antibody was suspended in 10 mM Tris-10 mM NaCl and coated onto Nunc Maxisorbs microtiter plates overnight at 4°C. After one wash step, plates were blocked for 2 hr at 25°C with PBS-0.1 % casein. The test was performed by simultaneous incubation (one hour, 25°C) of 75 µl biotinylated 3D6 and 25 µl CSF or standard. After several wash steps, the amount of bound antibody was verified by adding 100 µl HRP-streptavidine (RDI, Flanders, New York, NY, USA). Incubation was continued for 30 min. at 25°C. Then, 100 µl of 0.42 mM 3,5,3',5'-tetramethylbenzidine was added as peroxidase substrate. The reaction was stopped after 30 min with 50 µl 0.9N H₂SO₄.

### 3.3 β-amyloid₍₁₋₄₀₎

β-amyloid₍₁₋₄₀₎ concentrations were measured using a C-terminal specific affinity purified polyclonal antibody from Quality Controlled Biochemicals (QCB, Hopkinton, MA, USA) as capturing antibody and 3D6 (Citron et al., 1997; Johnson-Wood et al., 1997) as detector antibody. Nunc maxisorps microtitre plates were coated for 2 hrs at 25°C with 5 µg/ml affinity purified goat anti rabbit IgG (H+L) (Jackson Immuno Research Laboratories, Inc., West Grove, Pennsylvania, USA; Cat. No 111-005-144) in 10 mM Tris-10 mM NaCl buffer. Thereafter, plates were blocked with PBS-0.1% casein overnight at 4°C. The rabbit polyclonal (QCB, Hopkinton, MA, USA; Cat No 44-348-20) was added at a concentration of 0.5 µg/ml for 1 hr at 25°C. After several wash steps, 100 µl CSF or standard were incubated for 2 hrs at 25°C. The amount of bound amyloid was verified by addition of 100 µl biotinylated 3D6-antibody, added at a concentration of 0.1 µg/ml in conjugate diluent for 1 hr at 25°C. Plates were washed again five times. The amount of antibody bound was verified by adding 100 µl SV-AP (Gibco, Rockville, MD, USA; Cat No JK-4410). Incubation was continued for one hour at 25°C. After a final wash step (five times), 100 µl of TMB, dissolved in substrate buffer, was added as peroxidase substrate. The reaction was stopped after 30 min. with 50 µl 0.9N H₂SO₄.

### 3.4 Neuromodulin

Neuromodulin was also measured with a sandwich-type ELISA, using two epitope-specific monoclonal antibodies (NM2, NM4; Oestreicher et al., 1994). NM2 was selected as capturing antibody, biotinylated NM4 as detector antibody. Recombinant neuromodulin was used as standard.

### 3.5 Neuron-specific enolase

NSE measurements were based on a sandwich ELISA using an anti-NSE monoclonal antibody, 2E7, as capturing antibody, and the peroxidase-labelled anti-NSE monoclonal antibody, 10Cl, as detector antibody. Purified NSE from human brain was used as standard (Vanmechelen et al., 1997).

Protein concentrations were determined with the BCA protein reagent (Pierce, Rockford, Illinois, USA).

### Example 4: Combination assay, making use of CSF-Rab3a, CSF-SNAP25, CSF-tau and CSF-beta-amyloid₍₁₋₄₂₎ as neurological markers for the specific detection of Alzheimer's disease and the differentiation of Alzheimer's disease versus age-matched controls

### 4.1 Patients and control subjects

The Alzheimer's disease (AD) group included 32 patients, 15 men and 17 women, with a mean age ± SD of 75.0 ± 6.6 years. The vascular dementia (VAD) group existed of 20 patients, 10 men and 10 women, with a mean age ± SD of 81.0 ± 7.0 years. The control group contained 18 individuals, 7 men and 11 women, with a mean age ± SD of 67.5 ± 5.5 years. Diagnosis of probable AD was made by exclusion, in accordance with the NINCDS-ADRDA criteria (McKhann et al., 1984). VAD was diagnosed in patients with transitory ischemaemic attacks and/or stroke episodes in relation to the evolution of dementia and/or CT finding of large infarcts and/or multiple lacunas, and/or history of or clinical findings of severe vascular diseases, such as arterial hypertension or diabetes mellitus with complications. The control group consisted of individuals without histories, symptoms or signs of psychiatric or neurological disease, malignant disease, or systemic disorders (e.g. rheumatoid arthritis, infectious disease). In individuals over 60 years of age, the cognitive status was examined using the Mini-Mental state examination (Folstein et al., 1975). Individuals with scores below 28 were not included. The study was approved by the Ethics Committee of the University of Göteborg (Göteborg, Sweden). The patients (or their nearest relatives) and the individuals of the control group gave their informed consent to participate in the study.

### 4.2 Isolation of brain-specific cerebrospinal fluid

The procedure has been described in detail by Davidsson et al., 1996. In short, 5-10 ml of CSF was loaded on a Blue Sepharose column (Pharmacia, Uppsala, Sweden) for selective removal of albumin. The albumin-free fraction was than applied on a column with staphylococcal Protein G covalently linked to Sepharose 4B (Pharmacia, Uppsala, Sweden) to absorb IgG. The unabsorbed proteins were separated by mR-HPLC using the SMART-system (Pharmacia LKB technology, Uppsala, Sweden) equiped with a mRPC C₂/C₁₈ column (dim. i.d. 2.1 x 100 mm, gel volume 0.35 ml, particle size 3 mm). Proteins were eluted with two linear gradients of trifluoroacetic acid. In total 40 fractions were dried in a Savant Speed Vac Concentrator. Fractions were dissolved in SDS-PAGE sample buffer, sonicated and boiled for 15 min before separating on 12% polyacrylamide gels.

### 4.3 Evaluation of a combination assay for the specific detection of Alzheimer's disease

By use of a sandwich ELISA the levels of CSF-Rab3a, CSF-SNAP25, CSF-tau and CSF-β-amyloid₍₁₋₄₂₎ were measured in the CSF samples of the 32 AD patients, the 20 VAD patients and the 11 controls. Average levels of all markers are summarized in table 4. Individual values of tau and Rab3 a are given in Fig. 7.

Based on a cut-off value determined by the highest sensitivity for AD patients and highest specificity for the control patient, likelihood ratios were determined (Table 5). Since Rab3a and SNAP25 levels are correlated, one can only use either Rab3 a or SNAP25 in combination with tau and β-amyloid₍₁₋₄₂₎ (Fig. 8). Thus likelihood-ratios for combinations of Rab3a and SNAP25 were not determined. The increased likelihood ratios for the combination of the three neurological markers tau-β-amyloid₍₁₋₄₂₎-Rab3a and tau- β-amyloid₍₁₋₄₂₎-SNAP25 compared to the likelihood ratios for only one or for the combination of only two of the respective markers, indicates that these combinations of three markers enable a more specific and sensitive detection of Alzheimer's disease.

A fully factorial multiple analysis of variance (ANOVA), with all markers as dependent variable, gender as factor, age and minimal mental score evaluation (MMSE; Folstein et al., 1975) as covariates, within the different groups showed that none of these parameters covaried. Furthermore no significant correlation between tau, β-amyloid₍₁₋₄₂₎ and Rab3a/SNAP25 could be detected either in each group individually or in all groups together.

Additional antibodies are isolated for Rab3a and SNAP25. Levels of CSF-Rab3a, CSF-SNAP25, CSF-tau and CSF-β-amyloid₍₁₋₄₂₎ are studied in well-defined clinical diagnostic groups in which an acceptable size of samples allow statistically significant comparisons.

### Example 5: Combination assay, making use of CSF-tau, CSF-neuromodulin and CSF-neuron-specific enolase as neurological markers for the diagnosis of chemotherapy-induced neuronal damage in children treated for leukemia

### 5.1 Patients and control subjects

Between August 1996 and September 1998, 448 samples of CSF were taken from 83 children being treated for cancer at the Pediatric Hemato-oncology Department of the Catholic University of Leuven, Belgium. All patients underwent a thoroughly clinical evaluation at the time of diagnosis. Parental informed consent was available.
Samples were only taken in the course of scheduled lumbar punctions (LPs) for staging or treatment for malignancy. Different groups of patients with hematological malignancies were enrolled in the present study (Table 6). A first group included 9 B-cell non-Hodgkin's lymphoma patients (B-NHL), treated according to the United Kingdom Children Cancers group (UKCCSG 9602) NHL protocol (Table 7a). In this group, four patients had B-cell lymphomas, 3 patients had Burkitt's lymphoma, and 2 patients had anaplastic large cell lymphoma (ALCL). Eight of these patients were studied longitudinally. A second and largest group consisted of 42 patients with non-B-cell acute lymphoblastic leukemia/non Hodgkin's lymphoma (NB ALL/NHL), treated according to the 'European Organization for Research and Treatment of Cancer' (EORTC) protocol 58881 (Table 7b). In this second group, 18 children had CD10(+) blasts or common NB ALL, two patients had Down syndrome (DS), 1 patient had the Brachmann-de Lange syndrome, 3 patients had common B-cell blasts, 1 patient had common T-cell blasts, 2 patients had pro-B-cell blasts, 9 patients had pre-B-cell blasts, and 8 patients had T-cell blasts. Thirty-eight children had leukemia, 5 patients had non-Hodgkin's lymphoma stage II (1 patient), Stage III (3 patients) or Stage IV (1 patient), of which one patient had overt CNS involvement (CNS⁺), defined according to the study protocol with malignant cells in the CSF, eye funduscopy and contrast captation. Five patients were considered as very high risk (VHR) patients according to the criteria defined in the treatment protocol (2 patients with T-cell blast and 3 patients with corticoid-resistance). Twenty-seven of the patients within this group could be followed longitudinally. A third patient group consisted of 6 children with acute myelomic leukemia (AML), of which 1 patient had CNS involvement and two patients had Down syndrome. There were 3 patients with M0, and 1 patient each with M1, M2 or M7 phenotype. All these patients were treated according to the EORTC 58921 protocol (Table 7c) and followed longitudinally. The other patients consisted of a heterogeneous group of children (n=18) in which for clinical reasons a lumbar puncture was performed for diagnosis of infection and staging, during or after their treatment. This group includes 5 children with medulloblastoma (3 staging, 1 during treatment, 1 follow up), 3 children with Hodgkin's disease (staging), 3 children with rhabdomyosarcoma (2 staging, 1 during treatment), 2 children with myelodysplastic syndrome (MDS) (staging), 2 children with Langerhans cell histiocytis (LCH/HLH, staging), 1 child with juvenile chronic myelomic leukemia (CML) (during treatment), choriocarcinoma (follow up), or germinoma (staging). A large group of healthy newborns was not available, since taking cerebrospinal fluid from these patients would be unethical. As controls children suspected of having meningitis but with negative findings (n=4), localized retinoblastoma (n=1) and familial hemophagocytic lymphohistiocytosis (n=1) were enrolled. Parental informed concent was obtained.

### 5.2 Study design

A prospective and longitudinal single-center study design was used. No patients received any treatment prior to entry into the study. In the present explorative study, CSF samples from 58 children were available prior to any treatment (=diagnostic lumbar puncture or LP1) (see Table 7 for additional details). Leftover samples were not available at all time-points for most patients. Missing values are not due to the disease status of the children, but the result of artifacts during sampling or storage of samples.
Lumbar punctures were performed for routine analysis either at baseline for diagnostic work-up or just prior to the IT administration of chemotherapy. Five ml of CSF was collected in different polypropylene tubes. One sample was centrifuged immediately at 1500 rpm for 2 minutes to eliminate cells and other insoluble material. The supernatant was stored at -70°C for subsequent analysis. The number of freeze/thaw cycles was restricted to a minimum. Routine CSF measurement included cytology, protein concentration, glucose, etc.
Since normality for all neurological marker data, independent of the diagnostic groups, was rejected, non-parametric statistics were used for the analysis. The Kruskal-Wallis test was used to investigate group differences regarding the effect variables (tau, neuromodulin, protein, etc.). The Wilcoxon signed ranks test, matched-pairs was used to check for differences between the first diagnostic LP and subsequent LPs. Pearson's correlation was used to investigate possible covariates. Analyses were done with Prism software v2.01 (Graphpad Software Inc., San Diego, CA, USA), Systat version 7 (SPSS, Chicago, IL, USA).

### 5.3 Evaluation of a combination assay for the diagnosis of chemotherapy-induced neuronal damage

### Level of the neurological markers before the treatment.

Normal upper limit levels for tau were firstly determined on CSF samples from children with infectious disease (but with negative viral and bacterial cultures) (n=4), one patient with a very localized retinoblastoma, and one patient screened for familial HLH. The mean tau values in control children was 106.2 pg/ml (95% CI = 34.3-178.0). Arbitrary cut-off normal value was considered as 312 pg/ml (mean + 3 SD) which is in the range of values observed in adults. 80% of normal adult controls have tau values below 352 pg/ml, while 425 pg/ml (p25-p75: 274-713, n=150) were the median tau-levels in Alzheimer's Disease patients (Hulstaert et al, 1999).

Samples were analyzed firstly independent of the patient number; afterwards, all samples derived from one patient were analyzed again on one immunoplate. The correlation coefficient between the results from the first and the second approach for a set of 104 samples was 0.901 (95% CI: 0.856-0.933).
Tau levels at diagnosis were analyzed for each subgroup of patients (Fig. 9). Tau levels at diagnosis ranged from 66 to 1500 pg/ml. The data for tau, neuromodulin, β-amyloid₍₁₋₄₂), β-amyloid₍₁₋₄₀₎, serum LDH and CSF white blood cell count (Fig. 10) show no obvious difference in patient groups with and without Down syndrome or between diagnositc groups. In addition, no correlation was found between the tau level and WBC or LDH levels. No significant correlation was found between the tau concentration and the age of the children. The two patients with MDS, patients with overt CNS invasion (CNS⁺), but not children with AML had markedly enhanced levels of tau at diagnosis. Also, three patients entering the hospital with rised intracranial pressure due to medulloblastoma in the fossa posterior, from which CSF was taken for stageing, had very high CSF-tau concentrations (823,1397,1500). However, 7/21 children with non-B ALL/NHL, 1/4 non-B ALL/NHL patients with very high risk criteria, 2/4 patients with AML and 2/8 patients with B-cell NHL had a level of tau above 312 pg/ml, although using classical diagnostic procedures, CNS invasion was not detected.
For the 27 patients with non-B ALL/NHL or nine patients with B-cell NHL (data not shown), tau levels at diagnosis did not correlate with tumor burden, as reflected by the white blood cell count (p=0.935,n=40) or serum LDH (p=0.855, n=39). At LP1, there was a highly significant correlation between tau and neuromodulin [r=0.793; 95% CI: 0.658-0.928, n=50) indicating that the secretion of both proteins are interrelated at some point. No correlation was seen between tau and β-amyloid₍₁₋₄₂₎ (p=0.1032, n=52).

### Level of neurological markers during treatment of B-NHL patients

The most striking differences with respect to chemotherapy-induced increases of tau in the CSF were detected in the B-cell NHL patients. Results for the three patients from whom both a baseline and a day 9 LP (=LP2) follow-up sample was available, are presented in Fig. 11a. Maximum tau increases were already measurable at day 9, namely after IT administration ofMTX and hydrocortisone, together with IV MTX and vincristine. No additional increases for tau were measured later on. Chemotherapy-induced effects on neuronal metabolic activity were confirmed by similar findings for neuromodulin (Fig. 11b) and neuron-specific enolase (Fig. 11c). In addition, there was a striking correlation between tau and neuromodulin (r=0.722, 95% CI: 0.553-0.834, n=49) or neuromodulin and neuron-specific enolase (r=0.622, 95% CI: 0.247-0.835, n=20).

### Levels of neurological markers during treatment of non-B cell ALL/NHL patients

Data covering the entire treatment period (pre-post samples) was available for 13 non-B ALL patients treated according to EORTC protocol 58881. Median tau values for the different phases of the treatment are presented for the individual patients in figure 12a. Tau levels were significantly increased during the induction period when compared to levels before treatment (LP1) (p=0.048), or when compared to the maintenance period (p=0.040). One patient had already elevated tau levels (893 pg/ml) before initiation of the treatment, possibly reflecting already ongoing neurological dysfunction. There was also a trend (p=0.0522) for an increase in neuromodulin between levels before treatment and the induction period (Fig. 12b). The patient with the high tau level present at the initiation of the therapy also showed a high neuromodulin level at the start of chemotherapy addition. In two patients for which data for NSE for LP1 and LP2 was available [LP1 (4.0 ng/ml, 3.4 ng/ml); LP2 (11.7 ng/ml, 9.6 ng/ml)] a striking increase for NSE was noticed.

Analysis of all data from the non-B ALL patients revealed that the highest tau concentrations were seen in the induction period. During the induction period, 41% of analyzed samples (28/68) were higher than 500 pg/ml. This percentage then decreased to 18.9% (14/74) in the interval period, to 16.7% (3/18) during re-induction and finally to 9.7% (7/72) in the maintenance period (Fig. 13a). Tau values in the three patients who had already elevated tau (> 500 pg/ml) before treatment was initiated, were normalized after chemotherapy. Data for β-amyloid(₁₋₄₂₎, neuromodulin and NSE levels in the non-B ALL patients are shown in Fig. 13b, Fig. 13c and Fig. 13d, respectively. There was also here a striking correlation between levels of tau and neuromodulin (r=0.658, 95% CI = 0.580-0.72,n=251) or NSE (r=0.589, 95% CI = 0.363-0.749, n=47).

Five non-B ALL children with very high risk criteria were tested longitudinally in the present study. The first phase of the treatment was similar to EORTC 58881. Similar chemotherapy-induced changes in tau levels were observed in 4 out of 5 patients, together with highly significant correlation between tau and neuromodulin (n=55; r=0.880, 95% CI: 0.802-0.929).
One Down's syndrome-non-B ALL patient had a tau concentration at diagnosis of 70 pg/ml. While the mean increase of tau in all non-B ALL patients was 250% (95% CI = 130% - 370%, n=13) at day 8 and 200% (95% CI = 150%-260%, n=8) at day 21 in the longitudinal study, the percentage increase of tau in the patient with Down syndrome was 820% and 1200% on days 8 and 21, respectively.
One particular patient was treated with prednisolone for 8 days without IT MTX at day 1, due to the high leukemic burden (610000 WBC/mm³). After 8 days of treatment, tau levels remained low, namely 155 pg/ml. Afterwards, this patient entered into the EORTC protocol as all other non-B ALL patients, including IT injections of MTX during the next weeks. Tau levels increased rapidly to 744, 948, 1120, 861, and 1023 pg/ml at day 12, 15, 18, 22, and 44, respectively.
One CSF sample was available from a child who was treated in another center and who suffered from manifest neurotoxicity after treatment with MTX, and who had diplegia. The tau and neuromodulin levels in this child exceeded the highest standard used (1500 pg/ml for tau, 8000 pg/ml for neuromodulin), reflecting gross neuronal degeneration.

### Levels of neurological markers during treatment for AML

Figure 14a shows the evolution of tau in individual patients with AML. Patients 11, 12 and 23 did not have a significant increase in tau during treatment (Table 7c). Patient 12, from whom LPs were taken at days 1 and 8, had an aggressive disease and died early after bone marrow transplantation. From one out of two patients with Down's syndrome, longitudinal data were available, and this patient had tau levels raising slightly above 300 pg/ml, in contrast to the evolution of tau levels in the CSF of patient 11 and 23. Patient 69, with evidence of CNS invasion at diagnosis, had a tremendous increase of tau and neuromodulin (Fig. 14b) in the CSF. This child is still under treatment, and is in complete remission at the moment.

### Conclusion

We found in our longitudinal study significant increases of the level of tau, neuromodulin and NSE in liquor, which most likely reflect chemotherapy-related neuronal damage, and which were induced primarily at the time of IT MTX in combination with IV corticosteroids and chemotherapy (ALL induction and re-induction therapy, B-cell NHL therapy), but not during high dose IV and IT MTX during the interval therapy.

### Example 6: Combination assay for the diagnosis of brain damage resulting from perinatal asphyxia

Perinatal asphyxia may be associated with neuronal damage. In addition to electroencephalographic and neuroradiologic data, CSF neurological markers may complement clinical data in the evaluation of hypoxic-ischemic events (Garcia-Alix, 1994). It has become increasingly evident that modified brain metabolic activity is reflected by changes in components in the CSF. The perinatal levels of the CSF markers and the distribution of changes due to aspyxia are evaluated.

### Example 7: Combination assay, making use of CSF-neuromodulin, CSF-tau and CSF-β-amyloid ₍₁₋₄₂₎ as neurological markers for the specific detection of Alzheimer's disease and for the differentiation of Alzheimer's disease versus control subjects

### 7.1 Patients and control subjects

CSF was obtained from 109 individuals. Individuals were subdivided into four groups: (i) a group defined as neurological controls for dementia (n=70). This group included patients with multiple sclerosis, Guillain-Barré syndrome, polyneuropathy and epilepsy. The other groups consisted of (ii) elderly patients with memory impairment (n=7), (iii) patients with Alzheimer's disease (AD) (n=27), and (iv) patients with vascular dementia (VAD)(n=5). All patients were diagnosed according the the criteria defined by the International Classification of Diseases, version 9 (Manual of the international statistical classification of diseases, injuries, and causes of death: based on recommendations of the Ninth Revision Conference; 1975, and adopted by the Twenty-Ninth World Health Assembly. Geneva: World Health Organization, 1977). In the AD group, two patients were from a family in which a presenilin mutation determines an early onset of the disease. One of these patients was presymptomatic.

CSF was routinely sampled as part of the neurological examination. All tests were performed on the remainder of the CSF. β-amyloid₍₁₋₄₂₎ was only measured in CSF samples which were properly stored in polypropylene tubes and frozen only once.

### 7.2 Evaluation of a combination assay for the specific detection of Alzheimer's disease and the differentiation of Alzheimer's disease versus control subjects

The levels of tau, β-amyloid₍₁₋₄₂₎ and neuromodulin (GAP-43) in the CSF of these patients were determined. Average CSF levels for each group are shown in table 8. Individual levels of β-amyloid₍₁₋₄₂), tau and neuromodulin are show in figures 15a, 15b and 15c, respectively.

CSF-tau and CSF-β-amyloid₍₁₋₄₂₎ were significantly altered in the AD patients. Also, CSF-neuromodulin was significantly increased in AD. In contrast, in the aged memory-impaired patients, no significant effect in CSF-neuromodulin was observed, while CSF-tau level was significantly raised compared to the control subjects. Three of the 7 patients with memory-impairment had CSF-tau levels above the maximum defined by the control group (280 pg/ml). Due to improper storage, CSF.β-amyloid₍₁₋₄₂₎ levels could only be determined in 3 patients with memory impairment. In two of the three patients, levels below 300 pg/ml were found. One of these patients had also an elevated CSF-tau level. The other patient had a CSF-tau level of 278 pg/ml, i.e. just below the maximum defined by the control group. In the presymptomatic familial AD patient, an increase of CSF-tau level of 327 pg/ml was observed, together with a β-amyloid₍₁₋₄₂₎ level below 300 pg/ml. This analysis on a few samples of memory impaired individuals indicate that the combined use of tau, β-amyloid₍₁₋₄₂₎ and neuromodulin is a better indicator for the presence of Alzheimer's disease. In order to be statistically significant, a larger number of samples per group is being analyzed (at least 50 samples per diagnostic group).

### Example 8: Combination assay, making use of CSF-neuromodulin, CSF-beta-amyloid₍₁₋₄₂₎ and CSF-tau as neurological markers for the specific detection of Alzheimer's disease, to differentiate Alzheimer's disease versus control subjects, for the specific detection of Parkinson disease, to differentiate Parkinson disease versus control subjects and to differentiate between Alzheimer's disease and Parkinson disease.

### 8.1 Patients and control subjects

CSF was obtained from 60 patients with Alzheimer's disease, 23 patients with Parkinson disease and 32 age-mached controls. The levels of tau, β-amyloid₍₁₋₄₂₎ and neuromodulin (GAP-43) in the CSF of these patients were determined. Average CSF levels for each group are shown in table 9.

### 8.2 Evaluation of a combination assay for the specific detection of Alzheimer's disease and to differentiate Alzheimer's disease versus control subjects

Tau was significantly increased in the AD patients compared to the controls, while β-amyloid₍₁₋₄₂₎ levels were decreased. NM levels were not significantly different between both groups. However, NM and tau levels correlated significantly (Fig. 16). Visual inspection of figure 16 shows that values for AD patients could be separated from control patients. Backward discriminant analysis (Morrison, 1976) was used to determine which variables, tau, β-amyloid₍₁₋₄₂₎, neuromodulin or tau/neuromodulin were best to discriminate Alzheimer's disease patients from control patients. The variables tau/neuromodulin and β-amyloid₍₁₋₄₂₎ were selected and correctly classified 55 of the 58 Alzheimer's disease patients (sensitivity 94.8%, CI 85.6%-98.9%) and 30 of the 31 control patients (specificity 96.8%, CI 83.3%-99.9%) (Fig. 17). Further analysis of a larger number of samples will enable to demonstrate a statistically significant improvement of the diagnosis of Alzheimer's disease by use of these three markers.

### 8.3 Evaluation of a combination assay for the specific detection of Parkinson disease and to differentiate Parkinson disease versus control subjects

Backward discriminant analysis (Morrison, 1976) was used to determine which variables were optimal to discriminate controls from Parkinson disease patients. The variables tau/neuromodulin and β-amyloid₍₁₋₄₂₎ were chosen. 14 of the 23 Parkinson disease patients were correctly classified (sensitivity 60.9%, CI 38.6%-80.3%) and 27 of the 31 control patients (specificity 87.1%, CI 70.2%-96.4%) (Fig. 17). Further analysis of a larger number of samples will enable to demonstrate a statistically significant improvement of the diagnosis of Parkinson disease by use of these three markers.

### 8.4 Evaluation of a combination assay to differentiate Alzheimer's disease versus Parkinson disease

β-amyloid₍₁₋₄₂₎ and neuromodulin were selected to differentiate Alzheimer's disease patients from Parkinson disease patients with a sensitivity of 94.8% (CI 85.6%-98.9%) for the Alzheimer's disease patients and a specificity of 78.3% (CI 56.3%-92.5%) for the Parkinson disease patients. Further analysis of a larger number of samples will enable to demonstrate a statistically significant improvement of the differentiation between Alzheimer's disease and Parkinson disease by use of these three markers.

### Example 9: Use of CSF-α-synuclein as a neurological marker to differentiate between Lewy Body dementia and Alzheimer's disease

### 9.1 Patients and control subjects

CSF is obtained from 30-40 patients with Alzheimer's disease, 10-20 patients with Lewy Body dementia and 10-20 age-mached controls.

### 9.2 Assay for the differential diagnosis of Alzheimer's disease and Lewy Body dementia

The concentration of α-synuclein in the CSF of the different patients and control groups is quantified. A significant different mean value for the α-synuclein level in the Alzheimer's disease group versus the mean value for the α-synuclein level in the Lewy Body dementia group allows us to differentiate between both types of dementia. Differentiation between Alzheimer's disease and Lewy Body dementia is further improved by combining the quantification of α-synuclein with the quantification of at least 2 other neurological markers (such as tau and β-amyloid₍₁₋₄₂₎.

### Example 10: Combination assay, making use of CSF-beta-amyloid₍₁₋₄₂₎. CSF-tau and CSF-phospho-tau as neurological markers for the specific detection of Frontal Temporal Lobe dementia and to differentiate Frontal Temporal Lobe dementia versus other dementia

### 10.1 Patients and control subjects

CSF is obtained from 30-40 patients with Frontal Temporal Lobe dementia and 10-20 age-mached controls.

### 10.2 Combination assay for the differential diagnosis of Frontal Temporal Lobe dementia versus control subjects

The level of β-amyloid₍₁₋₄₂₎, tau and phospho-tau in the CSF of the different patients with Frontal Temporal Lobe dementia and in the control patients is quantified. The mean values for the level of tau and phospho-tau in the patients with Frontal Temporal Lobe dementia is significantly increased compared to mean values for the level of tau and phospho-tau in the control patients. The patients with Frontal Temporal Lobe dementia show a decreased level of β-amyloid₍₁₋₄₂₎ compared to the control patients.

### Example 11: Combination assay, making use of CSF-beta-amyloid₍₁₋₄₂₎, CSF-tau and plasma-beta-amyloid₍₁₋₄₂₎ as neurological markers for the specific detection of vascular problems in Alzheimer's disease and to differentiate vascular disease from other forms of Alzheimer's disease

### 11.1 Patients and control subjects

CSF and plasma is obtained from 30-40 patients with vascular disease, from 30-40 patients with other forms of Alzheimer's disease and from 10-20 age-mached controls.

### 11.2 Combination assay for the differential diagnosis of vascular disease versus other forms of Alzheimer's disease

The level of tau and β-amyloid₍₁₋₄₂₎ in the CSF and the level of β-amyloid₍₁₋₄₂₎ in the plasma of the different patients with vascular disease, with other forms of Alzheimer's disease and in the control patients is quantified. A quantitatively different level of plasma-β-amyloid₍₁₋₄₂₎, CSF-tau and CSF-β-amyloid₍₁₋₄₂₎ enables the differentiation between the vascular disease patients and the patients with other forms of Alzheimer's disease.

### REFERENCES

Abbruzzese M, Ferri S, Scarone S (1997) Neuropsychologia 35: 907-912.
Asami-Odaka A, Ishibashi Y, Kikuchi T (1995) Biochemistry 34: 10272-10278.
Auld DS, Kar S, Quirion R (1998) Trends Neurosci 21: 43-49.
Andreadis A, Brown W, Kosik K (1992) Biochem 31: 10626-10633.
Bachmann BJ (1987) In: *E. coli* and *S. typhimurium,* Cellular and molecular biology. Ed. Neidhardt FC et al., ASM 1190-1219.
Ballard C, Grace J, McKeith I, Holmes C (1998) Lancet 351: 1032-1033.
Basi GS (1987) Cell 49: 785-791.
Benowitz LI, Perrone-Bizzozero NI, Finklestein SP, Bird ED (1989) J Neurosci 9: 990-995. Blennow K, Davidson P, Wallin A, Ekman R (1995) Dementia 6: 306-311.
Blennow K, Bogdanovic N, Alafuzoff I, Ekman R, Davidson P (1996) J Neural Transm 103: 603-618.
Bonhard C et al (1984) In: Immunolabeling for Electron Microscopy. Eds. Polak JM, Varndell JM. Elsevier Scientific Publishers, Amsterdam, p. 95.
Bramblett G, Trojanowski J, Lee V (1992) Lab Invest 66: 212-222.
Bramblett G, Goedert M, Jakes R, Merrick S, Trojanowski J, Lee V (1993) Neuron 10: 1089-1099.
Brion J, Passareiro J, Nunez J and Flament-Durand J (1985) Arch Biol 95: 229-235.
Brun A (1993) Dementia 4: 126-131.
Citron M, Diehl TS, Gordon G, Biere AL, Seubert P, Selkoe DJ (1996) Proc Natl Acad Sci 93: 13170-13175.
Citron M, Westaway D, Xia W, Carlson G, Diehl T, Levesque G, Johnson-Wood K, Lee M, Suebert P, Davis A, Kholodenko D, Motter R, Sherrington R, Perry B, Yao H, Strome R, Lieberburg I, Rommens J, Kim S, Schenk D, Fraser P, StGeorge Hyslop P, Selkoe DJ (1997) Nature Medicine 3: 67-72.
Coleman PD, Kazee AM, Lapham L, Eskin T, Rogers K (1992) Neurobiol aging 13: 631-639. Davidsson P, Jahn R, Bergquist J, Ekman R, Blennow K (1996) Mol Chem Neuropathol 27: 195-210.
Delacourte A, Défossez A (1986) J. Neurol. Sci. 76: 173-180.
Delacourte A, Flament S, Dibe E, Hublau P, Sablonniere B, Hemon B, Sherrer V, Défossez A (1990) Acta Neuropathol 80: 111-117.
Ferrer I, Marti E, Tortosa A, Blasi J (1998) J Neuropathol Exp Neurol 57: 218-225.
Flament S, Delacourte A, Mann D (1990) Brain Res 516: 15-19.
Fletcher JM, Copeland DR (1988) J Clin Exp neuropsychol 10: 495-537.
Folstein M, Folstein S, McHugh P.(1975) J Psychol Res 12: 189-198.
Friedland RP, Majocha RE, Reno JM, Lyle LR, Marotta CA (1994) Mol Neurobiol 9: 107-113.
Ghanbari H, Kozuk T, Miller B, Riesing S (1990) J Clin Laboratory Anal 4: 189-192.
Garcia-Alix A, Cabanas F, Pellicer A, Hernanz A, Stiris. TA, Quero J (1994) Pediatrics 93: 234-240.
Goedert M, Spillantini M, Jakes R, Rutherford D, Crowther R (1989) Neuron 3: 519-526.4055.
Goedert M, Cohen E, Jakes R, Cohen P (1992) FEBS Lett. 312: 95-99.
Goedert M, Jakes R, Crowther R, Six J, Lübke U, Vandermeeren M, Cras P, Trojanowski JQ, Lee V (1993) Proc Natl Acad Sci (USA) 90: 5066-5070.
Goedert M, Jakes R, Crowther RA, Cohen P, Vanmechelen E, Vandermeeren M, Cras P (1994) Biochem J 301: 871-877.
Greenberg S, Davies P (1990) Proc Natl Acad Sci USA 87: 5827-5831.
Grundke-Iqbal I, Iqbal K, Quinlan M, Tung Y, Zaidi M, Wisniewski H (1986) J Biol Chem 261: 6084-6089.
Harrington C, Mukaetova E, Hills R, Edwards P, Montejo de Garcini E, Novak M, Wischik C (1991) Proc Natl Acad Sci (USA) 88: 5842-5846.
Hasegawa M, Morishima-Kawashima M, Takio K, Suzuki M, Titani K, Ihara Y (1992) J Biol Chem 267: 17047-17054.
Himmler A (1989) Mol Cell Biol 9: 1389-1396.
Hooten WM, Lyketsos CG (1998) Dement Geriatr Cogn Disord 9: 164-174.
Hulstaert F, Blennow K, Ivanoiu A, Schoonderwaldt HC, Riemenschneider M, De Deyn PP, Bancher C, Cras P, Wiltfang J, Mehta PD, Iqbal K, Pottel H, Vanmechelen E, Vanderstichele H (1999) Neurology 52: 1555-1562.
Hochuli E (1988) J Chromatogr 444: 293-302.
Iqbal K, Grundke-Iqbal I, Smith A, George L, Tung Y, Zaidi T (1989) Proc Natl Acad Sci (USA) 86: 5646-5650.
Jannoun L 1983 Arch Dis Child 58: 953-958.
Jensen PH, Nielsen MS, Jahes R, Dotti CG, Goedert M (1998) J Biol Chem 273: 26292-26294.
Johnson-Wood K, Lee M, Motter R, Hu K, Gordon G, Barbour R, Khan K, Gordon M, Tan H, Games D, Lieberburg I, Schenk D, Seubert P, McConlogue L (1997) Proc Natl Acad Sci 94: 1550-1555.
Kaiser E, Kuzmits R, Pregant P, Burghuber O, Worofka W (1989) Clin Chim Acta 183: 13-32. Kato K, Suzuki F, Umeda Y (1981) J Neurochem 36: 793-797.
Kim KS, Wen GW, Bancher C, Chen CMJ, Sapienza HH, Hong H, Wisniewski HM (1990) Neurosci Res Comm 7: 113-122.
Knopman DS (1993) Dementia 4: 132-136.
Kondo J, Honda T, Mori H, Hamada Y, Miura R, Ogawara M, Ihara Y (1988) Neuron 1: 82. Köhler G, Milstein C (1975 Nature 256: 495-497.
König G, Graham P, Bushnell A, Webster S, Wunderlich D, Perlmutter LS (1996) Ann NY Acad Sci 777: 344-355.
Kosik K, Joachim C, Selkoe (1986) Proc Natl Acad Sci (USA) 83: 4044-4048.
Ksiezak-Reding H, Liu WK, Yen SH (1992) Brain Res 597: 209-219.
Lannfelt-L, Basun-H, Wahlund-LO, Rowe-BA, Wagner-SL (1995) NatMed 1: 829-832.
Lee V, Balin B, Otvos L, Trojanowski J (1991) Science 251: 675-678.
Levy R, Eagger S, Griffiths M, Perry E, Honavar M, Daen A, Lantos P (1994) Lancet 343: 176-178.
Lewis S, Wang D, Cowan N (1988) Science 242: 936-939.
Majocha RE, Reno JM, Friedland RP, VanHaight C, Lyle LR, Marotta CA (1992) J Nucl Med 33: 2184-2189.
McKeith IG, Galasko D, Kosaka K, Perry EK, Dickson DW, Hansen LA, Salmon DP, Lowe J, Mirra SS, Byrne EJ, Lennox G, Quinn NP, Edwardson JA, Ince PG, Bergeron C, Bums A, Miller BL, Lovestone S, Collerton D, Jansen EN, Ballard C, de Vos RA, Wilcock GK, Jellinger KA, Perry RH (1996) Neurology 47: 1113-1124.
McKhann G, Drachman D, Folstein M, Katzman R, Price D, Stadlan EM (1984) Neurology 34: 939-944.
Mercken M, Lübke U, Vandermeeren M, Gheuens J, Oestreicher AB (1992a) J Neurobiol 23; 309-321.
Mercken M, Vandermeeren M, Lübke U, Six J, Boons J, Vanmechelen E, Van De Voorde A, Gheuens J (1992b) J Neurochem 58: 548-553.
Moore I, Kramer JH, Ablin A (1986) Oncol Nurs Forum 12: 45-51.
Morrison DF (1976) Multivariate statistical methods. McGraw-Hill, New York, NY, USA.
Motter R, Vigo-Pelfrey C, Kholodenko D, Barbour R, Johnson-Wood K, Galasko D, Chang L, Miller B, Clark C, Green R, Olson D, Southwick P, Wolfert R, Munroe B, Lieberburg I, Seubert P, Schenk D (1995) Ann Neurol 38: 643-648.
Nara T, Nozaki H, Nakae Y, Arai T, Ohashi T (1988) AJDC 142: 173-174.
Ochs J, Mulhern R, Fairclough D, Parvey L, Whitaker J, Ch'ien L, Mauer A, Simone J (1991) J Clin Oncol 9: 145-151.
Oestreicher AB, Hens JJH, Marquart A (1994) J Neurochem. 62: 881-889.
Perry EK, Haroutunian V, Davis KL, Levy R, Lantos P, Eagger S, Honavar M, Dean A, Griffiths M, McKeith IG, et al (1994) Neuroreport 5: 747-749.
Schoonjans F, Zalata A, Depuydt CE, Comhaire FH (1995) Comput Methods Programs Biomed 48: 257-62.
Selden S, Pollard T (1983) J Biol Chem 258(11): 7064-7071.
Shimohama S, Kamiya S, Taniguchi T, Akagawa K, Kimura J (1997) Biochem Biophys Res Comm 236: 239-242.
Skene JH, Willard M (1981) J Neurosci 1: 419-26.
Six J, Lübke U, Lenders M-B, Vandermeeren M, Mercken M, Villanova M, Van de Voorde A, Gheuens J, Martin J-J, Cras P (1992) Neurodegeneration, impairment of neuronal function 1:247-255.
Sjögren M, Edman A, Wallin A (1997) Psychiatry 12: 656-661.
Soji M, Matsubara E, Kanai M, Watanabe M, Nakamura T, Tomidokoro Y, Shizuka M, Wakabayashi K, Igeta Y, Ikeda Y, Mizushima K, Amari M, Ishiguro K, Kawarabayashi T, Harigaya Y, Okamotot K, Hirai S (1998) J Neurol Sci 158: 134-140.
Stern RA, Trojanowski J,; Lee VM (1990) FEBS-Lett. 264: 43-7.
Suzuki N, Cheung TT, Cai X-D, Odaka A, Otvos L, Eckman C, Golde TE, Younkin SG (1994) Science 264: 1336-1340.
Terry RD, Masliah E, Salmon DP, Butters N, DeTeresa R, Hill R, Hansen LA, Katzman R (1991) Ann Neurol 30: 572-580.
Vandermeeren M, Mercken M, Vanmechelen E, Six J, Van de Voorde A, Martin J, Cras P (1993) J Neurochem 61: 1828-1834.
Van de Voorde A, Vanmechelen E, Vandermeeren M, Dessaint F, Beeckman W, Cras P (1995) Detection of tau in cerebrospinal fluid. Research Advances in Alzheimer's disaese and related disorders, Ed. Ibqal, Mortimer, Winblad & Wisniewski, John Wiley & Sons Ltd.
Van Gelder P, Bosman F, de Meuter F, van Heuverswyn H, Herion P (1993) J Clin Microbiol 31: 9-15.
Vanmechelen E, Blennow K, Davidsson P, Cras P, Van De Voorde A (1997) Alzheimer's Disease: biology, diagnosis and therapeutics. Edited by K Iqbal, B Winblad, T Nishimura, M Takeda, HM Wisniewski, John Wiley & Sons, Ltd. pp.197-203.
Wertman KF, Wyman AR, Botstein D (1986) Gene 49: 253-262.
Wilcock GK, Scott MI (1994) Lancet 344: 544-544.
Wilson JD, Braunwald E, Isselbacher KJ, Petersdorf RG, Martin JB, Fauci AS, Root RK (1991) Harrison's Principles of Internal Medicine, 12th Edition, McGraw-Hill Inc, NY, USA.
Wood J, Mirra S, Pollock N, Binder L (1986) Proc Natl Acad Sci (USA) 83: 4040-4043. Zahraoui A, Touchot N, Chardin P and Tavitian A (1989) J Biol Chem 264: 12394-12401. Zhao N, Hashida H, Takahashi N and Sakaki Y (1994) Gene 145: 313-314.

## Claims

1. A method for specific detection, quantification and/or differential diagnosis of neurodegeneration in an individual comprising the step of determining the level of at least three neurological markers in one or more body fluid sample(s) from said individual, or determining the level of the same marker in two different body fluid samples in combination with the detection of at least one other marker in one or more body fluid sample(s) by means of antibodies specifically recognizing said neurological markers, whereby the type and degree of neurodegeneration is reflected by a quantitative change in the level of all of said neurological markers compared to a control sample, said method further **characterized in that**:
- for the specific detection or quantification of Alzheimer's disease and/or Lewy Body disease and/or for the differential diagnosis of Alzheimer's disease versus Lewy Body disease, the level of tau, β-amyloid₍₁₋₄₂₎ and α-synuclein is determined in a cerebrospinal fluid sample from said individual;
- for the specific detection or quantification of Alzheimer's disease and/or for the differential diagnosis of Alzheimer's disease versus other dementia
■ the level of tau, β-amyloid₍₁₋₄₂₎ and Rab3a is determined in a cerebrospinal fluid sample from said individual; or
■ the level of tau, β-amyloid₍₁₋₄₂₎ and SNAP25 is determined in a cerebrospinal fluid sample from said individual;
- for the specific detection or quantification of Alzheimer's disease and/or Parkinson's disease and/or for the differential diagnosis of Alzheimer's disease versus Parkinson's disease, the level of tau, β-amyloid₍₁₋₄₂₎ and neuromodulin is determined in a cerebrospinal fluid sample from said individual;
- for the specific detection or quantification of neurodegeneration induced by chemotherapy and/or exposure to chemical compounds and/or irradiation, the level of tau, neuron-specific enolase and neuromodulin is determined in a cerebrospinal fluid sample from said individual;
- for the specific detection or quantification of Frontal Temporal Lobe dementia and/or for the differential diagnosis of Frontal Temporal Lobe dementia versus other dementia, the level of tau, phospho-tau and β-amyloid₍₁₋₄₂₎ is determined in a cerebrospinal fluid sample from said individual;
- for the specific detection or quantification of vascular problems in Alzheimer's disease, for the differential diagnosis of different forms of Alzheimer's disease and/or for the differential diagnosis of Alzheimer's disease versus other dementia,
■ the level tau and β-amyloid₍₁₋₄₂₎ is determined quantitatively in a cerebrospinal fluid sample and the level of β-amyloid₍₁₋₄₂₎ is determined quantitatively in a plasma sample from said individual; or
■ the level of phospho-tau and β-amyloid₍₁₋₄₂₎ is determined quantitatively in a cerebrospinal fluid sample and the level of β-amyloid₍₁₋₄₂₎ is determined quantitatively in a plasma sample form said individual; or
■ the level of tau and phospho-tau is determined quantitatively in a cerebrospinal fluid sample and the level of β-amyloid₍₁₋₄₂₎ is determined quantitatively in a plasma sample from said individual; or
■ the level of tau, phospho-tau and β-amyloid₍₁₋₄₂₎ is determined quantitatively in a cerebrospinal fluid sample from said patient.

2. A method according to claim 1 further **characterized in that** the individual, whose neurodegeneration induced by chemotherapy, exposure to chemical compounds and/or irradiation is detected or quantified, is suffering from leukemia or brain tumor.

3. A diagnostic kit for the specific detection, quantification and/or differential diagnosis of neurodegeneration in an individual comprising at least 3 antibodies each recognizing a different neurological marker and recognizing one of the following combinations of neurological markers:
- tau, β-amyloid₍₁₋₄₂), α-synuclein;
- tau, β-amyloid₍₁₋₄₂₎, Rab3a;
- tau, β-amyloid₍₁₋₄₂₎, SNAP25;
- tau, β-amyloid₍₁₋₄₂), neuromodulin;
- tau, neuron-specific enolase, neuromodulin; or
- tau, phospho-tau, β-amyloid₍₁₋₄₂₎; or comprising at least 2 antibodies each recognizing a different neurological marker of which one antibody recognizes a neurological marker in 2 different body fluids, and recognizing one of the following combinations of neurological markers:
- tau, β-amyloid₍₁₋₄₂₎; or
- phospho-tau, β-amyloid₍₁₋₄₂₎.

4. A diagnostic kit according to claim 3, for the specific detection, quantification and/or differential diagnosis of neurodegeneration in an individual comprising:
- a support comprising, together or separately, at least 3 antibodies (primary antibodies or capturing antibodies) each recognizing a different neurological marker, or comprising at least 2 antibodies (primary antibodies or capturing antibodies) each recognizing a different neurological marker of which one antibody recognizes a neurological marker in 2 different body fluids;
- secondary antibodies (detector antibodies), each recognizing one of the neurological marker-primary antibody complexes;
- possibly, a marker either for specific tagging or coupling with said secondary antibodies;
- possibly, appropriate buffer solutions for carrying out the immunological reaction between the primary antibodies and the body fluid sample, between the secondary antibodies and the neurological marker-primary antibody complexes and/or between the bound secondary antibodies and the marker;
- possibly, for standardization purposes, purified proteins or synthetic peptides that are specifically recognized by the antibodies of the kit, used for the detection of the neurological marker.

5. Use of a method or a diagnostic kit according to any of claims 1 to 4, for therapeutic monitoring and/or determination of the effectiveness of a certain treatment.

## Patentansprüche

1. Verfahren zum spezifischen Nachweis, zur Quantifizierung und/oder zur Differenzialdiagnose von Neurodegeneration bei einem Patienten, umfassend den Schritt der Bestimmung des Spiegels von mindestens drei neurologischen Markern in einer oder mehreren Körperflüssigkeitsprobe(n) des Patienten oder der Bestimmung des Spiegels des gleichen Markers in zwei verschiedenen Körperflüssigkeitsproben in Kombination mit dem Nachweis mindestens eines anderen Markers in einer oder mehreren Körperflüssigkeitsprobe (n) mithilfe von Antikörpern, die spezifisch diese neurologischen Marker erkennen, wobei sich der Typ und der Grad der Neurodegeneration in einer quantitativen Veränderung im Spiegel all dieser neurologischen Marker verglichen mit einer Kontrollprobe widerspiegelt, wobei das Verfahren weiter **dadurch gekennzeichnet ist, dass**:
- zum spezifischen Nachweis oder zur Quantifizierung von Alzheimer-Krankheit und/oder Lewy-Körper-Krankheit und/oder zur Differenzialdiagnose von Alzheimer-Krankheit gegenüber Levey-Körper-Krankheit der Spiegel an Tau, β-Amyloid₍₁₋₄₂₎ und α-Synuclein in einer Zerebrospinalflüssigkeitsprobe des Patienten bestimmt wird;
- zum spezifischen Nachweis oder zur Quantifizierung von Alzheimer-Krankheit und/oder zur Differenzialdiagnose von Alzheimer-Krankheit gegenüber anderer Demenz
■ der Spiegel an Tau, β-Amyloid₍₁₋₄₂₎ und Rab3a in einer Zerebrospinalflüssigkeitsprobe des Patienten bestimmt wird oder
■ der Spiegel an Tau, β-Amyloid₍₁₋₄₂₎ und SNAP25 in einer zerebrospinalflüssigkeitsprobe des Patienten bestimmt wird;
- zum spezifischen Nachweis oder zur Quantifizierung von Alzheimer-Krankheit und/oder Parkinson-Krankheit und/oder zur Differenzialdiagnose von Alzheimer-Krankheit gegenüber Parkinson-Krankheit der Spiegel an Tau, β-Amyloid₍₁₋₄₂₎ und Neuromodulin in einer Zerebrospinalflüssigkeitsprobe des Patienten bestimmt wird;
- zum spezifischen Nachweis oder zur Quantifizierung von durch Chemotherapie und/oder Aussetzen gegenüber chemischen Verbindungen und/oder Bestrahlung induzierter Neurodegeneration der Spiegel an Tau, neuronenspezifischer Enolase und Neuromodulin in einer Zerebrospinalflüssigkeitsprobe des Patienten bestimmt wird;
- zum spezifischen Nachweis oder zur Quantifizierung von Demenz des Lobus frontalis/temporalis und/oder zur Differenzialdiagnose von Demenz des Lobus frontalis/temporalis gegenüber anderer Demenz der Spiegel an Tau, Phospho-Tau und β-Amyloid₍₁₋₄₂₎ in einer Zerebrospinalflüssigkeitsprobe des Patienten bestimmt wird;
- zum spezifischen Nachweis oder zur Quantifizierung von vaskulären Problemen bei Alzheimer-Krankheit, zur Differenzialdiagnose verschiedener Formen von Alzheimer-Krankheit und/oder zur Differenzialdiagnose von Alzheimer-Krankheit gegenüber anderer Demenz
■ der Spiegel an Tau und β-Amyloid₍₁₋₄₂₎ quantitativ in einer Zerebrospinalflüssigkeitsprobe und der Spiegel an β-Amyloid₍₁₋₄₂₎ quantitativ in einer Plasmaprobe des Patienten bestimmt wird oder
■ der Spiegel an Phospho-Tau und β-Amyloid₍₁₋₄₂₎ quantitativ in einer Zerebrospinalflüssigkeitsprobe und der Spiegel an β-Amyloid₍₁₋₄₂₎ quantitativ in einer Plasmaprobe des Patienten bestimmt wird oder
■ der Spiegel an Tau und Phospho-Tau quantitativ in einer Zerebrospinalflüssigkeitsprobe und der Spiegel an β-Amyloidd₍₁₋₄₂₎ quantitativ in einer Plasmaprobe des Patienten bestimmt wird oder
■ der Spiegel an Tau, Phospho-Tau und β-Amyloid₍₁₋₄₂₎ quantitativ in einer Zerebrospinalflüssigkeitsprobe des Patienten bestimmt wird.

2. Verfahren nach Anspruch 1, weiter **dadurch gekennzeichnet, dass** der Patient, dessen durch Chemotherapie, Aussetzen gegenüber chemischen Verbindungen und/oder Strahlung induzierte Neurodegeneration nachgewiesen oder quantifiziert wird, an Leukämie oder Hirntumor leidet.

3. Diagnostisches Kit zum spezifischen Nachweis, zur Quantifizierung und/oder zur Differenzialdiagnose von Neurodegeneration bei einem Patienten, umfassend mindestens 3 Antikörper, die jeweils einen unterschiedlichen neurologischen Marker erkennen und eine der folgenden Kombinationen von neurologischen Markern erkennen:
- Tau, β-Amyloid₍₁₋₄₂₎, α-Synuclein;
- Tau, β-Amyloid₍₁₋₄₂₎, Rab3a;
- Tau, β-Amyloid₍₁₋₄₂₎, SNAP25;
- Tau, β-Amyloid₍₁₋₄₂₎, Neuromodulin;
- Tau, neuronenspezifische Enolase, Neuromodulin oder
- Tau, Phospho-Tau, β-Amyloid₍₁₋₄₂₎; oder mindestens 2 Antikörper umfassen, die jeweils einen unterschiedlichen neurologischen Marker erkennen, von denen ein Antikörper einen neurologischen Marker in 2 verschiedenen Körperflüssigkeiten erkennt, und die eine der folgenden Kombinationen von neurologischen Markern erkennen:
- Tau, β-Amyloid₍₁₋₄₂₎ oder
- Phospho-Tau, β-Amyloid₍₁₋₄₂₎.

4. Diagnostisches Kit nach Anspruch 3 zum spezifischen Nachweis, zur Quantifizierung und/oder zur Differenzialdiagnose von Neurodegeneration bei einem Patienten, umfassend:
- einen Träger, der zusammen oder getrennt mindestens 3 Antikörper (Primärantikörper oder Fängerantikörper) umfasst, die jeweils einen unterschiedlichen neurologischen Marker erkennen, oder mindestens 2 Antikörper (Primärantikörper oder Fängerantikörper) umfasst, die jeweils einen unterschiedlichen neurologischen Marker erkennen, von denen ein Antikörper einen neurologischen Marker in 2 verschiedenen Körperflüssigkeiten erkennt;
- sekundäre Antikörper (Nachweisantikörper), die jeweils einen der Komplexe aus neurologischem Marker-primärem Antikörper erkennen;
- gegebenenfalls einen Marker entweder für die spezifische Markierung von oder die Kopplung mit den sekundären Antikörpern;
- gegebenenfalls geeignete Pufferlösungen für die Durchführung der immunologischen Reaktion zwischen den Primärantikörpern und der Körperflüssigkeitsprobe, zwischen den sekundären Antikörpern und den Komplexen aus neurologischem Marker-primärem Antikörper und/oder zwischen den gebundenen sekundären Antikörpern und dem Marker;
- gegebenenfalls zu Standardisierungszwecken gereinigte Proteine oder synthetische Peptide, die spezifisch von den Antikörpern des Kits erkannt werden, die zum Nachweis des neurologischen Markers verwendet werden.

5. Verwendung eines Verfahrens oder eines diagnostischen Kits nach einem der Ansprüche 1 bis 4 zur therapeutischen Überwachung und/oder Bestimmung der Wirksamkeit einer bestimmten Behandlung.

## Revendications

1. Procédé de détection spécifique, quantification et/ou diagnostic différentiel d'une neurodégénérescence chez un individu, comprenant les étapes consistant à :
déterminer le taux de trois marqueurs neurologiques au moins dans un ou plusieurs échantillon(s) de liquide(s) corporel(s) provenant dudit individu ; ou déterminer le taux du même marqueur dans deux échantillons différents de liquides corporels en combinaison avec la détection d'un autre marqueur au moins dans un ou plusieurs échantillon(s) de liquide(s) corporel(s), au moyen d'anticorps reconnaissant spécifiquement lesdits marqueurs neurologiques, moyennant quoi le type et le degré de neurodégénérescence sont reflétés par un changement quantitatif du taux de tous lesdits marqueurs neurologiques en comparaison avec un échantillon de contrôle, ledit procédé étant en outre
**caractérisé en ce que** :
- pour la détection spécifique ou quantification de la maladie d'Alzheimer et/ou de la maladie à corps de Lewy et/ou pour le diagnostic différentiel de la maladie d'Alzheimer par rapport à la maladie à corps de Lewy, les taux de tau, de β-amyloïde₍₁₋₄₂₎ et de α-synucléine sont déterminés dans un échantillon de liquide céphalo-rachidien provenant dudit individu ;
- pour la détection spécifique ou quantification de la maladie d'Alzheimer et/ou pour le diagnostic différentiel de la maladie d'Alzheimer par rapport à d'autres démences
• les taux de tau, de β-amyloïde₍₁₋₄₂₎ et de Rab3a sont déterminés dans un échantillon de liquide céphalo-rachidien provenant dudit individu ;
• les taux de tau, de β-amyloïde₍₁₋₄₂₎ et de SNAP25 sont déterminés dans un échantillon de liquide céphalo-rachidien provenant dudit individu ;
- pour la détection spécifique ou quantification de la maladie d'Alzheimer et/ou de la maladie de Parkinson et/ou pour le diagnostic différentiel de la maladie d'Alzheimer par rapport à la maladie de Parkinson, les taux de tau, de β-amyloïde₍₁₋₄₂₎ et de neuromoduline sont déterminés dans un échantillon de liquide céphalo-rachidien provenant dudit individu ;
- pour la détection spécifique ou quantification d'une neurodégénérescence induite par une chimiothérapie et/ou par une exposition à des composés chimiques et/ou à une irradiation, les taux de tau, d'une énolase spécifique des neurones et de la neuromoduline sont déterminés dans un échantillon de liquide céphalo-rachidien provenant dudit individu ;
- pour la détection ou la quantification spécifique d'une démence fronto-temporale et/ou pour le diagnostic différentiel d'une démence fronto-temporale par rapport à d'autres démences, les taux de tau, de phospho-tau et de β-amyloïde₍₁₋₄₂₎ sont déterminés dans un échantillon de liquide céphalo-rachidien provenant dudit individu ;
- pour la détection spécifique ou quantification de problèmes vasculaires dans la maladie d'Alzheimer, pour le diagnostic différentiel de différentes formes de la maladie d'Alzheimer et/ou pour le diagnostic différentiel de la maladie d'Alzheimer par rapport à d'autres démences,
• les taux de tau et de β-amyloïde₍₁₋₄₂₎ sont déterminés quantitativement dans un échantillon de liquide céphalo-rachidien et le taux de β-amyloïde₍₁₋₄₂₎ est déterminé quantitativement dans un échantillon de plasma provenant dudit individu ; ou
• les taux de phospho-tau et de β-amyloïde₍₁₋₄₂₎ sont déterminés quantitativement dans un échantillon de liquide céphalo-rachidien et le taux de β-amyloïde₍₁₋₄₂₎ est déterminé quantitativement dans un échantillon de plasma provenant dudit individu ; ou
• les taux de tau et de phospho-tau sont déterminés quantitativement dans un échantillon de liquide céphalo-rachidien et le taux de β-amyloïde₍₁₋₄₂₎ est déterminé quantitativement dans un échantillon de plasma provenant dudit individu ; ou
• les taux de tau, de phospho-tau et de β-amyloïde₍₁₋₄₂₎ sont déterminés quantitativement dans un échantillon de liquide céphalo-rachidien provenant dudit patient.

2. Procédé, selon la revendication 1, **caractérisé en outre en ce que** l'individu, dont la neurodégénérescence induite par une chimiothérapie, par une exposition à des composés chimiques et/ou à une irradiation est détectée ou quantifiée, est atteint d'une leucémie ou d'une tumeur cérébrale.

3. Kit de diagnostic destiné à la détection spécifique, la quantification et/ou le diagnostic différentiel d'une neurodégénérescence chez un individu, comprenant au moins 3 anticorps reconnaissant chacun un marqueur neurologique différent et reconnaissant l'une des combinaisons suivantes de marqueurs neurologiques :
- tau, β-amyloïde₍₁₋₄₂₎, α-synucléine ;
- tau, β-amyloïde₍₁₋₄₂₎, Rab3a ;
- tau, β-amyloïde₍₁₋₄₂₎, SNAP25 ;
- tau, β-amyloïde₍₁₋₄₂₎, neuromoduline ;
- tau, énolase spécifique des neurones, neuromoduline ; ou
- tau, phospho-tau, β-amyloïde₍₁₋₄₂₎ ;
ou comprenant au moins 2 anticorps reconnaissant chacun un marqueur neurologique différent, parmi lesquels un anticorps reconnaît un marqueur neurologique dans 2 liquides corporels différents, et reconnaissant l'une des combinaisons de marqueurs neurologiques suivantes :
- tau, β-amyloïde₍₁₋₄₂₎ ; ou
- phospho-tau, β-amyloïde₍₁₋₄₂₎.

4. Kit de diagnostic, selon la revendication 3, destiné à la détection spécifique, la quantification et/ou le diagnostic différentiel d'une neurodégénérescence chez un individu, comprenant :
- un support qui comprend, ensemble ou séparément, au moins 3 anticorps (anticorps primaires ou anticorps de capture) reconnaissant chacun un marqueur neurologique différent ou comprenant au moins 2 anticorps (anticorps primaires ou anticorps de capture) reconnaissant chacun un marqueur neurologique différent, parmi lesquels un anticorps reconnaît un marqueur neurologique dans 2 liquides corporels différents ;
- des anticorps secondaires (anticorps de détection), reconnaissant chacun l'un des complexes "marqueur neurologique-anticorps primaire" ;
- possiblement, un marqueur pour étiqueter spécifiquement lesdits anticorps secondaires ou se coupler à ceux-ci ;
- possiblement, des solutions appropriées de tampons pour effectuer la réaction immunologique entre les anticorps primaires et l'échantillon de liquide corporel, entre les anticorps secondaires et les complexes "marqueur neurologique-anticorps primaire" et/ou entre les anticorps secondaires liés et le marqueur ;
- possiblement, avec des objectifs de standardisation, des protéines purifiées ou des peptides de synthèse qui sont reconnus spécifiquement par les anticorps du kit, utilisés pour la détection du marqueur neurologique.

5. Utilisation d'un procédé ou d'un kit de diagnostic, selon l'une quelconque des revendications 1 à 4, pour le suivi thérapeutique et/ou la détermination de l'efficacité d'un certain traitement.
